(19) **Europäisches Patentamt / European Patent Office / Office européen des brevets**

(11) **EP 4 631 364 A2**

(12) # EUROPEAN PATENT APPLICATION

(43) Date of publication:
**15.10.2025 Bulletin 2025/42**

(21) Application number: **25190637.6**

(22) Date of filing: **02.03.2023**

(51) International Patent Classification (IPC):
**A23L 33/17** $^{(2016.01)}$

(52) Cooperative Patent Classification (CPC):
**A61K 31/19; A61P 25/00;** A61K 9/0043;
A61K 9/0075

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.03.2022 US 202263316845 P**

(62) Document number(s) of the earlier application(s) in
accordance with Art. 76 EPC:
**23763927.3 / 4 486 325**

(71) Applicant: **2508 Biosciences LLC
Denver, CO 80211 (US)**

(72) Inventor: **OLIVER, Emily
Denver, 80212 (US)**

(74) Representative: **J A Kemp LLP
80 Turnmill Street
London EC1M 5QU (GB)**

Remarks:
This application was filed on 21-07-2025 as a
divisional application to the application mentioned
under INID code 62.

(54) **COMPOSITIONS AND METHODS TO PROMOTE BRAIN HEALTH**

(57) The present disclosure provides a composition comprising an active ingredient that is encapsulated in suspension of particles comprising a biodegradable polymer containing an effective amount of the active ingredient, wherein the particles are configured to bypass the blood brain barrier, wherein the nutraceutical agent is synthetic D-beta hydroxybutyrate, and wherein targeted delivery is to the brain. It further provides a method for promoting healing of the brain after a traumatic brain injury comprising neuroinflammation and a method for promoting brain health. It further provides a pharmaceutical composition for use in reducing risk of brain damage due to an acquired brain injury in a subject susceptible to, or otherwise at risk of, the acquired brain injury, said composition comprising a pharmaceutically acceptable carrier and a formulation comprising a therapeutic amount of an API, wherein the API is a synthetic D-beta-hydroxybutyrate, wherein the treatment comprises targeted delivery to the olfactory region of the nasal cavity of the subject by administering the composition intranasally (a) before the subject participates in an event where an acquired brain injury is a known risk; (b) after the subject participates in the event for use in treating symptoms of the acquired brain injury of the subject comprising neuroinflammation; or both, wherein the treatment is neuroprotective.

FIG.1

## Description

### CROSS-REFERENCE TO RELATED APPLICATIONS

This application claims the benefit of priority to U.S. provisional application 63/316,845 entitled COMPOSITIONS AND METHODS TO PROMOTE BRAIN HEATH (filed March 4, 2022, the entire contents of which is incorporated herein by reference in its entirety.

### BACKGROUND OF THE INVENTION

### Biology of Wound Healing

A wound results from damage or disruption to normal anatomical structure and function (Robson MC et al., Curr Probl Surg 2001; 38: 72-140; Velnar T et al., The Journal of International Medical Research 2009; 37: 1528-1542). This can range from a simple break in the epithelial integrity of the skin to deeper, subcutaneous tissue with damage to other structures such as tendons, muscles, vessels, nerves, parenchymal organs and even bone (Alonso JE et al., Surg Clin North Am 1996; 76: 879-903). Irrespective of the cause and form, wounding damages and disrupts the local tissue environment.

Wound healing is a dynamic, interactive process involving soluble mediators, blood cells, extracellular matrix, and parenchymal cells. The wound repair process can be divided into four (4) temporally and spatially overlapping phases: (1) a coagulation phase; (2) an inflammatory phase, (3) a proliferative phase, and (4) a remodeling phase. The immune system is usually an active participant in wound healing. Much of what is known is based on wound healing of human skin.

### Coagulation Phase

Immediately after injury, platelets adhere to damaged blood vessels, initiate a release reaction, and begin a hemostatic reaction, giving rise to a blood-clotting cascade that prevents excessive bleeding and provides provisional protection for the wounded area. Blood platelets release well over a dozen growth factors, cytokines, and other survival or apoptosis-inducing agents (Weyrich AS and Zimmerman GA, Trends Immunol 2004 Sep; 25(9): 489-495). Key components of the platelet release reaction include platelet-derived growth factor (PDGF) and transforming growth factors AI and 2 (TGF-A1 and TGF-2), which attract inflammatory cells, such as leukocytes, neutrophils, and macrophages (Singer AF and Clark RA, N Engl J Med 1999 Sep 2; 341(10): 738-746).

### Inflammatory Phase

The inflammatory phase is triggered by capillary damage, which leads to the formation of a blood clot/provisional matrix composed of fibrin and fibronectin. This provisional matrix fills the tissue defect and enables effector cell influx. Platelets present in the clot release multiple cytokines that participate in the recruitment of inflammatory cells (such as neutrophils, monocytes, and macrophages, amongst others), fibroblasts, and endothelial cells (ECs).

### Proliferative Phase

The inflammatory phase is followed by a proliferative phase, in which active angiogenesis creates new capillaries, allowing nutrient delivery to the wound site, notably to support fibroblast proliferation. Fibroblasts present in granulation tissue are activated and acquire a smooth muscle cell-like phenotype, then being referred to as myofibroblasts. Myofibroblasts synthesize and deposit extracellular matrix (ECM) components that replace the provisional matrix. They also have contractile properties mediated by $\alpha$-smooth muscle actin organized in microfilament bundles or stress fibers. Myofibroblastic differentiation of fibroblastic cells begins with the appearance of the protomyofibroblast, whose stress fibers contain only $\beta$- and $\gamma$-cytoplasmic actins. Protomyofibroblasts can evolve into differentiated myofibroblasts whose stress fibers contain $\alpha$-smooth muscle actin.

### Remodeling Phase

The fourth healing phase involves gradual remodeling of the granulation tissue and reepithelialization. This remodeling process is mediated largely by proteolytic enzymes, especially matrix metalloproteinases (MMPs) and their inhibitors (TIMPs, tissue inhibitors of metalloproteinases). During the reepithelialization, Type III collagen, the main component of granulation tissue, is replaced gradually by type I collagen, the main structural component of the dermis. Elastin, which contributes to skin elasticity and is absent from granulation tissue, also reappears. Cell density normalizes through apoptosis of vascular cells and myofibroblasts (resolution).

**Inflammation**

Tissue injury causes the disruption of blood vessels and extravasation of blood constituents. The blood clot re-establishes hemostasis and provides a provisional extracellular matrix for cell migration. Platelets not only facilitate the formation of a hemostatic plug but also secrete several mediators of wound healing, such as platelet-derived growth factor, which attract and activate macrophages and fibroblasts (Heldin, C. and Westermark B., In: Clark R., ed. The molecular and cellular biology of wound repair, 2nd Ed. New York, Plenum Press, pp. 249-273, (1996)). It was suggested, however, that, in the absence of hemorrhage, platelets are not essential to wound healing; numerous vasoactive mediators and chemotactic factors are generated by the coagulation and activated-complement pathways and by injured or activated parenchymal cells that were shown to recruit inflammatory leukocytes to the site of injury (Id.).

Ingress of cells into a wound and activation of local cells are initiated by mediators that are either released de novo by resident cells or from reserves stored in the granules of platelets and basophils. Sephel, G.C. and Woodward, S.C., 3. Repair, Regeneration and Fibrosis," in Rubin's Pathology, Rubin, R. and Strayer, D.S. Eds; 5th Ed., Wolters Kluwyer Health, /Lippincott Williams & Wilkins, Philadelphia, PA (2008), at 71. Cell migration uses the response of cells to cytokines and insoluble substrates of the extracellular matrix. Id. At 72.

Infiltrating neutrophils cleanse the wounded area of foreign particles and bacteria and then are extruded with the eschar (a dead tissue that falls off (sheds) from healthy skin or is phagocytosed by macrophages). In response to specific chemoattractants, such as fragments of extracellular-matrix protein, transforming growth factor $\beta$ (TGF-$\beta$), and monocyte chemoattractant protein-1 (MCP-1), monocytes also infiltrate the wound site and become activated macrophages that release growth factors (such as platelet-derived growth factor and vascular endothelial growth factor), which initiate the formation of granulation tissue. Macrophages bind to specific proteins of the extracellular matrix by their integrin receptors, an action that stimulates phagocytosis of microorganisms and fragments of extracellular matrix by the macrophages (Brown, E. Phagocytosis, Bioessays, 17:109-117 (1995)). Studies have reported that adherence to the extracellular matrix also stimulates monocytes to undergo metamorphosis into inflammatory or reparative macrophages. These macrophages play an important role in the transition between inflammation and repair (Riches, D., In Clark R., Ed. The molecular and cellular biology of wound repair, 2nd Ed. New York, Plenum Press, pp. 95-141). For example, adherence induces monocytes and macrophages to express Colony-Stimulating Factor-1 (CSF-1), a cytokine necessary for the survival of monocytes and macrophages; Tumor Necrosis Factor-$\alpha$ (TNF-$\alpha$), a potent inflammatory cytokine; and Platelet-Derived Growth Factor (PDGF), a potent chemoattractant and mitogen for fibroblasts. Other cytokines shown to be expressed by monocytes and macrophages include Transforming Growth Factor (TGF-$\alpha$), Interleukin-1 (IL-1), Transforming Growth Factor $\beta$ (TGF-$\beta$), and Insulin-like Growth Factor-I (IGF-I) (Rappolee, D. et al., Science, 241, pp. 708-712 (1988)). The monocyte- and macrophage-derived growth factors have been suggested to be necessary for the initiation and propagation of new tissue formation in wounds, because macrophage depleted animals have defective wound repair (Leibovich, S, and Ross, R., Am J Pathol, 78, pp 1-100 (1975)).

**Epithelialization**

Reepithelialization of wounds begins within hours after injury. Epidermal cells from skin appendages, such as hair follicles, quickly remove clotted blood and damaged stroma from the wound space. At the same time, the cells undergo phenotypic alteration that includes retraction of intracellular tonofilaments (Paladini, R. et al., J. Cell Biol, 132, pp. 381-397 (1996)); dissolution of most inter-cellular desmosomes, which provide physical connections between the cells; and formation of peripheral cytoplasmic actin filaments, which allow cell movement and migration (Goliger, J. and Paul, D. Mol Biol Cell, 6, pp. 1491-1501 (1995); Gabbiani, G. et al., J Cell Biol, 76, PP. 561-568 (1978)). Furthermore, epidermal and dermal cells no longer adhere to one another, because of the dissolution of hemidesmosomal links between the epidermis and the basement membrane, which allows the lateral movement of epidermal cells. The expression of integrin receptors on epidermal cells allows them to interact with a variety of extracellular-matrix proteins (e.g., fibronectin and vitronectin) that are interspersed with stromal type I collagen at the margin of the wound and interwoven with the fibrin clot in the wound space (Clark, R., J Invest Dermatol, 94, Suppl, pp. 128S-134S (1990)). The migrating epidermal cells dissect the wound, separating desiccated eschar (a dead tissue that falls off (sheds) from healthy skin) from viable tissue. The path of dissection appears to be determined by the array of integrins that the migrating epidermal cells express on their cell membranes.

The degradation of the extracellular matrix, which is required if the epidermal cells are to migrate between the collagenous dermis and the fibrin eschar, depends on the production of collagenase by epidermal cells (Pilcher, B. et al., J Cell Biol, 137, pp. 1445-1457 (1997)), as well as the activation of plasmin by plasminogen activator produced by the epidermal cells (Bugge, T. et al., Cell, 87, 709-719 (1996)). Plasminogen activator also activates collagenase (matrix metalloproteinase-1) (Mignatti, P. et al., Proteinases and Tissue Remodeling. In Clark, R. Ed. The molecular and cellular biology of wound repair. 2nd Ed. New York, Plenum Press, 427-474 (1996)) and facilitates the degradation of collagen and extracellular-matrix proteins.

One to two days after injury, epidermal cells at the wound margin begin to proliferate behind the actively migrating cells. The stimuli for the migration and proliferation of epidermal cells during reepithelialization have not been determined, but several possibilities have been suggested. The absence of neighbor cells at the margin of the wound (the "free edge" effect) may signal both migration and proliferation of epidermal cells. Local release of growth factors and increased expression of growth-factor receptors may also stimulate these processes. Leading contenders include Epidermal Growth Factor (EGF), Transforming Growth Factor-$\alpha$ (TGF-$\alpha$), and Keratinocyte Growth Factor (KGF) (Nanney, L. and King, L. Epidermal Growth Factor and Transforming Growth Factor-$\alpha$. In Clark, R. Ed. The molecular and cellular biology of wound repair. 2nd Ed. New York, Plenum Press, pp. 171-194 (1996); Werner, S. et al., Science, 266, pp. 819-822 (1994); Abraham, J. and Klagsburn, M. Modulation of Wound Repair by Members of the Fibroblast Growth Factor family. In Clark, R. Ed. The molecular and cellular biology of wound repair. 2nd Ed. New York, Plenum Press, pp. 195-248 (1996)). As re-epithelialization ensues, basement-membrane proteins reappear in a very ordered sequence from the margin of the wound inward, in a zipper-like fashion (Clark R. et al., J. Invest Dermatol, 79, pp. 264-269 (1982)). Epidermal cells revert to their normal phenotype, once again firmly attaching to the reestablished basement membrane and underlying dermis.

## Formation of Granulation Tissue

New stroma, often called granulation tissue, begins to invade the wound space approximately four days after injury. Numerous new capillaries endow the new stroma with its granular appearance. Macrophages, fibroblasts, and blood vessels move into the wound space at the same time (Hunt, T. ed. Wound Healing and Wound Infection: Theory and Surgical Practice. New York, Appleton-Century-Crofts (1980)). The macrophages provide a continuing source of growth factors necessary to stimulate fibroplasia and angiogenesis; the fibroblasts produce the new extracellular matrix necessary to support cell ingrowth; and blood vessels carry oxygen and nutrients necessary to sustain cell metabolism.

Growth factors, especially Platelet-Derived Growth Factor-4 (PDGF-4) and Transforming Growth Factor $\beta$-1 (TGF-$\beta$1) (Roberts, A. and Sporn, M, Transforming Growth Factor-1, In Clark, R. ed. The molecular and cellular biology of wound repair. 2nd Ed. New York, Plenum Press, pp. 275-308 (1996)) in concert with the extracellular-matrix molecules (Gray, A. et al., J Cell Sci, 104, pp. 409-413 (1993); Xu, J. and Clark, R., J Cell Biol, 132, pp. 239-149 (1996)), were shown to stimulate fibroblasts of the tissue around the wound to proliferate, express appropriate integrin receptors, and migrate into the wound space. It was reported that platelet-derived growth factor accelerates the healing of chronic pressure sores (Robson, M. et al., Lancet, 339, pp. 23-25 (1992) and diabetic ulcers (Steed, D., J Vasc Surg, 21, pp. 71-78 (1995)). In some other cases, basic Fibroblast Growth Factor (bFGF) was effective for treating chronic pressure sores (Robson, M. et al., Ann Surg, 216, pp. 401-406 (1992).

The structural molecules of newly formed extracellular matrix, termed the provisional matrix (Clark, R. et al., J. Invest Dermatol, 79, pp. 264-269, 1982), contribute to the formation of granulation tissue by providing a scaffold or conduit for cell migration. These molecules include fibrin, fibronectin, and hyaluronic acid (Greiling, D. and Clark R., J. Cell Sci, 110, pp. 861-870 (1997)). The appearance of fibronectin and the appropriate integrin receptors that bind fibronectin, fibrin, or both on fibroblasts was suggested to be the rate-limiting step in the formation of granulation tissue. While the fibroblasts are responsible for the synthesis, deposition, and remodeling of the extracellular matrix, the extracellular matrix itself can have a positive or negative effect on the ability of fibroblasts to perform these tasks, and to generally interact with their environment (Xu, J. and Clark, R., J Cell Sci, 132, pp. 239-249 (1996); Clark, R. et al., J Cell Sci, 108, pp. 1251-1261).

Cell movement into a blood clot of cross-linked fibrin or into tightly woven extracellular matrix requires an active proteolytic system that can cleave a path for cell migration. A variety of fibroblast-derived enzymes, in addition to serum-derived plasmin, are suggested to be potential candidates for this task, including plasminogen activator, collagenases, gelatinase A, and stromelysin (Mignatti, P. et al., Proteinases and Tissue Remodeling. In Clark, R. Ed. The molecular and cellular biology of wound repair. 2nd Ed. New York, Plenum Press, 427-474 (1996); Vaalamo, M. et al., J Invest Dermatol, 109, pp. 96-101 (1997)). After migrating into wounds, fibroblasts commence the synthesis of extracellular matrix. The provisional extracellular matrix is replaced gradually with a collagenous matrix, perhaps in response to Transforming Growth Factor-$\beta$1 (TGF-$\beta$1) signaling (Clark, R. et al., J Cell Sci, 108, pp. 1251-1261 (1995); Welch, M. et al., J. Cell Biol, 110, pp. 133-145 (1990))

Once an abundant collagen matrix has been deposited in the wound, the fibroblasts stop producing collagen, and the fibroblast-rich granulation tissue is replaced by a relatively acellular scar. Cells in the wound undergo apoptosis triggered by unknown signals. It was reported that dysregulation of these processes occurs in fibrotic disorders, such as keloid formation, hypertrophic scars, morphea, and scleroderma.

## Neovascularization

The formation of new blood vessels (neovascularization) is necessary to sustain the newly formed granulation tissue. Angiogenesis is a complex process that relies on extracellular matrix in the wound bed as well as migration and mitogenic stimulation of endothelial cells (Madri, J. et al., Angiogenesis in Clark, R. Ed. The molecular and cellular biology of wound repair. 2nd Ed. New York, Plenum Press, pp. 355-371 (1996)). The induction of angiogenesis was initially attributed to acidic or basic Fibroblast Growth Factor. Subsequently, many other molecules have also been found to have angiogenic activity, including vascular endothelial growth factor (VEGF), Transforming Growth Factor-$\beta$ (TGF-$\beta$), angiogenin, angiotropin, angiopoietin-1, and thrombospondin (Folkman, J. and D'Amore, P, Cell, 87, pp. 1153-1155 (1996)).

These molecules induce angiogenesis by stimulating the production of basic Fibroblast Growth Factor (FGF) and Vascular Endothelial Growth Factor (VEGF) by macrophages and endothelial cells. For example, it was reported that activated epidermal cells of the wound secrete large quantities of Vascular Endothelial cell Growth Factor (VEGF) (Brown, L. et al., J Exp Med, 176, 1375-1379 (1992)).

Basic fibroblast growth factor was hypothesized to set the stage for angiogenesis during the first three days of wound repair, whereas vascular endothelial-cell growth factor is critical for angiogenesis during the formation of granulation tissue on days 4 through 7 (Nissen, N. et al., Am J Pathol, 152, 1445-1552 (1998)).

In addition to angiogenesis factors, it was shown that appropriate extracellular matrix and endothelial receptors for the provisional matrix are necessary for angiogenesis. Proliferating microvascular endothelial cells adjacent to and within wounds transiently deposit increased amounts of fibronectin within the vessel wall (Clark, R. et al., J. Exp Med, 156, 646-651 (1982)). Since angiogenesis requires the expression of functional fibronectin receptors by endothelial cells (Brooks, P. et al., Science, 264, 569-571 (1994)), it was suggested that perivascular fibronectin acts as a conduit for the movement of endothelial cells into the wound. In addition, protease expression and activity were shown to also be necessary for angiogenesis (Pintucci, G. et al., Semin Thromb Hemost, 22, 517-524 (1996)).

The series of events leading to angiogenesis has been proposed as follows. Injury causes destruction of tissue and hypoxia. Angiogenesis factors, such as acidic and basic Fibroblast Growth Factor (FGF), are released immediately from macrophages after cell disruption, and the production of vascular endothelial-cell growth factor by epidermal cells is stimulated by hypoxia. Proteolytic enzymes released into the connective tissue degrade extracellular-matrix proteins. Fragments of these proteins recruit peripheral-blood monocytes to the site of injury, where they become activated macrophages and release angiogenesis factors. Certain macrophage angiogenesis factors, such as basic fibroblast growth factor (bFGF), stimulate endothelial cells to release plasminogen activator and procollagenase. Plasminogen activator converts plasminogen to plasmin and procollagenase to active collagenase, and in concert these two proteases digest basement membranes. The fragmentation of the basement membrane allows endothelial cells stimulated by angiogenesis factors to migrate and form new blood vessels at the injured site. Once the wound is filled with new granulation tissue, angiogenesis ceases and many of the new blood vessels disintegrate as a result of apoptosis (Ilan, N. et al., J Cell Sci, 111, 3621-3631 (1998)). This programmed cell death has been suggested to be regulated by a variety of matrix molecules, such as thrombospondins 1 and 2, and anti-angiogenesis factors, such as angiostatin, endostatin, and angiopoietin 2 (Folkman, J., Angiogenesis and angiogenesis inhibition: an overview, EXS, 79, 1-8, (1997)).

**Wound Contraction and Extracellular Matrix Reorganization**

Wound contraction involves a complex and orchestrated interaction of cells, extracellular matrix, and cytokines During the second week of healing, fibroblasts assume a myofibroblast phenotype characterized by large bundles of actin-containing microfilaments disposed along the cytoplasmic face of the plasma membrane of the cells and by cell-cell and cell-matrix linkages (Welch, M. et al., J Cell Biol, 110, 133-145 (1990); Desmouliere, A. and Gabbiani, G. The role of the myofibroblast in wound healing and fibrocontractive diseases. In Clark, R. Ed. The molecular and cellular biology of wound repair. 2nd Ed. New York, Plenum Press, pp. 391-423 (1996)). The appearance of the myofibroblasts corresponds to the commencement of connective-tissue compaction and the contraction of the wound. This contraction was suggested to require stimulation by Transforming Growth Factor (TGF)-$\beta$1 or $\beta$2 and Platelet-Derived Growth Factor (PDGF), attachment of fibroblasts to the collagen matrix through integrin receptors, and cross-links between individual bundles of collagen. (Montesano, R. and Orci, Proc Natl Acad Sci USA, 85, 4894-4897 (1988); Clark, R. et al., J Clin Invest, 84, 1036-1040 (1989); Schiro, J. et al., Cell, 67, 403-410 (1991); Woodley, D. et al., J Invest Dermatol, 97, 580-585 (1991)).

Collagen remodeling during the transition from granulation tissue to scar is dependent on continued synthesis and catabolism of collagen at a low rate. The degradation of collagen in the wound is controlled by several proteolytic enzymes, termed matrix metalloproteinases (MMP), which are secreted by macrophages, epidermal cells, and endothelial cells, as well as fibroblasts (Mignatti, P. et al., Proteinases and Tissue Remodeling. In Clark, R. Ed. The molecular and cellular biology of wound repair. 2nd Ed. New York, Plenum Press, 427-474 (1996)). Various phases of wound repair have been suggested to rely on distinct combinations of matrix metalloproteinases and tissue inhibitors of metalloproteinases (Madlener, M. et al, Exp Cell Res, 242, 201-210 (1998)).

Wounds gain only about 20 percent of their final strength in the first three weeks, during which fibrillar collagen has accumulated relatively rapidly and has been remodeled by contraction of the wound. Thereafter, the rate at which wounds gain tensile strength is slow, reflecting a much slower rate of accumulation of collagen and collagen remodeling with the formation of larger collagen bundles and an increase in the number of intermolecular cross-links

## Acquired Brain Injury

An acquired brain injury is brain damage caused by events after birth, rather than as part of a genetic or congenital disorder. Examples of acquired brain injury include, without limitation, traumatic brain injury (TBI), stroke, brain tumor, poisoning, infection and disease, near drowning or other anoxic episodes, and alcohol and drug abuse. Examples of circumstances resulting in an acquired brain injury can include, without limitation, domestic violence, falls, car crashes, etc. Acquired brain injuries differ from degenerative brain conditions, such as Alzheimer's or Parkinson's disease.

## Neurodegenerative Diseases

Neurodegenerative diseases occur when nerve cells in the brain or peripheral nervous system lose function over time and ultimately die. Although certain treatments may help relieve some of the physical or mental symptoms associated with neurodegenerative diseases, slowing their progression is not currently possible, and no cures exist. Examples include, without limitation, Parkinson's disease, Alzheimer's disease and other memory disorders, Huntington's disease, motor neuron disease or Amyotrophic lateral sclerosis (ALS), ataxia, multiple system atrophy, and progressive supranuclear palsy. With their progression, these disorders can affect every aspect of a person's life, including mobility and balance, abnormal movements; swallowing; bladder and bowel function, blood pressure fluctuation; sleep; breathing; heart function; memory and cognitive abilities; mood and speech. t has been hypothesized that exposure to environmental contaminants, such as pesticides, fungicides, insecticides, metals (e.g., arsenic, lead, manganese); chemicals (e.g. polychlorinated biphenyls); air pollution, biological factors (e.g., bacterial endotoxins), dietary and lifestyle factors (e.g., caffeine, tobacco smoke, dietary antioxidants) may affect neurodegeneration, either individually or in combination with specific genes.

For example, Parkinson's disease progresses slowly as small clusters of brain neurons die. The gradual loss of those neurons reduces levels of the neurotransmitter dopamine that transmits messages to the parts of the brain that coordinate muscle movement. Common symptoms of Parkinson's disease include tremors or shaking in the hands, arms, legs, jaw, and face; rigidity or stiffness of the limbs and trunk; bradykinesia, or slowness of movement; and difficulties with balance, speech, and coordination. Symptoms begin gradually and typically worsen over time. Although scientists do not know the cause of Parkinson's disease, most agree that interaction between a person's genes and environment play a role in disease onset and progression.

Alzheimer's disease (AD) is a progressive neurodegenerative disease most often characterized by initial memory impairment and progressive and irreversible cognitive decline that can ultimately affect behavior, speech, visuospatial orientation and the motor system, Pathologically, AD is defined by two lesions evident on microscopic examination of postmortem tissue: extracellular plaques composed primarily of aggregated amyloid-$\beta$ (A$\beta$) 1-42 amino acid polypeptide, and intracellular neurofibrillary tangles (NFTs) formed from hyperphosphorylated aggregated forms of the microtubule-associated protein tau [Montine, TJ et al. Acta Neuropathol. (2012) 123 (1): 1-11). These proteins aggregate into highly toxic, soluble oligomeric structures and ultimately into microscopically detectable, fibrillar macrostructures such as plaques and tangles. This is associated with synaptic dysfunction and synapse loss, disruption of neurotransmitter systems, and neuronal death in regions where one or both protein aggregates are found (Selkoe, DJ, Science (2002) 298 (5594): 789-91; Spires-Jones, TL & Hyman, BT. Neuron (2014) 82 (4): 756-71). Ultimately, neurodegeneration gives way to cognitive decline and dementia [Hardy, J. and Selkoe, DJ. Science (2002) 297 (5580): 353-6].

Huntington's disease (HD) is a rare, progressive neurodegenerative disorder with autosomal-dominant inheritance. It is caused by a mutation in the gene for the protein huntingtin that causes a CAG trinucleotide (cytosine, adenine, and guanine) repeat expansion located in the first exon of the HD gene, which encodes huntingtin, a 350 kDa protein of unknown function. Expansions of CAG trinucleotide repeats (CAG repeats) in coding regions of human genes cause neurodegenerative disorders by generating proteins with elongated polyglutamine (polyQ) stretches. This group of disorders includes Huntington's disease (HD), dentatorubral pallidoluysian atrophy, spinal bulbar muscular atrophy and the spinocerebellar ataxia (SCA) types 1, 2, 3, 6 and 7 [Moncke-Buchner, E. et al. Nucleic Acid Res. (2002) 30 (16): e83].

The term "motor neuron disease" describes a heterogeneous group of conditions, all of which are relentlessly progressive and ultimately fatal (including amyotrophic lateral sclerosis, or ALS) that causes progressive muscle weakness through loss of upper and lower motor neurons. Non-motor pathways are also affected, and up to 50% of patients have detectable cognitive and behavioral changes. [Verber, NS, et al. Front. Neurol. (2019) 10: 291, citing Wooley, SC and Jonathan, SK. Phys. Med. Rehabil. Clin. N. Am. (2008) 19: 607-17].

Ataxia, or lack of coordination, is a common manifestation of various neurological conditions, including stroke, brain tumor, multiple sclerosis, traumatic brain injury, toxicity, infection (including following varicella) and congenital cerebellar defects. Its evolution can be acute, subacute, episodic or chronic. The ataxias may be broadly divided into those that are genetic (with or without a family history) and those that are acquired/degenerative. 'Sporadic' ataxia implies there is no family history. Acquired progressive ataxias can be immune mediated (e.g., paraneoplastic spinocerebellar degeneration, gluten ataxia), degenerative (e.g., cerebellar variant of multiple systems atrophy (type C)), caused by deficiency states (e.g., vitamin B12, vitamin E, etc.), toxicity (e.g., alcohol-related ataxia, phenytoin), or associated with infections (HIV, sporadic Creutzfeldt-Jakob disease, progressive multifocal leucoencephalopathy, etc.). Inherited ataxias can have autosomal dominant, autosomal recessive, X-linked or mitochondrial (maternal) inheritance. Metabolic disorders (e.g., Niemann-Pick type C, Tay-Sachs disease), even though 'inherited', can present as late-onset ataxia with no family history. [Nilantha de Silva, R. et al. Pract. Neurol. (2019) 19 (3): 196-207].

Progressive cognitive disorders related to accumulation of neurofibrillary tangles ("NFT") include, but are not limited to, progressive supranuclear palsy; dementia; dementia pugilistica; Creutzfeldt-Jakob disease; frontotemporal dementia; Pick's disease; other tau-positive pathology corticobasal degeneration; frontotemporal lobar degeneration (FTLD); and dementia lacking distinctive histology.

## Chronic traumatic encephalopathy (CTE)

Chronic traumatic encephalopathy is believed to be a rare progressive degenerative disease of the brain found in those with a history of repetitive head trauma. Because it can only be definitely diagnosed by autopsy after death, it is not yet well understood. It was originally named *dementia pugilistica,* but has been expanded to include brain damage from additional sources. Serious impacts suffered in cases of traumatic brain injury (TBI) can lead to CTE, especially when they are suffered on a repeated basis. Repetitive head impacts (RHI), defined as the cumulative exposure to recurrent concussive and subconcussive events, have long been associated with CTE. [Nowinski, CJ et al. Front. Neurol. (2022) 13: 938163]. Even relatively minor impacts in mild traumatic brain injuries (MTBI) or concussions can cause or contribute to the condition.

CTE is characterized by an ongoing degeneration of the brain tissue, along with the accumulation of tau protein. The condition may set in within months of the injury, or it may not begin for several years or even decades after the last impact. Symptoms of CTE include changes in behavior, memory loss, depression, aggression, impulsive behavior and impaired judgment, and the individual will gradually develop dementia.
[www.protectthebrain.org, visited 2.6.2023].

## Epilepsy

A seizure is a paroxysmal, rhythmic change in cortical electrical activity that is almost always accompanied by a change in behavior. This change may be purely subjective (i.e., sensory symptoms that are not apparent to an outside observer), and on occasion such electrical activity produces no symptoms at all-a so-called subclinical or electrographic seizure. A focal or partial seizure is one that originates in one hemisphere; the generator may be a small area, or a large multilobar network within a hemisphere, and the discharge may subsequently spread to involve both hemispheres diffusely. A generalized seizure originates from both hemispheres simultaneously. There are several different types of generalized seizures, the most common of which is the tonic-clonic ("grand mal") type in which loss of consciousness with tonic stiffening is followed by gradually increasing clonic (back-and-forth) muscle activity, all lasting about a minute. There are also isolated clonic seizures and isolated tonic seizures, each featuring the respective form of motor activity without the other kind, as well as the dreaded atonic seizures, in which the patient abruptly loses all muscle tone and collapses to the ground, frequently producing self-injury. Commonly seen in children are absence ("petit mal") seizures, episodes of loss of awareness and behavioral arrest for a few seconds, sometimes accompanied by eye fluttering, with the patient immediately returning to his or her previous activities as if nothing had happened (and usually unaware that anything has happened). Finally, myoclonic seizures-sudden, split-second, lightning-like contraction of a muscle or group of muscles, accompanied by a diffuse change on electroencephalography (EEG)-are often not mentioned by the patient and usually have little effect themselves on the patient's well-being but can provide very important clues to properly categorizing the patient's epilepsy syndrome.[Mintzer, S. Pharmacology and Therapeutics, Principles to Practice (2009), pages 663-83, Elsevier, Inc.]

The generalized epilepsies are further subdivided into idiopathic (primary) and symptomatic (secondary), with the former patients having normal intelligence and brain function, and the latter having generalized seizures secondary to some diffuse brain disease (e.g., congenital hypoxia, tuberous sclerosis) that has also produced mental retardation.

It is widely believed that the primary generalized epilepsies are genetic in nature, and there are numerous specific genes that have been associated with various generalized epilepsy syndromes, only some of which have clearly identified functions. The inheritance and penetrance patterns appear to be complex, however, and the genes thus far discovered account for only a small percentage of patients with primary generalized epilepsy. [Id., citing Berkovic, SF. Genetics of epilepsy syndromes. In Engel, J. Pedley, T. (eds) Epilepsy: A comprehensive Textbook. Philadelphia: Lippincott-Raven (1997).

Focal epilepsy, at the "macro" level, may be caused by a host of focal lesions occurring anywhere in the cerebral cortex, including cortical dysplasias, neoplasms, heterotopias and hamartomas, congenital malformations, infarctions, hemorrhages, vascular malformations, traumatic injuries, and central nervous system infections of various kinds. The most well-studied cause of focal epilepsy is hippocampal sclerosis, a condition with a very particular pattern of neuronal cell loss and gliosis that differs from what is seen with anoxic damage, Alzheimer's disease, or other hippocampal pathologies.8 Hippocampal sclerosis is often associated with a neurologic "hit" in childhood (e.g., a prolonged febrile seizure, meningitis), and it is likely the most common cause of drug-resistant focal epilepsy, often requiring resective surgery (discussed later in the chapter). Despite extensive study, and the striking success of hippocampal resection for this condition, it remains entirely unclear what it is about this condition that generates the seizures at a systems level.

At the "micro" level, seizures have been conceptualized as being due to excessive neuronal excitation, inadequate inhibition, or both. Focal seizures are a product of groups of electrically hyperexcitable neurons that fire synchronously, producing a rhythmic, evolving electrical discharge. In between seizures, most such groups of neurons produce much briefer synchronous depolarizations; in electrical field recordings such as EEGs these manifest as sharp-appearing waves (interictal epileptiform activity, also called spikes or sharp waves). These waves-and presumably the seizures that arise from the same neurons-result from the summation of many paroxysmal depolarizing shifts in postsynaptic neurons, which have been presumed to be due to excitatory postsynaptic potentials resulting from neurotransmitter release by presynaptic neurons.[Id., citing Prince, D.A. and Futamachi, KT. Intracellular recordings in chronic focal epilepsy. Brain Res. (1968) 11: 681-4; Dichter, M. and Spencer, WA. Penicillin-induced interictal discharges from the cat hippocampus. I. Characteristics and topographical features. J. Neurophysiol. (1969) 32: 649-62]. While recent evidence suggests that the excitatory neurotransmitters underlying many paroxysmal depolarizing shifts may come from astrocytes rather than neurons [Id., citing Tian, GF et al. An astrocytic basis of epilepsy. Nature Med. (2005) 11: 973-81],it is clear regardless that glutamate and its ionotropic receptors play a role in the generation of epileptiform activity. Some anticonvulsant drugs may work through glutamate, at least in part (e.g., N-methyl-d-aspartate [NMDA] receptor antagonism), while others may work by reducing fast-frequency neuronal firing so that less glutamate is released at the synapses. In an analogous fashion, GABA, the predominant inhibitory neurotransmitter in the central nervous system, can clearly impede the development of epileptiform activity and reduce seizures, and several anticonvulsant drugs (e.g., barbiturates, benzodiazepines, tiagabine) appear to exert their anticonvulsant properties by increasing synaptic GABA or potentiating its effects. It is worth noting that, in some circumstances (e.g., in absence seizures, as described previously), neuronal hypersynchrony may be produced by excessive, rather than inadequate, inhibition, depending on the epileptogenic circuitry.

One near-universal feature of seizures is that individual events terminate within a few minutes, or even seconds. If the pathophysiologic underpinnings of seizure onset are largely unknown, those underlying seizure termination remain even more obscure. Occasionally a seizure fails to terminate, resulting in an ongoing, rhythmic cortical discharge, usually with a profound clinical correlate, referred to as status epilepticus. It is presumed that the occurrence of a seizure engages inherent ictal termination mechanisms within the brain, and that status epilepticus results from failure of these termination mechanisms. Status epilepticus is a medical emergency under most circumstances, so that while the pharmacologic mechanisms engaged in combating it are similar to those employed for recurrent single seizures, the mode of delivery is generally more aggressive (e.g., intravenous [IV], rectal, etc.).

Seizures can occur early ( within the first week of the brain injury), or late ( more than a week after brain injury). Seizures which occur early after a traumatic brain injury are felt to be a symptom of the recent injury. Seizures which occur in the late period after TBI are more likely to recur and result in epilepsy.

According to the Epilepsy Foundation (www.epilepsy.com, visited 2.6.2023), approximately 1 in 10 people (10%) will experience an early seizure after TBI. 50% of early seizures occur during the first 24 hours following TBI, while 25% of early seizures occur during the first hour following TBI. Most very early seizures (within 24 hours of injury) are generalized tonic clonic seizures.

About 1 in 10 people will develop status epilepticus in the early period after a TBI. Younger children are at highest risk for early post traumatic seizures and status epilepticus

People who have head injuries that are more serious (examples: car accidents, fall from height, military blast injury), cause brain swelling or blood on the outside of the brain (subdural hemorrhage), or involve the brain being penetrated by a foreign object (example: bullet, combat injuries), or are accompanied by an extended period with loss of consciousness (>30 minutes) are more likely to have early seizures. One in four people who suffer from bleeding in the brain (intracerebral hematoma) requiring surgery or a skull fracture that compresses or injures brain tissue experience an early seizure.

In some cases, even if a head injury is "mild" and a person has no evidence of injury to the brain on brain imaging with CT or MRI, a seizure may still occur.

EEG changes may or may not be present in the immediate period after a head injury. If markers of seizures on EEG early after TBI this may mean a person is more likely to develop epilepsy. People with early seizures after a brain trauma are at higher risk for developing post-traumatic epilepsy.

Seizures which occur more than a week after a traumatic brain injury are considered late seizures. Most often when this happens, it is because there has been more serious injury to brain cells and the chemical environment around the cells has also changed. Late seizures are more likely to lead to the complication of post-traumatic epilepsy.

A person with post-traumatic epilepsy (PTE) is someone with risk for recurrent seizures as a result of a brain injury. About 1 in 50 people who have traumatic brain injuries will go on to develop PTE. There is a spectrum of severity of PTE that ranges from well controlled seizures to disabling seizures that are resistant to treatment. Most seizures (8 out of 10 people) in post-traumatic epilepsy are focal and may spread to become bilateral tonic clonic seizures.

In 1 out of 2 people post traumatic epilepsy occurs within one year of their brain injury. The more severe the head injury the longer a person is at risk for developing epilepsy. Risk for PTE developing decreases substantially with time but can extend out to 15 years after the original traumatic injury for people with the most severe brain injuries. A person is at higher risk for developing PTE if,

they experience early seizures after the TBI;

they had bleeding into brain tissue or bruising (contusion) of the brain at time of injury;

they suffered a skull fracture and a piece of the skull shifted into the brain injuring brain tissue, or compressed the brain (depressed skull fracture);

a penetrating brain trauma occurred (example bullet, combat injury);

they have a head injury that has occurred in relation to alcohol use;

surgery needed to remove bleeding (hematoma) from the brain, remove a foreign object from the brain, or to drain fluid (ventriculostomy) from the brain if there is swelling in the brain related to the trauma;

EEG has abnormalities which appear early in the course post injury;

a person is older than age 65;; and/or

a person has a family history of epilepsy.

**Traumatic brain injury**

Traumatic brain injury (TBI) is a nondegenerative, noncongenital insult to the brain from an external mechanical force, possibly leading to permanent or temporary impairment of cognitive, physical and psychosocial functions, with an associated diminished or altered state of consciousness. It can manifest clinically from concussion to coma and death. TBI as a result of a sport and recreation head injury has become of increasing concern.

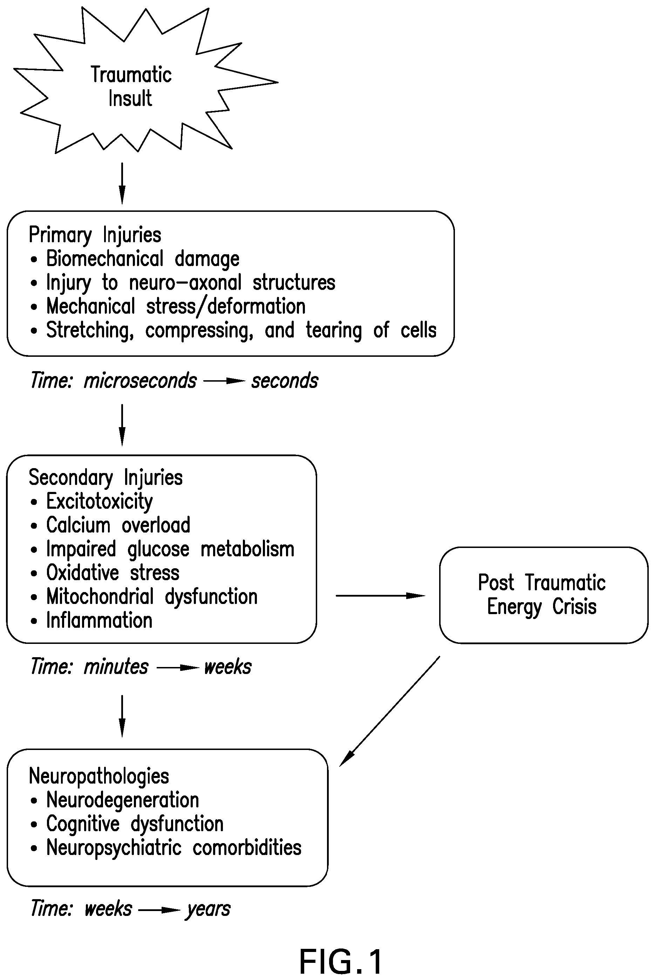

As depicted schematically in **FIG. 1,** TBIs are divided into two subcategories: (1) primary injury, which occurs at the moment of trauma, and (2) secondary injury, which occurs immediately after trauma and produces effects that may continue for a long time. Secondary injuries may develop over a period of hours or days following the initial traumatic assault.

Primary injuries can manifest as focal injuries (e.g., skull fractures, intracranial hematomas, lacerations, contusions, penetrating wounds), or they can be diffuse (as in diffuse axonal injury). Diffuse injuries are microscopic changes that do not appear on CT scans and are scattered throughout the brain.

Concussion is considered a mild form of diffuse axonal injury. It is caused by deformation of the deep structures of the brain, leading to widespread neurologic dysfunction that can result in impaired consciousness or coma.

One of the most widely used systems to classify outcomes from head injury is the Glasgow Outcome Scale (GOS).

Glasgow Outcome Scale'

| Outcome | Score | Description |
|---|---|---|
| Good Recovery (GR) | 5 | Minor disabilities, but able to resume normal life |

(continued)

| Outcome | Score | Description |
|---|---|---|
| Moderate Disability (MD) | 4 | More significant disabilities, but still able to live independently. Can use public transportation, work in an assisted situation, etc. |
| Severe Disability (SD) | 3 | Conscious, but dependent upon others for daily care. Often institutionalized. |
| Persistent Vegetative State (PVS) | 2 | Not conscious, though eyes may be open and may "track" movement |
| Death | 1 | Self-explanatory |

[1]Teasdale, G. and Jennett, B. Lancet (1974) 81-84; Teasdale, G. and Jennett, B. Acta Neurochir. (1976) 34: 45-55.

For patients with closed head injuries, those with mild head injury (usually defined as GCS score on admission of 13-15) tend to do well. They may experience headaches, dizziness, irritability, or similar symptoms, but these gradually improve in most cases. Patients with moderate head injuries fare less well. Approximately 60% will make a positive recovery, and an estimated 25% will be left with a moderate degree of disability. Death or a persistent vegetative state is the outcome in about 7-10% of cases. The remainder of patients will have a severe degree of disability.

Despite its usefulness, the GOS is not a good tool to measure subtle emotional or cognitive problems.

Other tools are available for evaluating athletes for concussion.

For example, the Sports Concussion Assessment Tool (SCAT5) is a standardized tool for evaluating athletes for concussion aged 13 years and older for use by physicians and licensed healthcare professionals. For children aged 12 years or younger, Child SCAT5 is recommended.

The BESS test (Balance Error Scoring System) is a balance assessment protocol developed specifically for assessing sports concussions. It is part of the Sport Concussion Assessment Tool (SCAT) developed by the Concussion in Sport Group as a standard protocol for concussion testing. It includes interpretation of the Romberg test, which consists of balance tests that were originally introduced in 1853. Although the BESS test is generally accepted by the clinical research community, some concerns have been raised about inter- and intra-rater reliability of the manual scoring. Pressure measuring mat systems like Tekscan's MobileMat™, combined with SportsAT™ software, aim to minimize the chances of human error by automating BESS test scoring. MobileMat serves as the firm surface on which test subjects stand for the test, and a foam pad can be placed on the mat for that portion of the test. The mat is 0.3 inches thick, and contains high-resolution pressure sensors, adding objective data to the BESS test.

ImPACT Applications, Inc. provides a suite of concussion assessment tools including a computerized battery of cognitive tests to assist in the management of concussions. For example, ImPACT version 4 is applicable from ages 12-80.

The King-Devick Test is a two minute rapid number naming assessment in which an individual quickly reads aloud single digit numbers and evaluates impairments of eye movements, attention and language function.

**Changes in cerebral glucose metabolism in the aftermath of TBI**

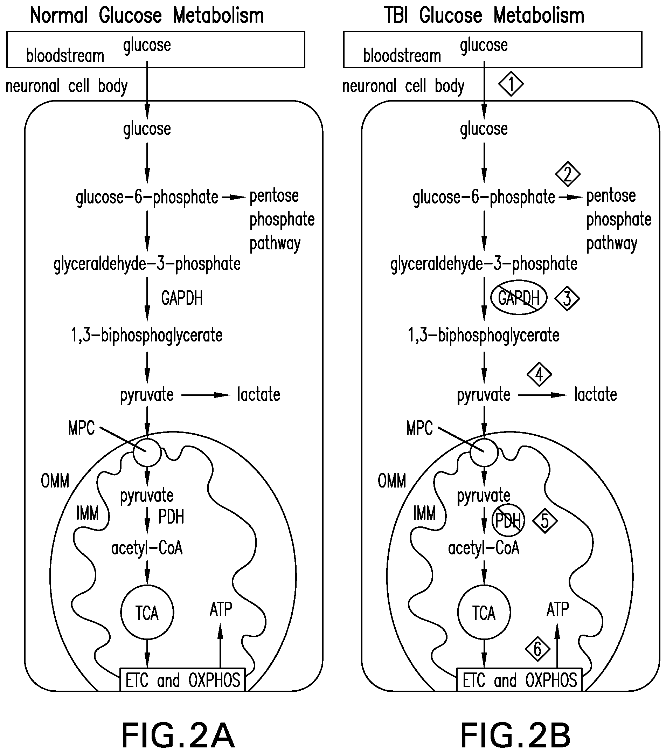

**FIG. 2A** and **FIG. 2B** illustrate normal glucose metabolism (**FIG. 2A**) and points of impairment in the glycolytic pathway and oxidative metabolism of glucose in the aftermath of TBI (**FIG. 2B**).

Upon impact, rapid movement of the brain within the skull initiates a series of neurochemical disruptions that alter cerebral metabolism. Within minutes after injury, the ionic equilibrium across the neuronal membranes is disrupted, with injury severity-dependent increases in the concentration of extracellular potassium and glutamate, as well as intracellular calcium accumulation [Prins, ML & Matsumoto, JH. The collective therapeutic potential of cerebral ketone metabolism in traumatic brain injury. J. Lipid Res. (2014) 55 (12): 2450-7, citing Fineman I., et al. Concussive brain injury is associated with a prolonged accumulation of calcium: a 45Ca autoradiographic study. Brain Res.(1993) 624: 94-102; Katayama Y., et al. Massive increases in extracellular potassium and the indiscriminate release of glutamate following concussive brain injury. J. Neurosurg.(1999) 73: 889-900]. This disruption of ionic equilibrium requires cellular energy to reestablish homeostasis, which is reflected by increases in cerebral glucose uptake observed within 30 min after adult rodent fluid percussion (FP) injury [Id., citing Yoshino A., et al. Dynamic changes in local cerebral glucose utilization following cerebral conclusion in rats: evidence of a hyper- and subsequent hypometabolic state. Brain Res. (1991) 561: 106-119 and within 8 days after human TBI [Id., citing Bergsneider M., et al. 1997. Cerebral hyperglycolysis following severe traumatic brain injury in humans: a positron emission tomography study. J. Neurosurg. (1997) 86: 241-251. This transient increase in glucose uptake is also known as "hyperglycolysis" and is followed by a prolonged period of glucose metabolic depression. These cerebral metabolic changes are a hallmark response described in both experimental and clinical brain trauma.

Experimental studies have shown that the magnitude and duration of the glucose metabolic depression increases with injury severity and age [Id., citing Prins, M. et al. Mapping cerebral glucose metabolism during spatial learning: interactions of development and traumatic brain injury. J. Neurotrauma. (2001) 18: 31-46; Thomas, S. et al. Cerebral metabolic response to traumatic brain injury sustained early in development: a 2-deoxy-D-glucose autoradiographic study. J. Neurotrauma. (2000) 17: 649-665; Hovda, DA et al. Long-term changes in metabolic rates for glucose following mild, moderate, and severe concussive head injuries in adult rats. Soc. Neurosci. Abstract (1994). 20: 845.

Other biochemical changes that occur in the aftermath of TBI further disrupt glucose uptake and metabolism. For instance, proton NMR spectroscopy of [1,2-$^{13}$C]-labeled glucose demonstrates a 9-12% increase in glucose processing through the pentose phosphate pathway between 3 and 24 h after controlled cortical impact (CCI) injury, thereby decreasing the available glucose supply for energy production [Id., citing Bitnik, BL et al. Upregulation of pentose phosphate pathway and preservation of tricarboxylic acid cycle flux after experimental brain injury. J. Neurotrauma (2005). 22: 1052-1065]. TBI also has been shown to generate early increases in reactive oxygen species (ROS) which damage lipids, protein, and DNA [Id., citing Hall, ED et al. Brain hydroxyl radical generation in acute experimental head injury. J. Neurochem. (1993) 60: 588-594; Althaus, JS et al. The use of salicylate hydroxylation to detect hydroxyl radical generation in ischemic and traumatic brain injury. Reversal by tirilazad mesylate (U-74006F). Mol. Chem. Neuropathol.(1993) 20: 147-162; Marklund, N. et al. Effects of the nitrone radical scavengers PBN and S-PBN on in vivo trapping of reactive oxygen species after traumatic brain injury in rats. J. Cereb. Blood Flow Metab. (2001) 21: 1259-1267; Sen, S. et al. Oxypurinol inhibits free radical release from the cerebral cortex of closed head injured rats. Neurosci. Lett. (1993) 162: 117-120; Sens, S. et al. Alpha-phenyl-tert-butyl-nitrone inhibits free radical release in brain concussion. Free Radic. Biol. Med (1994). 16: 685-691]. ROS-induced DNA damage activates DNA repair enzymes, such as poly-ADP ribose polymerase (PARP). In the presence of DNA strand breaks, pathological activation of PARP depletes cytosolic NAD$^+$, which ultimately inhibits glycolytic processing of glucose at the glyceraldehyde phosphate dehydrogenase step [Id., citing Sheline, C. et al. Zinc-induced cortical neuronal death: contribution of energy failure attributable to loss of NAD(+) and inhibition of glycolysis. J. Neurosci. (2000) 20: 3139-3146. Collectively, these series of biochemical changes divert and obstruct processing of glucose through the glycolytic pathway. The consequent decrease in glucose oxidation and ATP concentrations [Id., citing Singh, IN et al. Time course of post-traumatic mitochondrial oxidative damage and dysfunction in a mouse model of focal traumatic brain injury: implications for neuroprotective therapy. J. Cereb. Blood Flow Metab.(2006) 26: 1407-1418; Lee, S. et al. Evidence for energy failure following irreversible traumatic brain injury. Ann. N. Y. Acad. Sci. (1999) 893: 337-340 make glucose an inefficient energy substrate in the post-TBI brain.

**TBI elicits inflammatory responses in the brain**

Damage to the CNS also elicits inflammatory responses from resident microglia and macrophages, as well as peripheral immune cells, such as neutrophils, monocytes, and T cells. Microglia and resident macrophages immediately respond to injury after sensing damage-associated molecular patterns (DAMPs), such as the presence of adenosine triphosphate (ATP) or intracellular proteins that are released from damaged or dying cells. Signaling from DAMP receptors initiates local cytokine and chemokine production, which affects the immediate environment and provides a cue for peripheral immune infiltration ( Russo, M. and McGavern, DB, "Inflammatory neuroprotection following traumatic brain injury", Science (2016) 353 (6301): 783-6, citing Corps, KN. Et al. JAMA Neurol. (2015) 72: 355-62).

Within the CNS, positive aspects of immune cells that mobilize in response to damage include clearing dead cells, supporting the barrier system, and setting the stage for wound healing. Microglia, monocytes, macrophages, neutrophils, and T cells can collectively orchestrate a response that preserves neural tissue and fosters regeneration.

In general, microglia are critical participants in the acute phase of a CNS injury. Sealing barriers and clearing debris are just two benefits of having these cells involved in the response. Because of their ubiquitous presence and abundant DAMP sensors, microglia are usually among the first responders to brain damage. For example, microglia express several purinergic receptors that allow them to quickly respond to extracellular purines, such as ATP. Detection of extracellular ATP causes microglia to project processes toward a site of damage within minutes. Microglia also participate in immediate CNS barrier support following damage of cerebral capillaries (Id., citing Lou, N. et al. Proc. Natl Acad. Sci. USA (2016) 113: 1074-9). In addition to providing barrier support, microglia and macrophages can also clear debris from the injured CNS.

Despite the various protective layers separating the CNS from peripheral tissues, immune cells infiltrate the CNS in response to trauma. In a model of spinal cord injury, neutrophil recruitment was necessary for proper wound healing and repair (Id., citing Stirling, DP et al. J. Neurosci. (2009) 29: 753-64). Mice treated with neutrophil-depleting antibodies exhibited decreased astrocyte reactivity at the injury site, larger lesions, and worse neurological outcomes. Neutrophils are also recruited to the meninges in a purinergic receptor-dependent manner following acute brain injury, and interference with this response increased the amount of meningeal cell death (Id., citing Roth, TL et al. Nature (2014) 505: 223-8). It is presently unclear how neutrophils contribute to neuroprotective responses following CNS injury, but these cells do have the ability to recruit peripheral monocytes within a day or two. Spinal cord injury elicits a marked recruitment of pro-inflammatory macrophages that is followed several days later by wound-healing macrophages (Id., citing Shechter, R. et al. Immunity (2013) 38: 555-69). When this wound-healing response was blocked, mice were unable to properly repair the lesion and recover motor skills (Id., citing Shechter, R. et al. Immunity (2013) 38: 555-69). Similar to peripheral tissues, innate immune cells are essential for tissue remodeling and repair in the CNS.

T cells can also play a positive role in CNS injury responses. CNS damage can promote the nonspecific recruitment of CD4+ T cells that produce interleukin-4 (IL-4) in a major histocompatibility complex II-independent manner (Id., citing Walsh, JT et al. J. Clin. Invest. (2015) 125: 699-714). Release of IL-4 potentiates neurotrophin signaling that helps stimulate axonal regrowth after injury. Mice lacking T cells or IL-4 demonstrated increased neuronal loss and neurological dysfunction following CNS injury. During CNS tissue repair, effector and regulatory CD4+ T cells work in tandem. Regulatory T cells (Tregs) often keep immune responses in check by modulating inflammatory mediators to promote wound healing and remodeling. Depletion of Tregs before CNS injury increased the recruitment of effector CD4+ T cells and improved neurological recovery (Id., citing Raposo, C. et al. J. Neurosci. (2014) 34: 10141-55). By contrast, Treg depletion several days after injury interfered with the tissue repair process. Similarly to the transition from proinflammatory to wound-healing macrophages, these Treg data show that the neuroinflammatory response to injury changes as time progresses.

At present, treatments to prevent nerve damage or promote nerve healing after TBI are not available. Efforts to halt neuroinflammation, although successful for the treatment of multiple sclerosis, have failed in other conditions, because anti-inflammatory strategies block not only the detrimental but also the beneficial side of inflammation.

**Metabolism relationship of ketogenic diets, beta-hydroxybutyrate (BHB), to cell metabolism and energy,**

Metabolism is the sum of the chemical and physical changes occurring in tissue. It consists of anabolism, those reactions that convert small molecules into large, and catabolism, those reactions that convert large molecules into small.'

**Catabolism**

Under normal circumstances, the proteins, lipids and polysaccharides that make up the majority of the food we eat must be broken down into smaller molecules before our cells can use them. The enzymatic breakdown, or catabolism, of these molecules proceeds in three stages. In stage 1, large polymeric molecules are broken down into their monomeric subunits - proteins into amino acids, polysaccharides into sugars, and fats into fatty acids and glycerol - by digestion. These processes occur mainly outside cells through the action of secreted enzymes. In stage 2, the resultant small molecules enter cells and are further degraded in the cytoplasm. Most of the carbon and hydrogen atoms of sugars are converted into pyruvate, which then enters mitochondria, where it is converted to the acetyl groups of acetyl coenzyme A (Acetyl CoA). AcetylCoA, like ATP, is a chemically reactive compound that releases a great deal of energy when it is hydrolyzed. Major amounts of acetyl CoA are also produced by the oxidation of fatty acids. Stage 3 occurs when the acetyl group of acetyl CoA is completely degraded to CO2 and H2O. Most of the ATP is generated in this final stage. Through the generation of ATP, the energy originally derived from the combustion of carbohydrates and fats is redistributed as a conveniently packaged form of chemical energy that is easily released. [Alberts, B. et al. Molecular Biology of the Cell (1983) Garland Publishing, NY, Ch. 2, pages 67-75].

The most important part of stage 2 of catabolism is glycolysis, meaning a sequence of reactions involving the splitting of glucose. In glycolysis, a glucose molecule with six carbon atoms is converted into two molecules of pyruvate, each with three carbon atoms by a sequence of nine enzymatic reactions that involve a series of phosphate-containing intermediates. At the end of glycolysis, the ATP balance sheet shows a net profit of two molecules of ATP per glucose molecule. For most animal cells, the pyruvic acid that is formed quickly enters the mitochondria to be completely oxidized to $CO_2$ and water.

In addition to pyruvate, fatty acids and some amino acids also pass from the cytosol into mitochondria where they are converted into acetyl CoA or one of the other intermediates of the citric acid cycle.

**Glucose homeostasis**

Glucose, a fundamental source of cellular energy, is released by the breakdown of endogenous glycogen stores that are primarily located in the liver. Glucose is also released indirectly in the muscle through intermediary metabolites. These whole-body energy stores are replenished from dietary glucose, which, after being digested and absorbed across the gut wall, is distributed among the various tissues of the body. (Bryant, NJ et al, Nat. Rev. Mol. Cell Biol. 2002: 3(4): 267-77).

Normally, following glucose ingestion, the increase in plasma glucose concentration triggers insulin release, which stimulates splanchnic (liver and gastrointestinal tissue) and peripheral glucose uptake and suppresses endogenous (primarily hepatic) glucose production. In healthy adults, blood glucose levels are tightly regulated within a range of 70 to 99 mg/dL, and maintained by specific hormones (e.g., insulin, glucagon, incretins) as well as the central and peripheral nervous system, to meet metabolic requirements. Various cells and tissues (within the brain, muscle, gastrointestinal tract, liver, kidney and adipose tissue) also are involved in blood glucose regulation by means of uptake, metabolism, storage and secretion. DeFronzo, RA., Med. Clin. N. Am. 88: 787-835 (2004); Textbook of Medical Physiology; Gerich, JE, Diabetes Obes. Metab. 2000: 2: 345-350. Under normal physiologic circumstances, glucose levels rarely rise beyond 140 mg/dL, even after consumption of a high-carbohydrate meal.

Insulin, a potent antilipolytic (inhibiting fat breakdown) hormone, is known to reduce blood glucose levels by acceleratingtransport of flucose into insulin-sensitive cells and facilitating its conversion to storage compounds via glycogenesis (conversion of glucose to glycogen) and lipogenesis (fat formation). Glucagon, which also plays a role in glucose homeostasis, is produced in response to low normal glucose levels or hypoglycemia, and acts to increase glucose levels by accelerating glycogenolysis and promoting gluconeogenesis. After a glucose-containing meal, glucagon secretion is inhibited by hyperinsulinemia, which contributes to suppression of hepatic glucose production and maintenance of normal postprandial glucose tolerance.

Incretins, which include glucose-dependent insulinotropic polypeptide (GIP) and glucagon-like peptide 1 (GLP-1), are also involved in regulation of blood glucose, in part by their effects on insulin and glucagon. (Drucker, and Nauk, Lancet 368: 1696-1705 (2006). However, both GLP-1 and GIP are considered glucose-dependent hormones, meaning they are secreted only when glucose levels rise above normal fasting plasma glucose levels. Normally, these hormones are released in response to meals and, by activating certain receptors on pancreatic $\beta$ cells, they aid in stimulation of insulin secretion. When glucose levels are low, however, GLP-1 and GIP levels (and their stimulating effects on insulin secretion) are diminished. Drucker, Cell Metab. 3: 153-165 (2006).

**Ketosis**

As an alternative to glucose utilization, the body can metabolically flex into a state of ketosis. Ketosis relies on fat-derived ketones produced in the liver to provide fuel to nearly every cell in the body. Ketones-acetoacetate, beta-hydroxybutyrate, and acetone-are water-soluble molecules produced by the liver from fatty acids when blood glucose and liver glycogen stores have been minimized. Glycogen depletion occurs and ketone levels rise during periods of fasting, low carbohydrate intake, intense exercise, starvation, or due to complete lack of insulin in untreated type I diabetes. [Gershuni, VM et al. Curr. Nutr. Rep. (2018) 7 (3): 97-106].

Hormonal activation of lipolysis and ketogenesis is mediated by epinephrine and glucagon and opposed by insulin. With minimal dietary carbohydrate, insulin is low and glucagon increases. In addition to stimulating glycogenolysis in the liver, glucagon stimulates lipolysis to release stored fatty acids from adipose tissue. [Id., citing Sato, K. et al. Insulin, Ketone bodies and mitochondrial energy transduction. FASEB J. (1995) 9: 651-58]. In addition to forming ketones, fatty acids can be converted to acetyl CoA-an intermediary substrate between fatty acid oxidation and metabolism of glucose-that enters the citric acid cycle and then undergoes oxidative phosphorylation for ATP generation. Conversely, in response to high blood glucose (i.e. after a high carbohydrate meal), insulin levels rise and shut off ketogenesis in favor of de novo lipogenesis (fat storage). Thus, ketosis signifies a shift from an insulin-mediated glucose dependent state to an increased ability to use dietary fat and adipose stores for fuel.

**Nutritional ketosis**

Nutritional ketosis is the intentional restriction of dietary carbohydrate intake to accelerate the production of ketones and induce a metabolic effect that stabilizes blood sugar, minimizes insulin release, and thereby mitigates the downstream anabolic and tumorigenic effects of longstanding insulin resistance. As described by Volek and Phinney, a "well-formulated" ketogenic diet is composed of 5-10% carbohydrates (<20-50g/day), adequate protein (1-1.5g/kg/day), and fat until satiated. The hallmark of nutritional ketosis is blood ketone levels of 0.5 to 3 mg/dL.[Id., citing Volek, JS and Phinney SD. The Art and Science of Low Carbohydrate Living (Beyond Obesity, LLC, Miami FL USA]. This is in stark contrast to, and should not be confused with, the pathophysiologic state of type 1 diabetic ketoacidosis (DKA). Despite similar sounding names, they are two distinct metabolic processes. The production of endogenous insulin is protective against the occurrence of DKA; the range of ketones present in DKA is 5-10 fold greater than the levels achieved during nutritional ketosis. Additionally, while in nutritional ketosis, the body is able to maintain normal blood glucose levels and maintain a normal pH, as opposed to extremely elevated blood sugars and acidic pH associated with DKA.

After many weeks, "keto-adaptation" occurs, which signifies the body's ability to adapt and respond to primarily using ketones for fuel. One of the potential reasons this occurs is secondary to upregulated transcription of genes that encode the metabolic machinery resulting in increased mitochondrial density in oxidative tissues like the brain and muscle. Mouse studies suggest that this may occur via increased mitogenesis or decreased mitochondrial damage.[Id., citing Bough, KJ et al. Mitochondrial biogenesis in the anticonvulsant mechanism of the ketogenic diet. Ann. Neurol. (2006) 60: 223-35; Ahola-Erkkila, S. et al. Ketogenic diet slows down mitochondrial myopathy progression in mice. Hum. Mol. Genet.

(2010) 19: 1974-84] Additionally, ketones are capable of inducing epigenetic regulation via histone deacetylation inhibition indicating that they are a signaling molecule in addition to an energy source.[Id., citing Newman, JC and Verldin E. Beta-hydroxybutyrate: much more than a metabolite. Diabetes Res. Clin. Pract. 2014] 106: 173-81].

Through this process, fat-derived energy is generated in the liver and then shipped throughout the body to supply energy to the brain, renal cortex, heart, and skeletal muscles.[Id., citing Laffel, L. Ketone Bodies: a review of physiology, pathophysiology and application of monitoring to diabetes. Diabetes Metab. Res. Rev. (1999) 15: 412-26]. Ketones can supply up to 60% of ATP required by the body [Id., citing Veech, RL et al. Ketone bodies, potential therapeutic uses. IUBMB Life (2001) 51: 241-47]; the remainder is derived from endogenous gluconeogenesis that utilizes glycerol form triglycerides and glucogenic amino acids from protein for glucose production. Ketones cross the blood brain barrier and replace glucose as the primary energy source for the brain. Pioneering work by George Cahill using models of starvation ketosis revealed that the brain has metabolic flexibility and can switch from being a glucose dependent organ (~150g/day) to one that derives over 2/3rd of its energy from ketones.[Id., citing Veech, RL et al. Ketone bodies, potential therapeutic uses. IUBMB Life (2001) 51: 241-47; Westman, EC et al. Low-carbohydrate nutrition and metabolism. Am. J. Clin. Nutr. (2007) 86: 276-84]. The primary feature of ketogenic diets is the establishment of ketosis and stabilization of insulin levels, which addresses the biomarkers of metabolic syndrome [Id., citing Volek, JS et al. Carbohydrate restriction has a more favorable impact on the metabolic syndrome than a low-fat diet. Lipids (2009) 44: 297-309; Volek, JS & Feinman, RD. Carbohydrate restriction improves the features of Metabolic syndrome. Metabolic Syndrome may be defined by the response to carbohydrate restriction. Nutr. Metab. (Lond) (2005) 2: 31I]. By reducing insulin elevations, lipids are released from storage and oxidized. [Id., citing Volek JS, et al. Dietary carbohydrate restriction induces a unique metabolic state positively affecting atherogenic dyslipidemia, fatty acid partitioning, and metabolic syndrome. Prog Lipid Res (2008) 47, 307-318.

Intravenous ketone administration however may be an inefficient method for inducing changes in cerebral energy metabolism [Prins, ML and Matsumoto, JH. J. Lipid Res. (2014) 55: 2450-55, citing Pan J. W., et al (2001). Measurement of beta-hydroxybutyrate in acute hyperketonemia in human brain. J. Neurochem. 79: 539-544; White, H. et al. Effect of a hypertonic balanced ketone solution on plasma, CSF and brain beta-hydroxybutyrate levels and acid-base status. Intensive Care Med. (2013) 39: 727-733; Pan, J. et al. Human brain beta-hydroxybutyrate and lactate increase in fasting-induced ketosis. J. Cereb. Blood Flow Metab. (2000) 20: 1502-1507]. Strategies designed to primarily increase plasma ketone levels must also target increased ketone transport across the blood brain barrier (BBB) by monocarboxylate transporters (MCTs), membrane proteins acting as carriers for lactate, pyruvate and ketone bodies. Moreover, the high dose required to achieve desired ketone levels can lead to severe side effects involving insulin/glucagon balance. [Id, citing Clarke, :K. et al. Kinetics, safety and tolerability of (R)-3-hydroxybutyl (R)-3-hydroxybutyrate in healthy adult subjects. Regul. Toxicol. Pharmacol. (2012) 63: 401-408; van Delft, R. et al. Blood beta-hydroxybutyrate correlates better with seizure reduction due to ketogenic diet than do ketones in the urine. Seizure. (2010) 19: 36-39; Madison, LL, et al. The hypoglycemic action of ketones. II. Evidence for a stimulatory feedback of ketones on the pancreatic beta cells. J. Clin. Invest. (1964) 43: 408-415].

## HCA2 and its role in protecting against ischemic brain injury

HCA2 is a G protein-coupled receptor originally named GPR109A that belongs to the hydroxyl carboxylic acid (HCA) receptor family. [Offermanns, S. and Schwaninger, M. Nutritional or pharmacological activation of HCA2 ameliorates neuroinflammation. Trends in Molec. Med. (2015) 21 (40): 345-55, citing Offermanns, S. et al. (2011) Pharmacol. Rev. (2011) 63: 269-90]. It couples to G proteins of the G family [Id., citing Soga, T. et al. Molecular identification of nicotinic acid receptor. Biochem. Biophys. Res. Commun. (2003) 303: 364-69; Tunaru, S. et al. PUMA-G and HM74 are receptors for nicotinic acid and mediate its anti-lipolytic effect. Nat. Med. (2003) (9): 352-55; Wise, A. et al. Molecular identification of high and low affinity receptors for nicotinic acid. J. Biol. Chem. (2003) 278: 9869-74; Taggart, AK et al. D-$\beta$-hydroxybutyrate inhibits adipocyte lipolysis via the nicotinic aid receptor PUMA-G. J. Biol. Chem. (2005) 280: 26649-52; Benyo, Z. et al. GPR109A (PUMA-G/HM74A) mediates nicotinic acid-induced flushing. J. Clin. Invest. (2005) 115: 3634-40] and is activated by the ketone body BHB and, with lower potency, by butyrate [Id., citing Taggart, AK et al. D-$\beta$-hydroxybutyrate inhibits adipocyte lipolysis via the nicotinic aid receptor PUMA-G. J. Biol. Chem. (2005) 280: 26649-52]. HCA2 is expressed as well in various immune cells including neutrophils, macrophages, epidermal Langerhans cells, dendritic cells and microglia, but not in lymphocytes [Id., citing Benyo, Z. et al. GPR109A (PUMA-G/HM74A) mediates nicotinic acid-induced flushing. J. Clin. Invest. (2005) 115: 3634-40; Kostylinn, G. et al. Neutrophil apoptosis mediated by nicotinic acid receptors (GPR109A). Cell Death Differ. (2005) 15: 134-42; Kostylina, G. et al. Neutrophil apoptosis mediated by nicotinic acid receptors (GPR109A). Cell Death Differ. (2008) 15: 134-42; Maxiejewski-Lenoir, D. et al. Langerhans cells release prostaglandin D2 in response to nicotinic acid. J. Invest. Dermatol. (2006) 126: 2637-46; Rahman, M. et al. The $\beta$-hydroxybutyrate receptor HCA2 activates a neuroprotective subset of macrophages. Nat. Commun. (2014) 5: 3944; Schaub, A. et al. PUMA-G, an IFN-$\gamma$-inducible gene in macrophages is a novel member of the seven transmembrane spanning receptor superfamily. Eur. J. Immunol. (2001) 31: 3714-25; Singh, N. et al. Activation of Gpr109a, receptor for niacin and the commensal metabolite butyrate, suppresses colonic inflammation and carcinogenesis. Immunity (2014) 40:

128-39]. BHB, the main endogenous ligand of HCA2, activates human HCA2 with an EC50 of about 700 $\mu$M [Id., citing Taggart, AK et al. D-$\beta$-hydroxybutyrate inhibits adipocyte lipolysis via the nicotinic aid receptor PUMA-G. J. Biol. Chem. (2005) 280: 26649-52], a concentration that this ketone body reaches only after fasting or on a ketogenic diet. HCA2 also can mediate anti-inflammatory and immunomodulatory effects through its expression by immune cells, and it has been suggested that this activity, when induced by ketone bodies during starvation, helps to preserve energy. [Id. citing Lukasova, M. et al. Nicotinic acid (niacin): new lipid-independent mechanisms of action and therapeutic potentials. Trends Pharmacol. Sci. (2011) 32: 700-7].

Recent evidence indicates that HCA2 can mediate the neuroprotective effect of BHB [Id., citing Rahman, M. et al. The $\beta$-hydroxybutyrate receptor HCA2 activates a neuroprotective subset of macrophages. Nat. Commun. (2014) 5: 3944]. While the ketogenic diet is established as a treatment for childhood epilepsy [Id., citing Levy, RG et al. Ketogenic diet and other dietary treatments for epilepsy. Cochrane Database Syt. Rev. (20120 3: CD001903], preclinical studies have provided evidence that a ketogenic diet can also be protective in experimental models of other neurological diseases, including TBI. Research into the therapeutic potential of the ketogenic diet has been driven by the notion that it improves the energy balance of the diseased brain. However, BHB is not only a metabolic intermediate, but is also a signaling molecule that activates HCA2 and inhibits GPR41 and histone deacetylase (HDAC) 1, 3, and 4 [Id., citing Newman, JC and Verdin, E. Ketone bodies as signaling metabolites. Trends Endocrinol. Metab. (2014) 25: 42-52; Won, Y-J et al. $\beta$-hydroxybutyrate modulates N type calcium channels in rat sympathetic neurons by acting as an agonist for the G protein coupled receptor FFAS. J. Neurosci. (2013) 33: 19314-25], which led to the hypothesis that BHB induces neuroprotective effects through specific actions on receptors or enzymes. A recent study of a ketogenic diet in a mouse model of stroke supported a role for HCA2 in the BHB effect of this disease model [Id., citing Rahman, M. et al. The $\beta$-hydroxybutyrate receptor HCA2 activates a neuroprotective subset of macrophages. Nat. Commun. (2014) 5: 3944]. These investigators concluded that the extra supply of an energy substrate is not sufficient to explain the protection provided by BHB; rather, activation of $HCA_2$ on monocyte-derived cells, neutrophils or microglia is required to ameliorate brain damage. Without being limited by theory, HCA2 activation may be a therapeutic principle to treat brain disorders with a neuroinflammatory component. For example, it has been hypothesized that HCA2 on monocyte derived cells may provide a target to stimulate neuroprotective Ly6C[lo] monocyte-derived cells in the brain. Lymphocyte antigen 6 complex, (Ly6C) is a marker of macrophage subsets. Ly6C[hi] monocytes have proinflammatory and antimicrobial functions and express high levels of C-C chemokine receptor 2 (CCR2) and low levels of CX3C chemokine receptor 1 (CX3CR1). [Kratofil, RM et al. "Monocyte Conversion During Inflammation and Injury. Arteriosclerosis, Thrombosis and Vascular Biology (2017) 37 (1): 35-42, citing Geissmann, F. et al. Blood monocytes consist of two principal subsets with distinct migratory properties. Immunity (2003) 19: 71--82]. Ly6C[low] monocytes, also known as patrolling monocytes, survey the vasculature by constantly crawling along the lumen of the vasculature and are involved with early responses to inflammation and tissue repair.

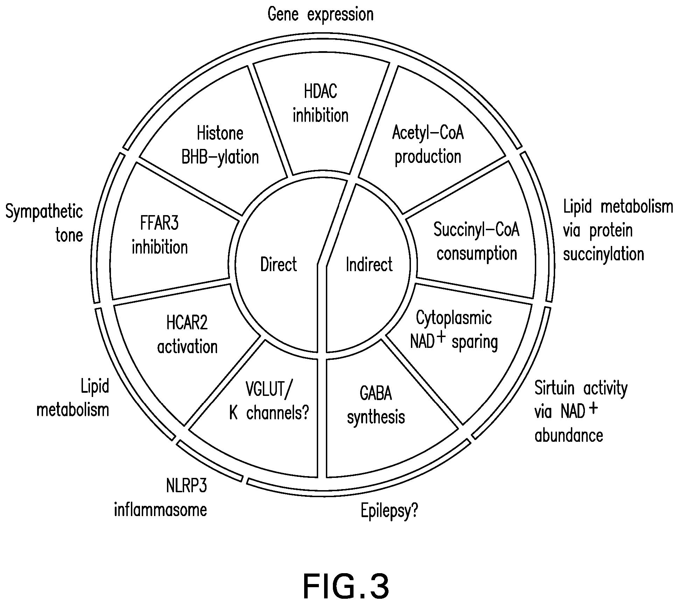

FIG. 3 schematically illustrates direct and indirect signaling effects of BHB. In addition to the direct signaling effects of BHB, indirect signaling mechanisms augment the impact of BHB through downstream effects seen in molecules at least one degree removed. In contrast to direct signaling functions which are attributable to the BHB compound itself, indirect signaling functions require catabolism to other molecules, Abbreviations: BHB, $\beta$-hydroxybutyrate; CoA, coenzyme A; FFAR3, free fatty acid receptor 3; GABA, $\gamma$-amino-butyric acid; HDAC, histone deacetylase; HCAR2, hydroxycarboxylic acid receptor 2; NAD, nicotinamide adenine dinucleotide; VGLUT, vesicular glutamate transporter. [Taken from Newman JC and Verdin, E. $\beta$-hydroxybutyrate: a signaling metabolite. Ann. Rev. Nutrition (2017) 37: 51-76].

Beta hydroxybutyrate (BHB) has a molecular weight of 104.1 g/mol. As a small, polar molecule, BHB is readily soluble in water and blood. [Newman, JC and Verdin, E $\beta$-Hydroxybutyrate. Annu. Rev. Nutr. (2017) 37: 51-76]. BHB is a chiral molecule at the 3' hydroxyl group. There are two enantiomers, R/d and S/l, typically denoted as D and L respectively. The human liver produces the D enantiomer of the molecule [Chriett, S. et al. Prominent action of butyrate over $\beta$-hydroxybutyrate as histone deactylase inhibitor, transcriptional modulator and anti-inflammatory molecule. Sci. Reports (2019) 9(1): 742].. R-BHB is the normal product of human and mouse metabolism. S-BHB itself is not a normal product of human metabolism. Experiments involving infusions of labeled R-BHB, S-BHB, or mixtures thereof into rats or pigs found that S-BHB is converted mostly to R-BHB (Newman, JC and Verdin, E $\beta$-Hydroxybutyrate. Annu. Rev. Nutr. (2017) 37: 51-76, citing Lincoln, BC et al. Arch. Biochem. Biophys. (1987) 259: 149-56); the molecular pathway for this is not known, but it may occur through conversion of S-BHB to acetyl-CoA and then production of R-BHB from acetyl-CoA. At least some of the S-BHB is eventually converted to $CO_2$, presumably also after being metabolized to acetyl-CoA. Data has shown that, at the same dose, the D enantiomer is superior at elevating D-beta hydroxybutyrate levels when compared to a racemic mixture of the molecule [Cuenoud, B. et al. Metabolism of exogenous D-beta-hydroxybutyrate, an energy substrate avidly consumed by the heart and kidney. Frontiers Nutrition (2020) 7: 13]. Many of the currently available exogenous forms of beta hydroxybutyrate are racemic mixtures, salt forms, or variations of the true molecule, such as 1,3-butanediol.

Data indicates that the effectiveness of D-beta hydroxybutyrate with respect to neurological indications is limited by the rate at which D-beta hydroxybutyrate can be transported across the BBB to sufficiently increase D-beta hydroxybutyrate concentrations in the brain. [White, H. et al. A systematic review of intravenous $\beta$-hydroxybutyrate use in humans - a promising future therapy? Frontiers Medicine (2021) 8: 740374]. Several monocarboxylic acid transporters, including MCT1 and MCT2, carry BHB across the blood-brain barrier (Pellerin, L. et al. J. Neurosci. Res. (2005) 79: 55-64), and their expression can regulate brain BHB uptake (Id., citing Barton, KM, and Palmer, SE (2016) Curr. HIV/AIDS Rep. (2016) 13: 77-84).

The present disclosure provides a targeted approach for delivery of a synthetic D-beta hydroxybutyrate to the brain that bypasses the BBB, wherein controlled release of the beta-hydroxybutyrate may lead to increased D-beta hydroxybutyrate levels in the brain both immediately upon administration and over time.

BRIEF DESCRIPTION OF THE DRAWINGS

**FIG. 1** depicts the cascade of negative effects in the brain of a traumatic insult. Primary injuries result within the first few seconds after impact and are generally mechanical in nature. Secondary injuries occur in the minutes to weeks following the impact and affect the brain's ability to properly modulate ion balance, metabolism, and inflammation. Tertiary injuries, or neuropathologies, occur weeks to years following the impact and are thought to be driven by the energy crisis and oxidative stress of the secondary injuries Taken from Daines, SA et al. The therapeutic potential and limitations of ketones in traumatic brain injury. Frontiers Neurology (2021), 12: 723148, **FIG. 1.**

**FIG. 2A** and **FIG. 2B** are a schematic comparison of glucose metabolism in normal (**FIG. 2A**) and post-TBI (**FIG. 2B**) physiology. The glucose metabolism pathway is impaired at six points in the glycolytic pathway and oxidative metabolism of glucose after TBI: (1) transient increase in glucose uptake followed by a prolonged decrease in glucose metabolism; (2) more glucose-6-phosphate is shunted to the pentose phosphate pathway to generate protective and reparative molecules. (3) glyceraldehyde-3-phosphate dehydrogenase activity is slowed, decreasing tricarboxylic acid cycle intermediates. (4) Lactate production increases in astrocytes to shuttle lactate to energy-deficient neurons. (5) Pruvate dehydrogenase complex is inhibited, decreasing tricarboxylic acid cycle intermediates;. (6) impairment of electron transport chain function and oxidative phosphorylation resulting in diminished ATP production. Taken from Daines, SA et al. The therapeutic potential and limitations of ketones in traumatic brain injury. Frontiers Neurology (2021), 12: 723148, **FIG. 2.**

**FIG. 3** schematically illustrates direct and indirect signaling effects of BHB. Abbreviations: BHB, β-hydroxybutyrate; CoA, coenzyme A; FFAR3, free fatty acid receptor 3; GABA, γ-amino-butyric acid; HDAC, histone deacetylase; HCAR2, hydroxycarboxylic acid receptor 2; NAD, nicotinamide adenine dinucleotide; VGLUT, vesicular glutamate transporter. Taken from Newman JC and Verdin, E. β-hydroxybutyrate: a signaling metabolite. Ann. Rev. Nutrition (2017) 37: 51-76.

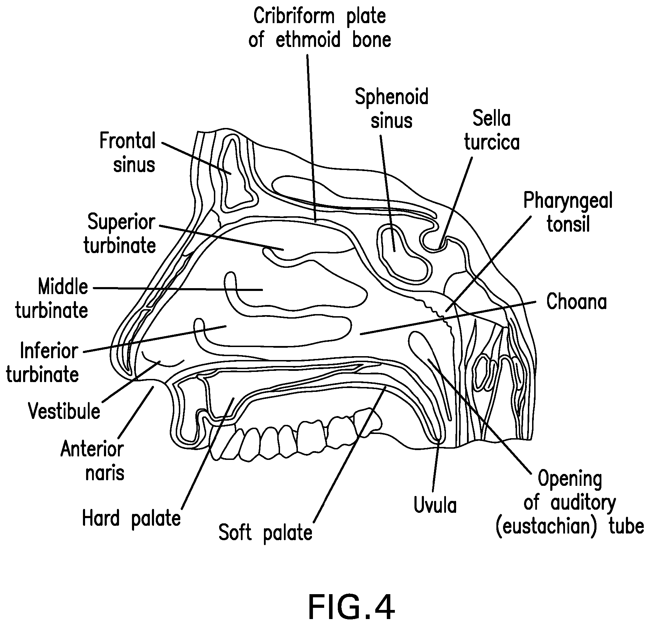

**FIG. 4** is an illustration showing internal nasal anatomy in cross section.[taken from Janfaza, P. et al. Surgical anatomy of the head and neck., Harvard University Press (June 15, 2011), Chapter 5.

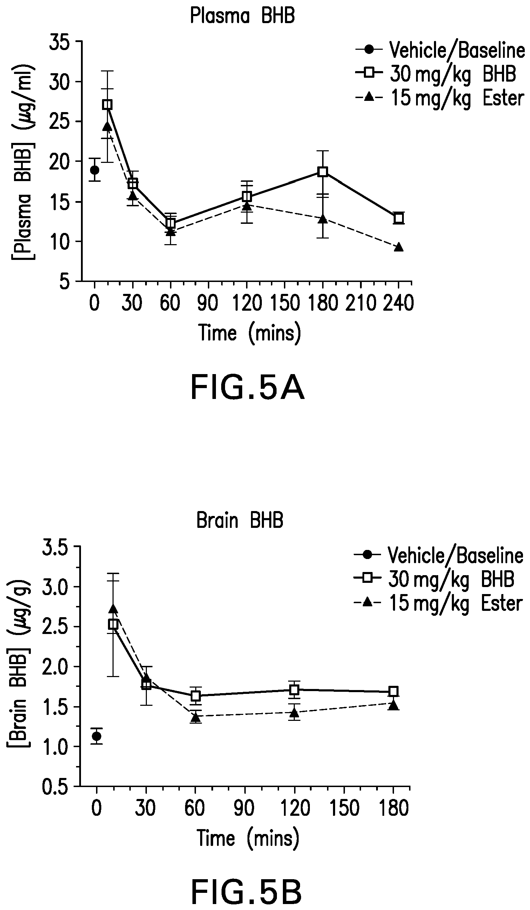

**FIG. 5A** is a graph of beta-hydroxybutyrate (BHB) concentration (μg/mL, y axis) in plasma on Day 14 over time (h) (x axis) following intranasal administration to C57BL/6J mice for Group 1 (vehicle), Group 2 (30 mg/kg BHB salt); Group 3 (BHB ester (15 mg/kg). **FIG. 5B** is a graph of beta-hydroxybutyrate (BHB) concentration (μg/mL, y axis) in brain on Day 14 over time (h) (x axis) following intranasal administration to C57BL/6J mice for Group 1 (vehicle), Group 2 (30 mg/kg BHB salt); Group 3 (BHB ester (15 mg/kg).

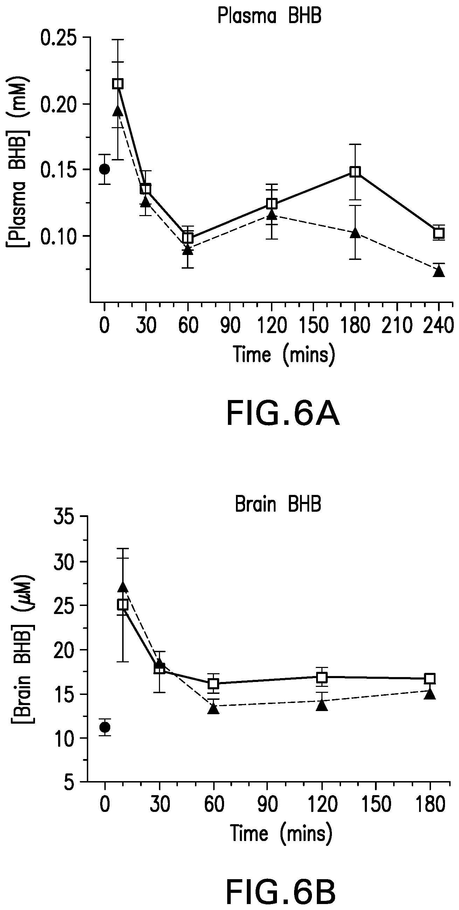

**FIG. 6A** is a graph of beta-hydroxybutyrate (BHB) concentration (ug/g, y axis) in plasma on Day 14 over time (h)(x axis) following intranasal administration to C57BL/6J mice for Group 1 (vehicle), Group 2 (30 mg/kg BHB salt); Group 3 (BHB ester (15 mg/kg). **FIG. 6B** is a graph of beta-hydroxybutyrate (BHB) concentration (ug/g, y axis) in brain on Day 14 over time (h)(x axis) following intranasal administration to C57BL/6J mice for Group 1 (vehicle), Group 2 (30 mg/kg BHB salt); Group 3 (BHB ester (15 mg/kg).

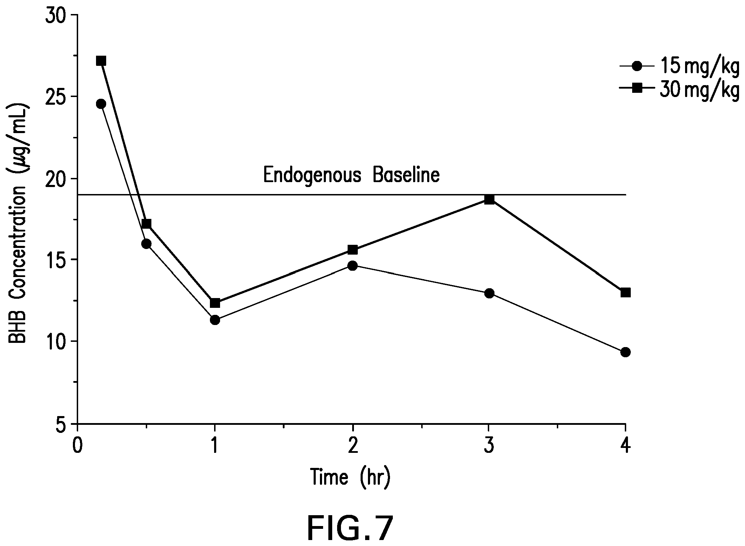

**FIG. 7** is a graph showing the endogenous baseline level of beta-hydroxybutyrate (BHB) (y-axis) over time (x-axis) when BHB is administered as 15 mg/kg and 30 mg/kg. Note that the endogenous baseline in **FIG. 7** is the same as the vehicle line in **FIG. 5A** (about 19 μg/mL).

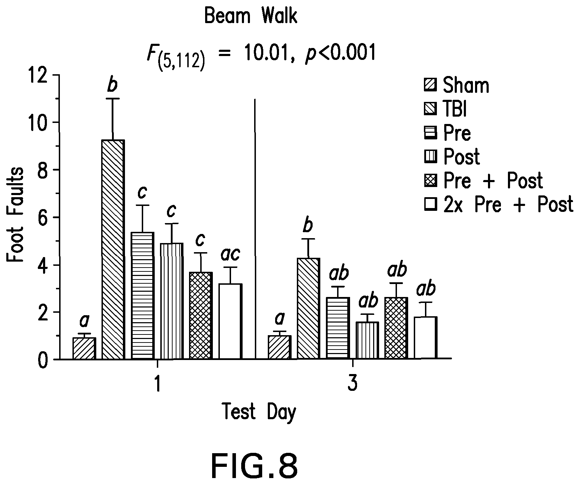

**FIG. 8** shows bar graphs of the results of the beam-walk behavioral assay after TBI or sham injury. The number of foot faults is plotted against the sample study group of C57BL/6J female mice, 20 animals per group. 20 μl of a standard stock solution (50 mg/kg) of the test compound R-(-)-3-hydroxybutyric acid sodium salt (Sigma,) (BHB) prepared fresh daily was administered daily intranasally starting from Day 0 immediately post-TBI through day 14. Saline was added to the compound to the appropriate concentration. Study groups were (1) sham injury, treated with vehicle; 2) TBI injury, treated with vehicle; (3) pretreated with BHB IN before TBI injury, and then treated with saline post-TBI injury;(4) pretreated with saline before TBI injury and then treated with BHB post-TBI injury; and (5) pretreated with BHB IN before TBI injury and then treated with BHB post-TBI injury; and pretreated with 2X dose BHB IN before TBI injury and then treated with BHB post TBO-injury. Sham injured mice remained sure-footed. TBI mice at day 1 showed the highest number of foot faults; by day 3 the number of foot faults in this group decreased by about 50%. The pretreated, post-treated, pre and post-treated, and 2x pre-and post-treated groups all showed improvement in beam walking at day 1 and at day 3. At day 3 all treated groups were significantly improved, with best results in the post and 2x pre + post groups. The 2x pre + post group saw a 70% improvement in foot faults 1 day after TBI.

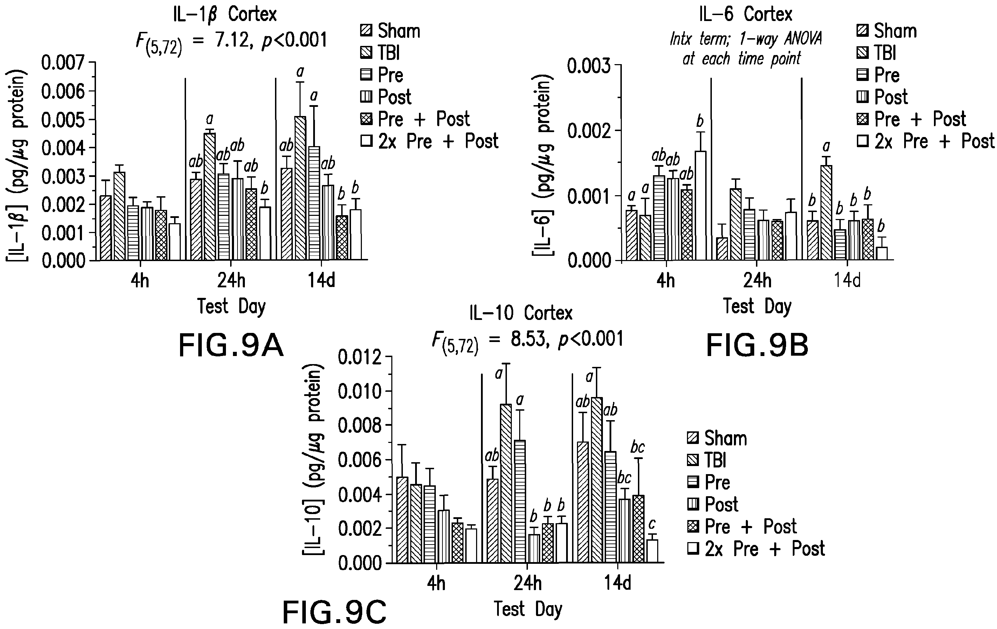

FIG. 9A - FIG. 9C shows pg/$\mu$g total protein of IL-1$\beta$ (FIG. 9A), IL-6 (FIG. 9B) and IL-10 (FIG. 9C) expressed in the cortex in each study group from FIG. 8. Results for TNF-$\alpha$ were not significant (data not shown). The data at the 24 h and 14 day time points in FIG. 9A, FIG. 9B and FIG. 9C show that treatment decreased expression of IL-1$\beta$, IL-6 and II-10. While IL-10 is generally thought of as an anti-inflammatory cytokine, it also may promote fibrotic processes [Steen, EH et al. Adv. Wound Care (New Rochelle) (2020) 9 (4): 184-98, citing O'Garra, A. et al. Immunol. Rev. (20008) 223: 114-31]. The data for the pre + post groups are consistent with the behavioral data.

SUMMARY OF THE INVENTION

According to one aspect, the present disclosure provides a composition comprising an active ingredient that is encapsulated in suspension of particles comprising a biodegradable polymer containing an effective amount of the active agent, wherein the particles are configured to bypass the blood brain barrier, wherein the active ingredient is synthetic D-beta hydroxybutyrate, and wherein targeted delivery is to the brain.

According to some embodiment of the composition, size of the particles is greater than 10 nm in diameter, greater than 15 nm in diameter; greater than 20 nm in diameter, greater than 30 nm in diameter, greater than 40 nm in diameter, greater than 50 nm in diameter, greater than 60 nm in diameter, greater than 70 nm in diameter, greater than 80 nm; in diameter, greater than 90 nm in diameter, greater than 100 nm in diameter, greater than 200 nm in diameter, greater than 300 nm in diameter, greater than 400 nm in diameter, greater than 500 nm in diameter, greater than 600 nm in diameter, greater than 700 nm in diameter, greater than 800 nm in diameter, greater than 900 nm in diameter, greater than 1000 nm in diameter, greater than 2000 nm in diameter, greater than 3000 nm in diameter, greater than 4000 nm in diameter, greater than 5000 nm in diameter, greater than 6000 nm in diameter, greater than 7000 nm in diameter, greater than 8000 nm in diameter, greater than 9000 nm in diameter, or greater than 10,000 nm in diameter. According to some embodiment of the composition, the synthetic D-beta hydroxybutyrate is dispersed throughout the particles; or the particles are impregnated with the D-beta hydroxybutyrate; or the particles comprise a matrix and the matrix comprises the D-beta hydroxybutyrate. According to some embodiment of the composition, integration of the synthetic D-beta-hydroxybutyrate into polymeric carriers can achieve controlled release, including delayed release, and sustained release. According to some embodiment of the composition, the polymer comprises a mucoadhesive polymer, a thermoresponsive polymer or both. According to some embodiment of the composition, a fraction of the synthetic D-beta hydroxybutyrate is adsorbed or weakly bound to the surface of the particles and contributes to a rapid initial release or burst release. According to some embodiment of the composition the particles are of any order release kinetics, including zero order release, first order release, second order release, delayed release, sustained release, immediate release, long term release, or a combination thereof. According to some embodiment of the composition release of the synthetic D-beta hydroxybutyrate from the particles is through diffusion, erosion or both. According to some embodiment of the composition, the composition is formulated for intranasal delivery.

According to some embodiments of the composition, the mucoadhesive polymer includes chitosan, alginate, and a cellulose or a derivative thereof.

According to some embodiments of the composition, the thermoreversible polymer includes gelatin, carrageenan, methylcellulose, hydroxypropyl methylcellulose (HPMC), xyloglucan, poly (N-isopropylacrylamide-c-acrylic acid), poly(N-isopropylacrylamide (PNIPAAm)/polyethylene oxide (PEO), poloxamer (Pluronic) or PEO/polylactic acid-co-glycolic acid (PLGA).

According to some embodiments of the composition, the polymer is selected from a cellulose derivative, a polyacrylate, starch, gelatin, a phospholipid, chitosan, poly-N-alkyl acrylamide/poly-N-isopropylacrylamide, cyclodextrin, a poloxamer and methylcellulose.

According to some embodiments of the composition, when formulated for intranasal delivery, a maximum amount of about 25 mg/dose is administered to achieve a minimum daily dose ranging from about 85 mg/day to about 800 mg/day, inclusive.

According to another aspect, the present disclosure provides a method for promoting brain health comprising (a) formulating a composition containing an active ingredient as a suspension of particles comprising a biodegradable polymer containing an effective amount of the active ingredient; (b) administering to a subject the composition comprising the active ingredient, wherein the active ingredient is D-beta hydroxybutyrate; and (c) targeting the composition to the brain, wherein burst release and controlled release of the D- beta-hydroxybutyrate can lead to effective D-beta hydroxybutyrate levels in the brain.

According to some embodiments of the method; size of the particles is greater than 10 nm in diameter, greater than 15 nm in diameter; greater than 20 nm in diameter, greater than 30 nm in diameter, greater than 40 nm in diameter, greater than 50 nm in diameter, greater than 60 nm in diameter, greater than 70 nm in diameter, greater than 80 nm; in diameter, greater than 90 nm in diameter, greater than 100 nm in diameter, greater than 200 nm in diameter, greater than 300 nm in diameter, greater than 400 nm in diameter, greater than 500 nm in diameter, greater than 600 nm in diameter, greater than 700 nm in diameter, greater than 800 nm in diameter, greater than 900 nm in diameter, greater than 1000 nm in diameter, greater than 2000 nm in diameter, greater than 3000 nm in diameter, greater than 4000 nm in diameter, greater than 5000 nm in diameter, greater than 6000 nm in diameter, greater than 7000 nm in diameter, greater than 8000 nm in diameter, greater than 9000 nm in diameter, or greater than 10,000 nm in diameter. According to some embodiments of the method, the synthetic D-beta hydroxybutyrate is dispersed throughout the particles; or the particles are impregnated with the D-beta hydroxybutyrate; or the particles comprise a matrix and the matrix comprises the D-beta hydroxybutyrate. According to some embodiments of the method, integration of the synthetic D-beta-hydroxybutyrate into polymeric carriers can achieve controlled release, including delayed release, and sustained release. According to some embodiments of the method, the polymer comprises a mucoadhesive polymer, a thermoresponsive polymer or both. According to some embodiments of the method, a fraction of the synthetic D-beta hydroxybutyrate is adsorbed or weakly bound to the surface of the particles and contributes to a rapid initial release or burst release. According to some embodiments of the method, the particles are of any order release kinetics, including zero order release, first order release, second order release, delayed release, sustained release, immediate release, long term release, or a combination thereof. According to some embodiments of the method release of the synthetic D-beta hydroxybutyrate from the particles is through diffusion, erosion or both. According to some embodiments of the method the composition is formulated for oral delivery or intranasal delivery.

According to some embodiments of the method, the mucoadhesive polymer includes chitosan, alginate, and a cellulose or a derivative thereof.

According to some embodiments of the method, the thermoreversible polymer includes gelatin, carrageenan, methylcellulose, hydroxypropyl methylcellulose (HPMC), xyloglucan, poly (N-isopropylacrylamide-c-acrylic acid), poly(N-isopropylacrylamide (PNIPAAm)/polyethylene oxide (PEO), poloxamer (Pluronic) or PEO/polylactic acid-co-glycolic acid (PLGA).

According to some embodiments of the method, the polymer is selected from a cellulose derivative, a polyacrylate, starch, gelatin, a phospholipid, chitosan, poly-N-alkyl acrylamide/poly-N-isopropylacrylamide, cyclodextrin, a poloxamer and methylcellulose.

According to some embodiments of the method when formulated for intranasal delivery, a maximum amount of about 25 mg/dose is administered to achieve a minimum daily dose ranging from about 85 mg/day to about 800 mg/day, inclusive.

According to another aspect, the present disclosure provides a method for reducing risk of brain damage due to an acquired brain injury for a subject susceptible to, or otherwise at risk of, the acquired brain injury comprising administering a pharmaceutical composition comprising a pharmaceutically acceptable carrier and a formulation comprising a therapeutic amount of an API, wherein the API is a synthetic D-beta-hydroxybutyrate, wherein the administering is intranasally (IN), wherein the administering comprises targeted delivery to the olfactory region of the nasal cavity of the subject; wherein the targeted delivery achieves an effective amount of the API in the brain, and wherein the effective amount of the pharmaceutical composition: (i) eliminates or reduces the risk of the acquired brain injury, or (ii) lessens the severity of the acquired brain injury, or (iii) delays the onset of the acquired brain injury; or (iv) a combination thereof.

According to some embodiments of the method, the acquired brain injury is a traumatic brain injury. According to some embodiments of the method, the administering is neuroprotective. According to some embodiments of the method, the subject's risk of the acquired brain injury is increased by participating in an event where an acquired brain injury is a known risk. According to some embodiments of the method, the administering is a maximum amount of 25 mg/dose within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before the event. According to some embodiments of the method, the pharmaceutical composition comprises a liquid spray formulation or a dry powder formulation. According to some embodiments of the method, the pharmaceutical composition comprises the active formulated as a solution, a suspension or a dispersion. According to some embodiments of the method, a minimum daily dose of the pharmaceutical composition contains from about 85 mg/day to about 800 mg/day inclusive of the API.

According to some embodiments of the method, the traumatic brain injury comprises a concussion. According to some embodiments of the method, the event is a military operation or a sports event. According to some embodiments of the method, the sports event is hockey, football, soccer, baseball, polo, rugby, horseback riding, a car race, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing.

According to some embodiments of the method, the pharmaceutical composition is delivered as a liquid spray comprising a droplet size distribution comprising droplets containing the API. According to some embodiments of the method, the dry powder formulation when aerosolized comprises a cloud of very fine particles comprising the API. According to some embodiments of the method, the formulation is formulated with excipients.

According to some embodiments of the method, the droplet size distribution of the liquid spray ranges from about $20 \mu$ to about $120 \mu m$, inclusive containing a therapeutic amount of the API. According to some embodiments of the method, the distribution of particles is depleted of smaller particles that would otherwise go into the lung.

According to another aspect, the present disclosure provides a method for treating symptoms of a brain injury of a subject comprising neuroinflammation comprising administering a pharmaceutical composition comprising a pharmaceutically acceptable carrier and a formulation comprising a therapeutic amount of an API, wherein the API is a synthetic D-beta-hydroxybutyrate, wherein the administering is intranasally (IN); wherein the administering comprises targeted delivery to the olfactory region of the nasal cavity of the subject, wherein the targeted delivery achieves an effective amount of the API in the brain, and wherein the effective amount of the pharmaceutical composition accomplishes one or more therapeutic effects including: (i) reducing the severity of the acquired brain injury; or (ii) limiting development of symptoms characteristic of the acquired brain injury being treated; or (iii) limiting worsening of symptoms characteristic of the acquired brain injury being treated; or (iv) limiting recurrence of the acquired brain injury in subjects that have previously had the acquired brain injury; or limiting recurrence of symptoms in subjects that were previously asymptomatic for the acquired brain injury; or a combination thereof.

According to some embodiments of the method, the acquired brain injury is a traumatic brain injury. According to some embodiments of the method, the administering is neuroprotective. According to some embodiments of the method, the subject's risk of the acquired brain injury is increased by participating in an event where an acquired brain injury is a known risk. According to some embodiments of the method, the administering is 25 mg/dose within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, of the brain injury and for at least 1 hour, 2 hours, 3 hours, 4 hours, 5 hours, 6 hours, 7 hours, 8 hours, 9 hours, 10 hours, 11 hours, 12 hours, 13 hours, 14 hours, 15 hours ,16 hours, 17 hours, 18 hours, 19 hours, 20 hours, 21 hours, 22 hours, 23 hours, at least 24 hours, at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after the event where the acquired brain injury is acquired. According to some embodiments of the method, the pharmaceutical composition comprises a liquid spray formulation or a dry powder formulation. According to some embodiments of the method, the pharmaceutical composition comprises the active formulated as a solution, a suspension or a dispersion. According to some embodiments of the method, a minimum daily dose of the pharmaceutical composition contains from about 85 mg/day to about 800 mg/day, inclusive, of the API. According to some embodiments of the method, the traumatic brain injury comprises a concussion. According to some embodiments of the method, the event is a military operation or a sports event. According to some embodiments of the method, the sports event is hockey, football, soccer, baseball, polo, rugby, horseback riding, a car race, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. According to some embodiments of the method, the pharmaceutical composition is delivered as a liquid spray comprising a droplet size distribution comprising droplets containing the API. According to some embodiments of the method, the dry powder formulation when aerosolized comprises a cloud of very fine particles comprising the API. According to some embodiments of the method, the formulation is formulated with excipients. According to some embodiments of the method, the droplet size distribution of the liquid spray ranges from about 20 μ to about 120 μm, inclusive containing a therapeutic amount of the API.

According to another aspect, the present disclosure provides a method for reducing risk of an acquired brain injury for a subject susceptible to, or otherwise at risk of, the acquired brain injury and for treating symptoms of the acquired brain injury after the brain injury has been acquired by the subject, wherein the subject's risk of the acquired brain injury is increased by participating in an event where an acquired brain injury is a known risk; comprising (a) administering a pharmaceutical composition comprising a pharmaceutically acceptable carrier and a formulation comprising a therapeutic amount of an API, wherein the API is a synthetic D-beta-hydroxybutyrate before an event, wherein the administering is intranasally (IN) and neuroprotective, wherein the administering comprises targeted delivery to the olfactory region of the nasal cavity of the subject; wherein the targeted delivery achieves an effective amount of the API in the brain, and wherein the effective amount of the pharmaceutical composition (i) eliminates or reduces the risk of the acquired brain injury or (ii) lessens the severity of the acquired brain injury, or (iii) delays the onset of the acquired brain injury; or (iv) a combination thereof; and (b) continuing the administering intranasally (IN) after acquiring the brain injury at the event, wherein the effective amount of the pharmaceutical composition accomplishes one or more therapeutic effects comprising: (i') reducing the severity of the acquired brain injury; (ii') limiting development of symptoms characteristic of the acquired brain injury being treated; (iii') limiting worsening of symptoms characteristic of the acquired brain injury being treated; (iv') limiting recurrence of the acquired brain injury in subjects that have previously had the acquired brain injury; or (v') limiting recurrence of symptoms in subjects that were previously asymptomatic for the acquired brain injury;(vi') or a combination thereof.

According to some embodiments of the method, the acquired brain injury is a traumatic brain injury. According to some embodiments of the method, the pharmaceutical composition comprises a liquid spray formulation or a dry powder formulation. According to some embodiments of the method, the pharmaceutical composition comprises the API formulated as a solution, a suspension or a dispersion. According to some embodiments of the method, a minimum daily dose of the pharmaceutical composition contains from about 85 mg/day to about 800 mg/day, inclusive, of the API. According to some embodiments of the method, the traumatic brain injury comprises a concussion. According to some embodiments of the method, the event is a military operation or a sports event. According to some embodiments of the method, the sports event is hockey, football, soccer, baseball, polo, rugby. horseback riding, a car race, gymnastics, mountain climbing, basketball, mixed martial arts, Brazilian Jiu-jitsu, Muay Thai, tae kwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. According to some embodiments of the method, the administering is a maximum amount of 25 mg/dose as frequently as tolerated, e.g. every 2 minutes, 5 minutes, 10 minutes, 20 minutes, 30 minutes; 40 minutes, 50 minutes, or 60 minutes, for at least 1 hour, for 2 hours, for 3 hours, for 4 hours, for 5 hours, for 6 hours, for 7 hours, for 8 hours, for 9 hours, for 10 hours, for 11 hours, for 12 hours, for 13 hours, for 14 hours, for 15 hours, for 16 hours, for 17 hours, for 18 hours, for 19 hours, for 20 hours, for 21 hours, for 22 hours, for 23 hours, for 24 hours, within at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days before the event; and a maximum of 25 mg/dose as frequently as tolerated, e.g. every 2 minutes, 5 minutes, 10 minutes, 20 minutes, 30 minutes; 40 minutes, 50 minutes, or 60 minutes for at least 1 hour, 2 hours, 3 hours, 4 hours, 5 hours, 6 hours, 7 hours, 8 hours, 9 hours, 10 hours, 11 hours, 12 hours, 13 hours, 14 hours, 15 hours ,16 hours, 17 hours, 18 hours, 19 hours, 20 hours, 21 hours, 22 hours, 23 hours, or at least 24 hours, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, after the brain injury is acquired.

According to some embodiments of the method, the pharmaceutical composition is delivered as a liquid spray comprising a droplet size distribution comprising droplets containing the API. According to some embodiments of the method, the dry powder formulation when aerosolized comprises a cloud comprising a distribution of fine particles comprising the API. According to some embodiments of the method, the formulation is formulated with excipients. According to some embodiments of the method, the droplet size distribution of the liquid spray ranges from about 20 μ to about 120 μm, inclusive containing a therapeutic amount of the API. According to some embodiments of the method, the distribution of particles is depleted of smaller particles that would otherwise go into the lung.

According to another aspect, the present disclosure provides a pharmaceutical composition for use in reducing risk of brain damage due to an acquired brain injury in a subject susceptible to, or otherwise at risk of, the acquired brain injury, said composition comprising a pharmaceutically acceptable carrier and a formulation comprising a therapeutic amount of an API, wherein the API is a synthetic D-beta-hydroxybutyrate, wherein the treatment comprises targeted delivery of 25 mg/dose to the olfactory region of the nasal cavity of the subject (a) before the subject participates in an event where an acquired brain injury is a known risk; (b) after the subject participates in the event for use in treating symptoms of the acquired brain injury of the subject comprising neuroinflammation; or both.

According to some embodiments of the pharmaceutical composition for use, the targeted delivery achieves an effective amount of the API in the brain of the subject. According to some embodiments the effective amount of the pharmaceutical composition limits development of symptoms characteristic of the acquired brain injury; and/or limits worsening of symptoms characteristic of the acquired brain injury; and/or limits recurrence of the acquired brain injury in subjects that have previously had the acquired brain injury; and/or limits recurrence of symptoms in subjects that were previously asymptomatic for the acquired brain injury.

According to some embodiments of the pharmaceutical composition for use, the acquired brain injury is a traumatic brain injury; and/or the brain damage comprises neuroinflammation; and/or the event is a sports event; and/or the event is a military operation; and/or the administering is neuroprotective; and/or the pharmaceutical composition comprises the API formulated as a solution, a suspension or a dispersion; and/or the administering is a minimum amount of about 85 mg/day to about 800 mg/day, inclusive, of the API. According to some embodiments of the pharmaceutical composition for use, the traumatic brain injury comprises a concussion. According to some embodiments of the pharmaceutical composition for use, the sports event is hockey, football, soccer, baseball, polo, rugby, horseback riding, autoracing, motorcycling, skiing, gymnastics, mountain climbing, basketball, mixed martial arts, Brazilian Jiu-jitsu, Muay Thai, tae kwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing.

DETAILED DESCRIPTION

Glossary

The term "aerodynamic diameter" as used herein refers to the diameter of a sphere of unit density which behaves aerodynamically the same as the particle of the test substance/compound. It is used to predict where particles of different size and density may be deposited in the respiratory tract.

The term "active ingredient" as used herein refers to a drug, molecule, composition or other substance that provides a biological activity or affect.

The term "active pharmaceutical ingredient" ("API" or "bulk active") is the substance in a drug that is pharmaceutically active.

The term "adenosine 5'-triphosphate" or "ATP" is the principal molecule for storing and transferring energy in cells.

The term "administer" as used herein means to give or to apply. The term "administering" as used herein includes in vivo administration, as well as administration directly to tissue ex vivo. Generally, compositions may be administered systemically either orally, buccally, parenterally, by inhalation or insufflation (i.e., through the mouth or through the nose), or rectally in dosage unit formulations containing conventional nontoxic pharmaceutically acceptable carriers, adjuvants, and vehicles as desired, or may be locally administered by means such as, but not limited to, injection, implantation, grafting, or topical application. Administering can be performed, for example, once, a plurality of times, and/or over one or more extended periods either as individual unit doses or in the form of a treatment regimen comprising multiple unit doses of multiple drugs and/or substances.

The term "adsorb" as used herein refers to adhesion to a surface.

The term "aerosol" as used herein refers to a substance enclosed under pressure and able to be released as a fine spray, typically by means of a propellant gas. For example, a colloidal dispersion of solid or liquid particles in a gas is an aerosol.

The term "aerosol generator" refers to a device that gives rise to an aerosol.,

The term "aerosolized" as used herein refers to being dispersed or discharged in the form of a colloidal dispersion of solid or liquid particles in a gas.

***Anatomical Terms:***

When referring to animals, that typically have one end with a head and mouth, with the opposite end often having the anus and tail, the head end is referred to as the cranial end, while the tail end is referred to as the caudal end. Within the head itself, rostral refers to the direction toward the end of the nose, and caudal is used to refer to the tail direction. The surface or side of an animal's body that is normally oriented upwards, away from the pull of gravity, is the dorsal side; the opposite side, typically the one closest to the ground when walking on all legs, swimming or flying, is the ventral side. On the limbs or other appendages, a point closer to the main body is "proximal"; a point farther away is "distal". Three basic reference planes are used in zoological anatomy. A "sagittal" plane divides the body into left and right portions. The "midsagittal" plane is in the midline, i.e. it would pass through midline structures such as the spine, and all other sagittal planes are parallel to it. A "coronal" plane divides the body into dorsal and ventral portions. A "transverse" plane divides the body into cranial and caudal portions.

When referring to humans, the body and its parts are always described using the assumption that the body is standing upright. Portions of the body which are closer to the head end are "superior" (corresponding to cranial in animals), while those farther away are "inferior" (corresponding to caudal in animals). Objects near the front of the body are referred to as "anterior" (corresponding to ventral in animals); those near the rear of the body are referred to as "posterior" (corresponding to dorsal in animals). A transverse, axial, or horizontal plane is an X-Y plane, parallel to the ground, which separates the superior/head from the inferior/feet. A coronal or frontal plane is an Y-Z plane, perpendicular to the ground, which separates the anterior from the posterior. A sagittal plane is an X-Z plane, perpendicular to the ground and to the coronal plane, which separates left from right. The midsagittal plane is the specific sagittal plane that is exactly in the middle of the body.

Structures near the midline are called medial and those near the sides of animals are called lateral. Therefore, medial structures are closer to the midsagittal plane, lateral structures are further from the midsagittal plane. Structures in the midline of the body are median. For example, the tip of a human subject's nose is in the median line.

Ipsilateral means on the same side, contralateral means on the other side and bilateral means on both sides. Structures that are close to the center of the body are proximal or central, while ones more distant are distal or peripheral. For example, the hands are at the distal end of the arms, while the shoulders are at the proximal ends.

The term "axon" as used herein refers to the nerve fiber that carries a nerve impulse from a nerve cell to a target cell and also carries material from the nerve terminals back to the nerve cell.

The term "axonal injury" refers to impaired function and gradual loss of axons that enable nerve cells to communicate with each other.

The terms "beta-hydroxybutyrate, $\beta$-hydroxybutyrate", and "BHB" are used herein interchangeably to refer to the predominant ketone body in the blood or to a synthetic BHB compound. In the body, it is formed mainly in the liver from free fatty acids during fasting or on a high-fat, low-carbohydrate diet ketogenic diet.

The term "binding" and its other grammatical forms means a lasting attraction between chemical substances. Binding specificity involves both binding to a specific partner and not binding to other molecules. "Relative binding specificity" is a characteristic whereby in a biochemical system a molecule interacts with its targets or partners differentially, thereby impacting them distinctively depending on the identity of individual targets or partners.

The term "bioavailability" refers to the extent and rate at which an active moiety is absorbed from a drug product and becomes available at the site of action. 21 CFR 320.1. For a product that enters systemic circulation, thereby accessing the site of action, surface charge on polymeric particles influences the particles' distribution throughout the body and level of cellular uptake. [Vhora, I. et al. Applications of Polymers in Drug Delivery, Ch. 8, pages 221-61, at 234; Elsevier, Inc. (2021)]. Because cell membranes are negatively charged, a positive charge favors a higher intracellular concentration. The exterior of particles can be modified to attach targeting moieties to restrict the distribution at a target site. PEG, PVP, and dextran, for example, are surface modifiers that guard particles from being captured by the reticuloendothelial system (RES), thereby increasing their blood residence time (increased half-life) and improving their bioavailability. [Id.]

The term "biocompatible" as used herein refers to a material that is generally non-toxic to the recipient and does not possess any significant untoward effects to the subject and, further, that any metabolites or degradation products of the material are non-toxic to the subject. Typically, a substance that is "biocompatible" causes no clinically relevant tissue irritation, injury, toxic reaction, or immunological reaction to living tissue.

The term "biodegradable" as used herein refers to a material that will erode to soluble species or that will degrade under physiologic conditions to smaller units or chemical species that are, themselves, non-toxic (biocompatible) to the subject and capable of being metabolized, eliminated, or excreted by the subject. The two major types for biodegradation of delivered biodegradable polymeric material are chemical and enzymic degradation, e.g., by hydrolysis and oxidation-dependent degradation Polymers also may degrade by mechanical or thermal processes. [Vhora, I. et al. Applications of Polymers in Drug Delivery, Ch. 8, pages 221-61, at 228; Elsevier, Inc. (2021)

The term "bioequivalence" as used herein refers to the absence of a significant difference in the rate and extent to which an active ingredient or active moiety in pharmaceutical equivalents or pharmaceutical alternatives becomes available at the site of drug action when administered at the same molar dose under similar conditions in an appropriately designed study. 21 CFR 320.21.

The term "biofermentation" or "microbial fermentation" are used interchangeably herein refers to an anaerobic biological process in which oxidation of a substrate is coupled to the reduction of another substrate or an intermediate derived from the oxidation. Fermentation bacteria are anaerobic, but use organic molecules as their final electron acceptor to produce fermentation end-products.

The term "blood brain barrier" ("BBB") as used herein refers to a network of blood vessels and tissue that is made up of closely spaced cells and helps keep harmful substances in the blood from reaching the brain.

The term "brain damage" as used herein refers to an injury that causes the destruction or deterioration of brain cells. According to some embodiments, brain damage includes neuroinflammation.

The term "brain health" as defined by the World Health Organization refers to a state of brain functioning across cognitive, sensory, social-emotional, behavioral and motor domains, allowing a person to realize their full potential over their life course, irrespective of the presence or absence of disorders.

The term "carrier" as used herein describes a material that does not cause significant irritation to an organism and does not abrogate the biological activity and properties of the compound of the composition of the described invention. Carriers must be of sufficiently high purity and of sufficiently low toxicity to render them suitable for administration to the mammal being treated. The carrier can be inert, or it can possess pharmaceutical benefits. The terms "excipient", "carrier", or "vehicle" are used interchangeably to refer to carrier materials suitable for formulation and administration of pharmaceutically acceptable compositions described herein. Carriers and vehicles useful herein include any such materials know in the art which are nontoxic and do not interact with other components.

The term "carryover" or "CO" as used herein refers to one type of systematic error in an HPLC based analytical method that is derived from a preceding sample and introduced into the next sample.

The term "central nervous system" ("CNS") as used herein refers to that part of the nervous system that consists of the brain and spinal cord.

The term "cerebrospinal fluid" as used herein refers to an ultrafiltrate of plasma contained within the ventricles of the brain and the subarachnoid spaces of the cranium and spine. It is predominantly secreted by the choroid plexus. The constant secretion contributes to complete CSF renewal 4-5 times per 24 hour period in the average young adult.

The term "chemical bond" as used herein refers to an attractive force between atoms strong enough to permit the combined aggregate to function as a unit.

The term "chiral" is used to describe asymmetric molecules that are non-superposable since they are mirror images of each other and therefore have the property of chirality. Such molecules are also called enantiomers and are characterized by optical activity.

The term "chirality" refers to the geometric property of a rigid object (or spatial arrangement of points or atoms) of being non-superposable on its mirror image. If the object is superposable on its mirror image, the object is described as being achiral.

The term "chirality axis" refers to an axis about which a set of ligands is held so that it results in a spatial arrangement which is not superposable on its mirror image.

The term "chirality center" refers to an atom holding a set of ligands in a spatial arrangement, which is not superimposable on its mirror image. A chirality center may be considered a generalized extension of the concept of the asymmetric carbon atom to central atoms of any element.

The term "closed head injury" as used herein refers to impact to the head from an outside force, without any skull fracture or displacement. The term "open head injury" refers to trauma to the brain resulting in loss of consciousness due to the penetration of the brain by a foreign object, such as a bullet.

The term "compatible" as used herein means that the components of a composition are capable of being combined with each other in a manner such that there is no interaction that would substantially reduce the efficacy of the composition under ordinary use conditions.

The term "composition" as used herein refers to a mixture of ingredients.

The term "concussion" as used herein refers to a disruption of neurological function resulting from a head injury or violent shaking.

The term "condition", as used herein, refers to a variety of health states and is meant to include disorders or diseases caused by any underlying mechanism or disorder, injury, and the promotion of healthy tissues and organs.

The term "configuration" as used herein denotes the stereochemical arrangement of atoms that cannot be altered without breaking chemical bonds.

:The term "conformation" refers to the geometrical arrangement of atoms in a polymer chain.

The term "contact" and its various grammatical forms as used herein refers to a state or condition of touching or of being in immediate or local proximity and includes contacting a composition to a target destination.

The term "container closure system" as used herein refers to the sum of packaging components that together contain, protect and deliver the dosage form, including the pump for nasal and inhalation sprays.

The term "controlled release" is intended to refer to any active-containing formulation in which the manner and profile of active release from the formulation are controlled. This includes immediate as well as non-immediate release formulations, with non-immediate release formulations including, but not limited to, sustained release and delayed release formulations. The term "delayed release" is used herein in its conventional sense to refer to an active formulation in which there is a time delay between administration of the formulation and the release of the active therefrom. "Delayed release" may or may not involve gradual release of active over an extended period of time, and thus may or may not be "sustained release." The term "long-term" release, as used herein, means that the implant is constructed and arranged to deliver therapeutic levels of the active ingredient for at least 7 days, and preferably about 30 to about 60 days. The term "sustained release" (also referred to as "extended release") is used herein in its conventional sense to refer to an active formulation that provides for gradual release of an active over an extended period of time, and that preferably, although not necessarily, results in substantially constant blood levels of an active over an extended time period.

The term "cytokine" as used herein refers to small soluble protein substances secreted by cells which have a variety of effects on other cells. Cytokines mediate many important physiological functions including growth, development, wound healing, and the immune response. They act by binding to their cell-specific receptors located in the cell membrane, which allows a distinct signal transduction cascade to start in the cell, which eventually will lead to biochemical and phenotypic changes in target cells. Cytokines can act both locally and distantly from a site of release. They include type I cytokines, which encompass many of the interleukins, as well as several hematopoietic growth factors; type II cytokines, including the interferons and interleukin-10; tumor necrosis factor ("TNF")-related molecules, including TNF$\alpha$ and lymphotoxin; immunoglobulin super-family members, including interleukin 1 ("IL-1"); and the chemokines, a family of molecules that play a critical role in a wide variety of immune and inflammatory functions. The same cytokine can have different effects on a cell depending on the state of the cell. Cytokines often regulate the expression of, and trigger cascades of other cytokines. Nonlimiting examples of cytokines include e.g., IL-1$\alpha$., IL-$\beta$., IL-2, IL-3, IL-4, IL-5, IL-6, IL-7, IL-8, IL-9, IL-10, IL-11, IL-12/IL-23 P40, IL13, IL-15, IL-17, IL-18, IL-21, IL-23, TGF-$\beta$, IFN-$\gamma$, GM-CSF, Gro.alpha., MCP-1 and TNF-$\alpha$.

The terms "D value" or "mass division diameter" as used herein, refer to the diameter which, when all particles in a sample are arranged in order of ascending mass, divides the sample's mass into specified percentages. The percentage mass below the diameter of interest is the number expressed after the "D". For example, the D10 diameter is the diameter at which 10% of a sample's mass is comprised of smaller particles, and the D50 is the diameter at which 50% of a sample's mass is comprised of smaller particles. The D50 is also known as the "mass median diameter" as it divides the sample equally by mass. The D90 diameter is the diameter at which 90% of a sample's mass is comprised of smaller particles. While D-values are based on a division of the mass of a sample by diameter, the actual mass of the particles or the sample does not need to be known. A relative mass is sufficient as D-values are concerned only with a ratio of masses. This allows optical measurement systems to be used without any need for sample weighing.

From the diameter values obtained for each particle a relative mass can be assigned according to the following relationship:

$$\text{Mass of a sphere} = \pi/6 \ d3 \ \rho$$

Assuming that $\rho$ is constant for all particles and cancelling all constants from the equation:

$$\text{Relative mass} = d3$$

i.e., each particle's diameter is therefore cubed to give its relative mass. These values can be summed to calculate the total relative mass of the sample measured. The values may then be arranged in ascending order and added iteratively until the total reaches 10%, 50% or 90% of the total relative mass of the sample. The corresponding D value for each of these is the diameter of the last particle added to reach the required mass percentage.

The term "delivery" as used herein refers to the bringing of an active agent to a place in the body.

The term "derived from" as used herein, is meant to encompasses any method for receiving, obtaining, or modifying something from a source of origin.

"Diastereoisomers" are stereoisomers that have at least two asymmetric atoms, but which are not mirror-images of each other.

The term "diffusion" as used herein refers to the movement of individual molecules of a substance through a semipermeable barrier from an area of higher concentration to an area of lower concentration.

The terms "disease" or "disorder" as used herein refer to an impairment of health or a condition of abnormal functioning.

**EP 4 631 364 A2**

The term "dispersed" as used herein refers to the action or process of distributing things over a wide area.

The term "dispersion", as used herein, refers to a two-phase system, in which one phase is distributed as droplets in the second, or continuous phase. In these systems, the dispersed phase frequently is referred to as the discontinuous or internal phase, and the continuous phase is called the external phase and comprises a continuous process medium. For example, in course dispersions, the particle size is 0.5 $\mu$m. In colloidal dispersions, size of the dispersed particle is in the range of approximately 1 nm to 0.5 $\mu$m. A molecular dispersion is a dispersion in which the dispersed phase consists of individual molecules; if the molecules are less than colloidal size, the result is a true solution.

The term "dissolution" as used herein refers to the process by which a substance forms a solution. "Rate of dissolution" is the amount of active substance that goes in solution per unit time under standardized conditions of liquid/solid interface, temperature and solvent composition.

The term "dose" as used herein refers to the predetermined amount of an active agent administered at one time.

The term "dosage" as used herein refers to a predetermined amount and rate of administration of an active agent. Dosage levels are based on a variety of factors, including the type of injury, the age, weight, sex, medical condition of the patient, the severity of the condition, the route of administration, and the particular active agent employed. Thus the dosage regimen may vary widely, but can be determined routinely by a physician using standard methods.

The term "dosage form" refers to the physical form that contains the active agent combined with excipients. Examples include solid dosage forms (tablet, capsule, pellets, pills, lozenges, granules, etc.), liquid dosage forms (solution, suspension, emulsion, elixir, etc.), semisolid dosage forms (ointment, cream, paste, etc.) and gaseous dosage forms (aerosol, insufflation, etc.).

The term "dosing regimen" as used herein refers to the frequency at which an active agent is administered and includes formulation, route of administration, dose, dosing interval and duration of administration. The total daily dose is calculated from the dose and the number of times per day the dose is administered. Designing a correct dosage regimen is important for achieving the desired efficacy and for avoiding undesired effects.

The term "droplet" as used herein refers to a small drop, meaning a very small volume of a flowable substance. Some factors that affect droplet size are pressure, viscosity, fluid temperature, and surface tension. For example, increased pressure, increased fluid temperature, increased viscosity and increased surface tension decrease droplet size.

The term "droplet size distribution" as used herein refers to a statistical indication of the percentage of droplets of a certain size (or in a certain size interval). A simple distribution function assumes that all droplets have the same shape (usually spheres), which is generally valid for fully developed sprays. Such a distribution is completely characterized by (1) the maximum diameter; (2) the size parameter; and (3) the distribution parameters, which can be calculated from any three independent mean diameters which are known. The remaining problem consists in correlating these parameters with the physical characteristics of the spray-generating system, including physical properties of the droplet phase and of the continuous medium. Among these properties are densities, viscosities, and interfacial tensions.[Mugele, RA and Evans, HD. Droplet size distribution in sprays. Indust. And Engineering Chemistry (1951) 43 (6): 1317-24].

The terms "drug load (%)" and drug loading capacity" are used interchangeably herein to refer to a ratio of the weight of the compound of the present invention in microparticles relative to the total weight of the microparticles, expressed as a percentage. It reflects the drug content of the microparticle.

The term "drug product" as used herein refers to a finished dosage form that contains a drug substance, generally, but not necessarily, in association with one or more other ingredients.

The term "drug substance" as used herein refers to an active ingredient intended to furnish pharmacological activity or other direct effect in the diagnosis, cure, mitigation, treatment or prevention of a disease, or to affect the structure and function of the body, but does not include intermediates used in synthesis of such ingredient.

The term "effective amount" refers to the amount of the pharmaceutical composition of the described invention necessary or sufficient to realize a desired biologic effect. Additionally, the term "effective amount" includes prophylactic or preventative amounts of the compositions of the described invention. In prophylactic or preventative applications of the described invention, pharmaceutical compositions or medicaments are administered to a patient susceptible to, or otherwise at risk of, a disease, disorder or condition in an amount sufficient to eliminate or reduce the risk, lessen the severity, or delay the onset of the disease, disorder or condition, including biochemical, histologic and/or behavioral symptoms of the disease, disorder or condition, its complications, and intermediate pathological phenotypes presenting during development of the disease, disorder or condition.

The term "emulsion" as used herein refers to a two-phase system prepared by combining two immiscible liquid carriers, one of which is disbursed uniformly throughout the other. It consists of globules that have diameters equal to or greater than those of the largest colloidal particles. The phase existing as small droplets is called the dispersed phase and the surrounding liquid is known as the continuous phase. The globule size must be such that the system achieves maximum stability. Usually, separation of the two phases will occur unless a third substance, an emulsifying agent, is incorporated.

"Enantiomers" are a pair of stereoisomers that are non- superimposable mirror images of each other. A 1 :1 mixture of a pair of enantiomers is a "racemic" mixture. When a compound is a pure enantiomer, the stereochemistry at each chiral carbon may be specified by either R or S. Resolved compounds whose absolute configuration is unknown can be designated (+) or (-) depending on the direction (dextro- or levorotatory) in which they rotate plane polarized light at the wavelength of the sodium D line.

The term "endogenous" as used herein refers to relating to or developing from factors from within the body.

The term "entrapment efficiency (%)" as used herein refers to a ratio of drug retained by the microparticle relative to the total amount available, expressed as a percentage.

The term "erosion" as used herein refers to a loss of material from the polymer bulk.

The term "excipient" is used herein to include any other agent or compound that may be contained in a formulation that is not the active agent. As such, an excipient should be pharmaceutically or biologically acceptable or relevant (for example, an excipient should generally be non-toxic to the subject). "Excipient" includes a single such compound and is also intended to include a plurality of such compounds.

The term "exogenous" as used herein refers to relating to or developing from factors outside the body.

The term "formulation" as used herein refers to a mixture prepared according to a specific procedure, or to a formula prepared according to a particular recipe or rule.

The term "G proteins" as used herein refers to specialized proteins with the ability to bind the nucleotides guanosine triphosphate (GTP) and guanosine diphosphate (GDP).

The term "G-protein-coupled receptor" (GPCR) refers to a membrane receptor in eukaryotes that allows a cell to receive information from its environment. GPCRs consist of a single polypeptide that is folded into a globular shape and embedded in a cell's plasma membrane. Seven segments of this molecule span the entire width of the membrane - explaining why GPCRs are sometimes called seven-transmembrane receptors - and the intervening portions loop both inside and outside the cell. The extracellular loops form part of the pockets at which signaling molecules bind to the GPCR. GPCRs interact with G proteins in the plasma membrane. When an external signaling molecule binds to a GPCR, it causes a conformational change in the GPCR. This change then triggers the interaction between the GPCR and a nearby G protein. the G proteins that associate with GPCRs are heterotrimeric, meaning they have three different subunits: an alpha subunit, a beta subunit, and a gamma subunit. Two of these subunits - alpha and gamma - are attached to the plasma membrane by lipid anchors. A G protein alpha subunit binds either GTP or GDP depending on whether the protein is active (GTP) or inactive (GDP). In the absence of a signal, GDP attaches to the alpha subunit, and the entire G protein-GDP complex binds to a nearby GPCR. This arrangement persists until a signaling molecule joins with the GPCR. At this point, a change in the conformation of the GPCR activates the G protein, and GTP physically replaces the GDP bound to the alpha subunit. As a result, the G protein subunits dissociate into two parts: the GTP-bound alpha subunit and a beta-gamma dimer. Both parts remain anchored to the plasma membrane, but they are no longer bound to the GPCR, so they can now diffuse laterally to interact with other membrane proteins. G proteins remain active as long as their alpha subunits are joined with GTP. However, when this GTP is hydrolyzed back to GDP, the subunits once again assume the form of an inactive heterotrimer, and the entire G protein reassociates with the now-inactive GPCR. In this way, G proteins work like a switch - turned on or off by signal-receptor interactions on the cell's surface. Whenever a G protein is active, both its GTP-bound alpha subunit and its beta-gamma dimer can relay messages in the cell by interacting with other membrane proteins involved in signal transduction. Specific targets for activated G proteins include various enzymes that produce second messengers, as well as certain ion channels that allow ions to act as second messengers. Some G proteins stimulate the activity of these targets, whereas others are inhibitory. [Li, J. et al. The Molecule Pages database. Nature (2002) 420: 716-17].

The term "glial cells", "neuroglial cells" or glia" are small cells that lack axons and/or dendrites that exist in the central nervous system and the peripheral nervous system. Glial cells function as modulators of the CNS and PNS environments; they increase and decrease activity within the synapses by regulating neurotransmitter, oxygen, and ion uptake; and aid in recovery from neural injury. The macroglia (which include astrocytes and oligodendrocytes) insulate, protect, and help neurons to develop and migrate. Microglial cells are smaller cells derived from hematopoietic stem cells (although some may be derived directly from neural stem cells). They share many properties with tissue macrophages, and are primarily scavenger cells that remove cellular debris from sites of injury or normal cell turnover. Indeed, some neurobiologists prefer to categorize microglia as a type of macrophage. Following brain damage, the number of microglia at the site of injury increases dramatically. Some of these cells proliferate from microglia resident in the brain, while others come from macrophages that migrate to the injured area from the circulation. Furthermore, micro- and macroglia work together to ensure optimal neuron function and neurotransmission through the synapses. [See Purves D, et al., Ed. (2001). "Neuroglial Cells." Neuroscience. 2nd edition. Retrieved from: https://www.ncbi.nlm.nih.gov/books/NBK10869/

The term "glial fibrillary acidic protein" or "GFAP" as used herein refers to a marker of astroglial injury which is a marker for predicting TBI. Several mouse model studies have been conducted to assess functional outcomes after TBI. Increases in GFAP level correlate with inhibited spatial learning, as evidenced by decreased performance in maze trials [Brennan, J. et al. Biomarkers for Traumatic Brain Injury, Chapter 26 - Neuropsychological testing, Editors: Alan HB Wu, W. Peacock, Academic Press (2020), pp. 397-409, citing Ferguson, S. et al. J. Neurotrauma (2016) 34 (8): 1676-91;

Marschner, L. et al. Behav. Brain Res. (2019) 365: 222-30; Broussard, JI et al. Brain In. (2018) 32 (1): 113-22]. Human trials focusing on TBI outcome and GFAP have been conducted, and GFAP was found to be significantly associated with Glasgow outcome score (GOS) [Id., citing Nylen, K. et al. J. Neurol. Sci. (2006) 240 (1): 85-91].

The term "$HCA_2$" as used herein refers to a G protein-coupled receptor for BHB and nicotinic acid.

The term "healthy control" as used herein refers to a subject having no symptoms or other clinical evidence of a TBI.

The term "immune cell" as used herein refers to a cell that is part of the immune system and helps the body to fight infections and other diseases. Immune cells include lymphocytes (e.g., B cells, T cells, natural killer (NK) cells), neutrophils, and monocytes/macrophages.

The term "immune system" as used herein refers to the body's system of defenses against disease, which comprises the innate immune system and the adaptive immune system. The innate immune system provides a non-specific first line of defense against pathogens. It comprises physical barriers (e.g. the skin) and both cellular (granulocytes, natural killer cells) and humoral (complement system) defense mechanisms. The reaction of the innate immune system is immediate, but unlike the adaptive immune system, it does not provide permanent immunity against pathogens. The adaptive immune response is the response of the vertebrate immune system to a specific antigen that typically generates immunological memory.

The term "impregnated", as used herein in its various grammatical forms refers to causing to be infused or permeated throughout; to fill interstices with; and/or to saturate with a substance.

The term "impurity profile" as used herein refers to a description of the identified and unidentified impurities present in an API.

The term "intermediate" as used herein refers to a material produced during steps of the processing of an API that undergoes further molecular change or purification before it becomes an API. Intermediates may or may not be isolated.

The term "inflammation" as used herein refers to the physiologic process by which vascularized tissues respond to injury. See, e.g., FUNDAMENTAL IMMUNOLOGY, 4th Ed., William E. Paul, ed. Lippincott-Raven Publishers, Philadelphia (1999) at 1051-1053, incorporated herein by reference. During the inflammatory process, cells involved in detoxification and repair are mobilized to the compromised site by inflammatory mediators. Inflammation is often characterized by a strong infiltration of leukocytes at the site of inflammation, particularly neutrophils (polymorphonuclear cells). These cells promote tissue damage by releasing toxic substances at the vascular wall or in uninjured tissue. Traditionally, inflammation has been divided into acute and chronic responses.

The term "acute inflammation" as used herein refers to the rapid, short-lived (minutes to days), relatively uniform response to acute injury characterized by accumulations of fluid, plasma proteins, and neutrophilic leukocytes. Examples of injurious agents that cause acute inflammation include, but are not limited to, pathogens (e.g., bacteria, viruses, parasites), foreign bodies from exogenous (e.g. asbestos) or endogenous (e.g., urate crystals, immune complexes), sources, and physical (e.g., burns) or chemical (e.g., caustics) agents.

The term "chronic inflammation" as used herein refers to inflammation that is of longer duration and which has a vague and indefinite termination. Chronic inflammation takes over when acute inflammation persists, either through incomplete clearance of the initial inflammatory agent or as a result of multiple acute events occurring in the same location. Chronic inflammation, which includes the influx of lymphocytes and macrophages and fibroblast growth, may result in tissue scarring at sites of prolonged or repeated inflammatory activity.

The term "injury," as used herein, refers to damage or harm to a structure or function of the body caused by an outside agent or force, which may be physical or chemical.

The term "insufflation" as used herein refers to the act of delivering air, a gas, or a powder under pressure to a cavity or chamber of the body. For example, nasal insufflation relates to the act of delivering air, a gas, or a powder under pressure through the nose.

The term "isomer" as used herein refers to one of two or more molecules having the same number and kind of atoms and hence the same molecular weight, but differing in chemical structure. Isomers may differ in the connectivities of the atoms (structural isomers), or they may have the same atomic connectivities but differ only in the arrangement or configuration of the atoms in space (stereoisomers). Stereoisomers may include, but are not limited to, double bond isomers, enantiomers, and diastereomers. Enantiomers are non-superimposable mirror images. A mixture of equal parts of the optical forms of a compound is known as a racemic mixture or racemate. Diastereomers are stereoisomers that are not mirror images. Stereoisomers may include enantiomers, diastereomers, or E or Z alkene, imine or oxime isomers. Stereoisomeric mixtures include racemic mixtures, diastereomeric mixtures, or E/Z isomeric mixtures. Stereoisomers can be synthesized in pure form (Nogradi, M.; Stereoselective Synthesis, (1987) VCH Editor Ebel, H. and Asymmetric Synthesis, Volumes 3-5, (1983) Academic Press, Editor Morrison, J.) or they can be resolved by a variety of methods such as crystallization and chromatographic techniques (Jaques, J.; Collet, A.; Wilen, S.; Enantiomer, Racemates, and Resolutions, 1981, John Wiley and Sons and Asymmetric Synthesis, Vol. 2, 1983, Academic Press, Editor Morrison, J). Unless specified otherwise, the formulations of the present disclosure are meant to include all such possible isomers, including racemic mixtures, optically pure forms and intermediate mixtures. Optically active (R)- and (S)- isomers may be

**EP 4 631 364 A2**

prepared using chiral synthons or chiral reagents, or resolved using conventional techniques. If the compound contains a double bond, the substituent may be E or Z configuration. If the compound contains a disubstituted cycloalkyl, the cycloalkyi substituent may have a cis- or transconfiguration. All tautomeric forms are also intended to be included.

The term "interleukin" or "IL" as used herein is a generic name for cytokines produced by leukocytes.

The term "interleukin-1β" or "IL-1β" as used herein refers to a member of the IL-1 family of inflammatory cytokines. As of 2019, human sequence algorithm technologies suggest that the IL-1 family comprises a total of 11 members with similar or distinct biological effects [32, 33]. IL-1α, IL-1β, IL-1Ra, IL-18, IL-33, IL-36α, IL-36β, IL-36γ, IL-36Ra IL-37, and IL-38 have been identified and characterized [Kaneko, N. et al. Inflammation and Regeneration (2019) 39: article 12, citing Dinarello, C. et al. Nature Immunol. (2010) 11: 973]. Among them, IL-1α, IL-1β, IL-18, IL-33, and IL-36 are receptor-agonistic, and IL-1Ra, IL-36Ra, and IL-38 are receptor-antagonistic. IL-37 is the only anti-inflammatory cytokine. There are two individual forms of IL-1, IL-1α and IL-1β, isolated from two distinct cDNAs, but they are indistinguishable in terms of their biological functions [Id., citing Lachman, LB et al. J. Immunol. 91977] 119: 2019-23]. Although the homology between IL-1α and IL-1β is not high (27%) in terms of amino acid sequences, IL-1α and IL-1β are structurally similar and show the same functions by sharing a common receptor, IL-1 type 1 receptor (IL-1R1). IL-1R1 initiates inflammatory responses when binding to the ligands IL-1α and IL-1β and has been reported to be expressed by T-lymphocytes, fibroblasts, epithelial cells, and endothelial cells.

The term "interleukin-6" or "IL-6" as used herein refers to a pleiotropc cytokine involved not only in immune responses but also in inflammation, hematopoiesis, bone metabolism, embryonic development and other fundamental processes [Hirano, T. Intl Rev. Immunol. (1998) 16: 249; Hunter, CA and Jones, SA. Nat. Immunol. (2015) 16: 448; Van Snick, J. Annu. Rev. Immunol. (1990) 8: 253; Heinrich, PC et al. Biochem. J. (2003) 374 (Pt. 1): 1; Kamimura, D. et al. Rev. Physiol. Biochem. Pharmacol. (2003) 149: 1; Hasegawa, H. et al. Front. Immunol. (2016) 7: 479]. IL-6 is a prototypical member of the IL-6 family of cytokines, which is composed of 10 members including IL-6, IL-11, IL-27, oncostatin M (OSM), leukemia inhibitory factor (LIF), ciliary neurotrophic factor (CNTF), cardiotrophin 1 (CT-1), cardiotrophin-like cytokine factor 1 (CLCF1), IL-35 and IL-39 [Id., citing Murakami, M. et al. Immunity (2019) 50: 812]. IL-6 is involved in cell senescence, is produced by senescent cells and is critically involved in senescence-induced inflammation and age-dependent pathologies and cancer [Id., citing Kuilman, T. et al. Cell (2008) 133: 1019]. IL-6 is also a key factor in inflammation, autoimmunity and cancer, with its effect mainly exerted through the IL-6-signal transducer and activator of transcription 3 (STAT3) pathway [Id., citing Grivennikov, S. et al. Cancer Cell (2009) 15: 103; Yu;, H. et al. Nat. Rev. Cancer (2009) 9: 798; Jones, SA and Jenkins, BJ. Nat. Rev. Immunol. (2018) 18: 773; Hirano, T. et al. Oncogene (2000) 19: 2548; Jenkins, BJ et al. Blood (2007) 109: 2380].

The term "interleukin 10" or "IL-10" as used herein refers to a pleotropic cytokine produced by almost all species of activated immune cells, including B cells, mast cells, granulocytes (e.g., neutrophils, basophils, eosinophils), macrophages, dendritic cells, and multiple T cell subsets. [Steen, EH et al. Adv. Wound Care (New Rochelle) (2020) 9 (4): 184-98, citing O'Garra, A. et al. Immunol. Rev. (20008) 223: 114-31]. Its principal actions are primarily considered anti-inflammatory, inhibitory, or self-regulating, in that IL-10 appears to be a potent negative feedback regulator that effects the control and resolution of inflammation via autocrine and paracrine mechanisms. This immunosuppressive effect is broad and occurs at both the cellular and humoral levels, although there are two dominant means by which IL-10 limits potentially damaging inflammatory responses: (1) inhibiting antigen presentation by dendritic cells and (2) inhibiting macrophage activation and infiltration into the site of injury, with the secondary effect of attenuating proinflammatory cytokine expression.[Id., citing O'Garra, A. et al. Immunol. Rev. (20008) 223: 114-31]. At the cellular level, IL-10 is believed to act as a posttranscriptional regulatory agent to suppress the messenger RNA (mRNA) stabilizing protein HuR (human antigen R), promoting the specific destabilization of inflammatory cytokine mRNA.[Id., citing Willis-Karp, M. et al. Mucosal Immunol. (2010) 3: 104-10]. In addition, IL-10 is thought to inhibit apoptotic signaling pathways, such as the p38 MAPK (mitogen-activated protein kinase) pathway, via signal transducer and activator of transcription 3 (STAT3)-dependent signaling, thereby limiting tissue death and organ dysfunction after injury.[Id., citing Rajasingh, J. et al. FASEB J. (2006) 20: 2112-14; Krishnamurthy, P. et al. Circ. Res. (2009) 104: e9-e18; Krishnamurthy, P. et al. FASEB J. (2010) 24: 2484-94]. IL-10 signals through a tetramer receptor complex (IL-10R) composed of two identical binding subunits IL-10Rα and two homolog signal-transducing IL-10Rβ subunits. Although some studies posit that IL-10 inhibits fibrosis primarily by regulating the inflammatory processes thought to be driving fibroproliferation, the molecular mechanisms behind this stated effect are still incompletely characterized. For example, long-term IL-10 exposure or IL-10 application to chronic disease processes may actually exacerbate tissue injury and promote fibrotic outcomes, despite being largely beneficial in the setting of acute inflammation and the early stages of wound healing.

The term "ketogenic diet" as used herein refers to a diet high in fat and low in carbohydrates (sugars) that causes the body to break down fat into molecules called ketones. Ketones circulate in the blood and become the main source of energy for many cells in the body.

The term "label" as used herein refers to a traceable constituent incorporated onto a molecule in order to spatially locate the molecule or follow it through a reaction or purification scheme. As a verb, it means to add such a group or atom that can be detected or measured.

The term "labile" as used herein refers to that which is subject to increased degradation.

The terms "LLOQ" or "lower limit of quantitation" are used interchangeably to refer to the lowest amount of an analyte in a sample that can be quantitatively determined with suitable precision and accuracy. Because bioanalytical assays are validated within a pre-specified range, results below LLOQ are BLQ (below the limit of quantification).

The term "logS" as used herein is the 10-based logarithm of the solubility measured in mol/l unit, that is logS = log (solubility measured in mol/l), which is a common unit for measuring solubility.

The term "macrophages" as used herein refers to large mononuclear phagocytic cells present in most tissues that have many functions, such as scavenger cells, pathogen-recognition cells, production of pro-inflammatory cytokines.

The term "microglia" as used herein refers to the smallest of the glial cells that can act as phagocytic cells, cleaning up CNS debris. They are considered to be a type of immune cell found in the brain. Microglia are close cousins of other phagocytic cells including macrophages and dendritic cells. Like macrophages, microglia are derived from myeloid progenitor cells from the bone marrow. During embryonic development, these cells migrate to the CNS where they differentiate into microglia.

The term "modulate" as used herein refers to regulating, altering, adapting, or adjusting to a certain measure or proportion.

The term "mutation" refers to a change of the DNA sequence within a gene or chromosome of an organism resulting in the creation of a new character or trait not found in the parental type, or the process by which such a change occurs in a chromosome, either through an alteration in the nucleotide sequence of the DNA coding for a gene or through a change in the physical arrangement of a chromosome. Three mechanisms of mutation include substitution (exchange of one base pair for another), addition (the insertion of one or more bases into a sequence), and deletion (loss of one or more base pairs).

The term "nasal spray" as used herein refers to a drug product that contains active ingredients dissolved or suspended in a formulation, typically aqueous-based, which can contain other excipients and are intended for use by nasal inhalation. Container closure systems for nasal sprays include the container and all components that are responsible for metering, atomization, and delivery of the formulation to the subject.

The term "neurofilament light" or "NfL" as used herein refers to a recognized biomarker of subcortical large-caliber axonal degeneration [Mielke, MM et al. Neurology (2019) 93 (3): e252-260, citing Hoffman, PN et al. Proc. Natl Acad. Sci. USA (1987) 84: 3472-6; Norgren, N. et al. Brain Res. (2003) 987: 25-31]. CSF and plasma NfL levels are elevated in multiple neurodegenerative disorders, including Alzheimer disease (AD dementia) [Id., citing Mattsson, N. et al. JAMA Neurol. (2017) 74: 557-66; Kern, S. et al. JAMA Neurol. (2019) 76 (2): 187-933], frontotemporal dementia [Id., citing Scherling, CS et al. Ann. Neurol. (2014) 75: 116-26], multiple sclerosis [Id., citing Teunissen, CE et al. Lancet Neurol. (2005) 4: 32-41] and TBI.

The term "neuroinflammation" as used herein refers to an inflammatory response in the central nervous system that is mounted by peripheral immune cells and glial cells, during which the permeability of the BBB often is increased.

The term "neuroprotective" as used herein refers to serving to protect nerve cells against damage, degeneration or impairment of function.

The term "nicotinic acid" as used herein refers to a B-complex vitamin that prevents pellagra (like nicotinamide). Daily requirements are 15-20 mg in adults. In higher doses (0.5-2g) nicotinic acid can activate the $HCA_2$ receptor.

As used herein the phrase "nutraceutically acceptable carrier" refers to any substantially non-toxic carrier useable for formulation and administration of the composition of the described invention in which the product of the described invention will remain stable and bioavailable. The pharmaceutically acceptable carrier must be of sufficiently high purity and of sufficiently low toxicity to render it suitable for administration to the mammal being treated. It further should maintain the stability and bioavailability of a nutraceutical agent. The pharmaceutically acceptable carrier can be liquid or solid and is selected, with the planned manner of administration in mind, to provide for the desired bulk, consistency, etc., when combined with an nutraceutical agent and other components of a given composition.

The term "nutraceutical" or "dietary supplement" as used herein refers to a food stuff that provides health benefits in addition to its basic nutritional value.

The terms "nutraceutical amount", or an "amount effective", of the active agent and used interchangeably to refer to an amount that is sufficient to provide the intended health benefit. dosage levels are based on a variety of factors, including the type of injury, the age, weight, sex, medical condition of the patient, the severity of the condition, the route of administration, and the particular active agent employed. Thus the dosage regimen may vary widely, but can be determined routinely by a physician using standard methods.

The term "nutraceutical effect", as used herein, refers to a result or consequence of exposure to an active ingredient that is not intended to diagnose, treat, cure or prevent any disease.

The term "nutraceutical load" as used herein refers to a ratio of the weight of a nutraceutical in particles relative to the total weight of the particles, expressed as a percentage. It reflects the nutraceutical content of the particle.

The term "optical activity" as used herein refers to the ability of many organic compounds to rotate the plane of plane-polarized light. In describing an optically active compound, the prefixes D and L or R and S are used to denote the absolute configuration of the molecule about its chiral center(s). The prefixes d and l or (+) and (-) are employed to designate the sign of rotation of plane-polarized light by the compound, with (-) or 1 meaning that the compound is levorotatory. A compound prefixed with (+) or d is dextrorotatory.

The term "parenteral" as used herein refers to a route of administration where the active or agent enters the body without going through the stomach or "gut", and thus does not encounter the first pass effect of the liver. Examples include, without limitation, otic, ophthalmic, intranasal, inhalation, topicals, suppositories, transbuccal, sublingual and introduction into the body by way of an injection (i.e., administration by injection), including, for example, subcutaneously (i.e., an injection beneath the skin), intramuscularly (i.e., an injection into a muscle); intravenously (i.e., an injection into a vein), intrathecally (i.e., an injection into the space around the spinal cord or under the arachnoid membrane of the brain), intraventricular injection, intracisternal injection, or infusion techniques.

The term "particle" as used herein refers to an extremely small constituent, e.g., nanoparticles or microparticles) that may contain in whole or in part at least one active agent as described herein. The term "microparticle" is used herein to refer generally to structures including microcapsules, microparticles, nanoparticles, nanocapsules, and nanospheres. The particles may contain the active agent(s) in a core surrounded by a coating. Therapeutic agent(s) also may be dispersed throughout the particles. The active agent(s) also may be adsorbed into the particles. The particles may be of any order release kinetics, including zero order release, first order release, second order release, delayed release, sustained release, immediate release, etc., and any combination thereof. The particles may include, in addition to the active agent(s), any of those materials routinely used in the art of pharmacy and medicine, including, but not limited to, erodible, nonerodible, biodegradable, or nonbiodegradable material or combinations thereof. The particles may be microcapsules that contain the active agent in a solution or in a semi-solid state. The particles may be of virtually any shape.

The term "particle size distribution" as used herein refers to a statistical in indication of the percentage of particles of a certain size (or in a certain size interval). An exemplary descriptive way of representing a particle size distribution is the histogram, where the width of a bar corresponds to the lower or upper limit of the size class and the height of the bar corresponds to the quantity in that size class. In particle measurement technology, it is common to generate a cumulative distribution from the class-dependent values. For this purpose, the quantities in each measurement class are summed up, starting with the smallest fraction. This produces a curve that increases continuously from 0 % to 100 %, the "cumulative curve". Cumulative particle size distributions are denoted by the letter Q. Each value Q(x) indicates the amount of the sample consisting of particles smaller than size x. Many statistical parameters can be derived from a particle size distribution. The cumulative distribution is particularly suitable for this purpose. Among the most important parameters are the percentiles. These indicate in each case the size x below which a certain quantity of the sample lies. Percentiles are denoted by the letter d followed by the % value. Mean values (or mean particle size) can also be calculated from the tabulated values. This is done by multiplying the quantity in each measurement class by the mean size measurement class and summing the individual values. The cumulative curve is also useful in the determination of oversize and undersize particles, which are small portions of particles that are significantly larger or significantly smaller than the bulk of the sample.

The term "partition coefficient" (P) as used herein is the ratio of the concentration of a substance in lipid to that of the aqueous phase. It is thus a measure of lipophilicity. The higher the P, the greater the amount of the substance in the lipid phase (typically octanol) and the greater the lipophilicity. The log of P ("log P) is commonly used for convenience. When P <1, log P is negative, and the greater the compound solubility in the aqueous phase. When P > 1, log P is positive, and the compound favors the lipid phase. The term "Log D" as used herein is the log P of a compound at a particular pH (e.g., log $D_{5.5}$). According to some embodiments, log P for beta-hydroxybutyrate is predicted to range from -0.49 to -0.39.

The term "peripheral nervous system" ("PNS") as used herein refers to the part of the nervous system that lies outside the brain and spinal cord that connects the CNS to sensory organs, other organs of the body, muscles, blood vessels and glands. It includes the 12 cranial nerves, the spinal nerves and roots and the autonomic nerves concerned with regulation of the heart muscle, the muscles in blood vessel walls and glands.

The term "pharmaceutical composition" is used herein to refer to a composition that is employed to prevent, reduce in intensity, cure or otherwise treat a target condition or disease.

The term "pharmaceutically acceptable carrier" as used herein refers to any substantially non-toxic carrier conventionally useable for administration of pharmaceuticals in which the synthetic compound of the described invention will remain stable and bioavailable. The pharmaceutically acceptable carrier must be of sufficiently high purity and of sufficiently low toxicity to render it suitable for administration to the mammal being treated. It further should maintain the stability and bioavailability of an active agent. The pharmaceutically acceptable carrier can be liquid or solid and is selected, with the planned manner of administration in mind, to provide for the desired bulk, consistency, etc., when combined with an active agent and other components of a given composition.

The term "pharmaceutically acceptable salt" as used herein refers to those salts which are, within the scope of sound medical judgment, suitable for use in contact with the tissues of humans and lower animals without undue toxicity, irritation, allergic response and the like and are commensurate with a reasonable benefit/risk ratio. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts may be prepared as alkaline metal or alkaline earth salts, such as sodium, potassium or calcium salts of the carboxylic acid group. By "pharmaceutically acceptable salt" is meant those salts which are, within the scope of sound medical judgment, suitable for use in contact with the tissues of humans and lower animals without undue toxicity, irritation, allergic response and the like and are commensurate with a reasonable benefit/risk ratio. Pharmaceutically acceptable salts are well-known in the art. For example, P. H. Stahl, et al. describe pharmaceutically acceptable salts in detail in "Handbook of Pharmaceutical Salts: Properties, Selection, and Use" (Wiley VCH, Zurich, Switzerland: 2002). The salts may be prepared in situ during the final isolation and purification of the compounds described within the present invention or separately by reacting a free base function with a suitable organic acid. Representative acid addition salts include, but are not limited to, acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate(isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups may be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid. Basic addition salts may be prepared in situ during the final isolation and purification of compounds described within the invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine and the like. Pharmaceutically acceptable salts also may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium or magnesium) salts of carboxylic acids may also be made.

The term "plume width" as used herein refers to the width of the plume at a given distance (e.g. 3 cm) from the spray nozzle.

The term "polymer" as used herein refers to a large molecule, or macromolecule, composed of many repeated subunits of identical structure (monomers). Polymers with two different repeating units in their chains are called copolymers.

The term "powder" as used herein refers to fine, dry particles produced by the grinding, crushing, or disintegration of a solid substance

The term "pump" as used herein refers to all of the components of a container closure system responsible for metering, atomization, and delivery of a formulation to the subject.

The term "receptor" as used herein refers to a protein that binds a specific extracellular signal molecule (ligand) and initiates a response in the cell. Cell-surface receptors are located in the plasma membrane, with their ligand-binding site exposed to the external medium. Intracellular receptors bind ligands that diffuse into the cell across the plasma membrane.

The term "reduce" or "reducing" as used herein refers to a diminution, a decrease, an attenuation, limitation or abatement of the degree, intensity, extent, size, amount, density, number or occurrence.

The term "release" and its various grammatical forms, refers to dissolution of an active component and diffusion of the dissolved or solubilized species by a combination of the following processes: (1) hydration of a matrix, (2) diffusion of a solution into the matrix; (3) dissolution of the active; and (4) diffusion of the dissolved active out of the matrix.

The term "second messengers - such as cyclic AMP [cAMP], diacylglycerol [DAG], and inositol 1, 4, 5-triphosphate [IP3] -as used herein refers to small molecules that initiate and coordinate intracellular signaling pathways.

The terms "soluble" and "solubility" refer to the property of being susceptible to being dissolved in a specified fluid (solvent). The term "insoluble" refers to the property of a material that has minimal or limited solubility in a specified solvent.

A "solution" generally is considered as a homogeneous mixture of two or more substances. It is frequently, though not necessarily, a liquid. In a solution, the molecules of the solute (or dissolved substance) are uniformly distributed among those of the solvent.

The term "spray" as used herein refers to a system of liquid droplets in a fluid continuous phase.

The term "spray angle" as used herein refers to the angle of an emitted plume measured from the vertex of the spray cone and spray nozzle.

The terms "subject" or "individual" or "patient" are used interchangeably to refer to a member of an animal species of mammalian origin, including but not limited to, mouse, rat, cat, goat, sheep, horse, hamster, pig, dog, guinea pig, rabbit and a primate, such as, for example, a monkey, ape, or human.

The phrase "subject in need thereof" as used herein refers to a subject that (i) is at risk for a traumatic brain injury comprising neuroinflammation; (ii) is suffering from a traumatic brain injury comprising neuroinflammation; or (iii) has suffered a traumatic brain injury comprising neuroinflammation, unless the context and usage of the phrase indicates otherwise.

The term "support" is used herein to refer to sustain, supply, maintain, assist.

The term "susceptible" as used herein refers to a member of a population at risk.

A "suspension" is a dispersion (mixture) of finely divided, undissolved substances dispersed in liquid vehicles, with the former being so finely divided and mixed that it doesn't rapidly settle out. In everyday life, the most common suspensions are those of solids in liquid.

The term "sustained release" (also referred to as "extended release") is used herein in its conventional sense to refer to an active formulation that provides for gradual release of an active over an extended period of time, and that preferably, although not necessarily, results in substantially constant blood levels of an active over an extended time period. Alternatively, delayed absorption of a parenterally administered active form Is accomplished by dissolving or suspending the active in an oil vehicle. Non-limiting examples of sustained release biodegradable polymers include polyesters, polyester polyethylene glycol copolymers, polyamino-derived biopolymers, polyanhydrides, hydrogels, polyorthoesters, polyphosphazenes, SAIB, photopolymerizable biopolymers, protein polymers, collagen, polysaccharides, chitosans, and alginates.

The term "symptom" as used herein refers to a sign or an indication of disorder or disease, especially when experienced by an individual as a change from normal function, sensation, or appearance.

The term "synthetic" as used herein refers to being made by a chemical process, or altered by deliberate human intervention. As used herein, "synthetic" includes products derived from biofermentation.

The term "T cell" or "T lymphocyte" as used herein refers to one of the two types of antigen-specific lymphocytes responsible for adaptive immune responses, the other being B cells. T cells are responsible for the cell-mediated adaptive immune reactions. They originate in the bone marrow but undergo most of their development in the thymus. The highly variable antigen receptor on T cells is called the T cell receptor. Effector T cells perform a variety of functions in an immune response, acting always by interacting with another cell in an antigen-specific manner. Some T cells activate macrophages, some help B cells produce antibody, and some kill cells infected with viruses and other intracellular pathogens.

The term "targeted delivery" as used herein refers to a system of specifying an active moiety directly into its targeted body area (organ, cellular, and subcellular level of specific tissue) to overcome specific toxic effects of conventional active delivery, thereby reducing the amount of active agent required for efficacy.

The term "therapeutic amount", is an amount that is sufficient to provide the intended benefit of treatment. Combined with the teachings provided herein, by weighing factors such as potency, relative bioavailability, subject body weight, severity of adverse side-effects and preferred mode of administration, an effective prophylactic or therapeutic treatment regimen may be planned which does not cause substantial toxicity and yet is effective to treat the particular subject.

The term "therapeutic component" as used herein refers to a therapeutically effective dosage (i.e., dose and frequency of administration) that eliminates, reduces, or prevents the progression of a particular disease manifestation in a percentage of a population. An example of a commonly used therapeutic component is the ED50, which describes the dose in a particular dosage that is therapeutically effective for a particular disease manifestation in 50% of a population.

The term "therapeutic effect" as used herein refers to a consequence of treatment, the results of which are judged to be desirable and beneficial. A therapeutic effect may include, directly or indirectly, the arrest, reduction, or elimination of a disease manifestation. A therapeutic effect also may include, directly or indirectly, the arrest reduction or elimination of the progression of a disease manifestation.

The term "therapeutic window" as used herein reflects a concentration range that provides efficacy without unacceptable toxicity. The duration of a substance's action is determined by the time period over which concentrations exceed the minimum effective concentration (MEC). Generally, another dose can be administered to maintain concentrations within the therapeutic window over time.

The term "treat" or "treating" includes abrogating, substantially inhibiting, slowing or reversing the progression of a disease, condition or disorder, substantially ameliorating clinical or esthetical symptoms of a condition, substantially preventing the appearance of clinical or esthetical symptoms of a disease, condition, or disorder, and protecting from harmful or annoying symptoms. Treating further refers to accomplishing one or more of the following: (a) reducing the severity of the disorder; (b) limiting development of symptoms characteristic of the disorder(s) being treated; (c) limiting worsening of symptoms characteristic of the disorder(s) being treated; (d) limiting recurrence of the disorder(s) in patients that have previously had the disorder(s); and (e) limiting recurrence of symptoms in patients that were previously asymptomatic for the disorder(s).

The term "ubiquitin C-terminal hydrolase L1" or "UCHL1" as used herein refers to a neuron-specific cytoplasmic enzyme, highly enriched in neurons. It is an indicator of acute injury to brain neurons. It is involved in the degradation of unwanted, misfolded or damaged proteins. Increased CSF and blood concentrations of UCH-L1 have been associated with processes of neuron destruction (loss) and increased blood brain barrier permeability. After TBI, blood UCH-L1 level correlates with injury severity. [Mondello, S. et al. BMC Neurology (2012) 12: article 85].

The term "ULOQ" or "upper limit of quantification" are used interchangeably to refer to the highest concentration in the calibration curve that can be determined with a given analytical assay with the required precision and accuracy. Values above the ULOQ are ALQ (above the limit of quantitation).

EMBODIMENTS

## Compositions

According to one aspect, the present disclosure provides a composition comprising an active ingredient that is encapsulated in a suspension of particles comprising a biodegradable polymer containing an effective amount of the active ingredient, wherein the particles are configured to bypass the blood brain barrier, wherein the active ingredient is a synthetic D-beta hydroxybutyrate, and wherein targeted delivery is to the brain.

According to some embodiments, the synthetic beta-hydroxybutyrate is soluble in water.

According to some embodiments, size of the particles is greater than 10 nm in diameter, greater than 15 nm in diameter; greater than 20 nm in diameter, greater than 30 nm in diameter, greater than 40 nm in diameter, greater than 50 nm in diameter, greater than 60 nm in diameter, greater than 70 nm in diameter, greater than 80 nm; in diameter, greater than 90 nm in diameter, greater than 100 nm, greater than 200 nm, greater than 300 nm, greater than 400 nm, greater than 500 nm, greater than 600 nm, greater than 700 nm, greater than 800 nm, greater than 900 nm, greater than 1000 nm, greater than 2000 nm, greater than 3000 nm, greater than 4000 nm, greater than 5000 nm, greater than 6000 nm, greater than 7000 nm, greater than 8000 nm, greater than 9000 nm, greater than 10,000 nm, etc.[See, e.g., Ohta, S.et al. Investigating the optimum size of nanoparticles for their delivery into the brain assisted by focused ultrasound-induced blood brain barrier opening. Sci. Reports (2020) article 18220].

According to some embodiments, the synthetic D-beta hydroxybutyrate is dispersed throughout the particles; or the particles are impregnated with the synthetic D-beta hydroxybutyrate; or the particles comprise a matrix and the matrix comprises the synthetic D-beta hydroxybutyrate.

According to some embodiments, a fraction of the synthetic D-beta hydroxybutyrate is adsorbed or weakly bound to the surface of the microparticle and contributes to a rapid initial release or burst release.

According to some embodiments, the particles are of any order release kinetics, including zero order release, first order release, second order release, delayed release, sustained release, immediate release, long term release, or a combination thereof.

According to some embodiments, release of the synthetic D-beta hydroxybutyrate from the particles is through diffusion, erosion or both.

## Routes of delivery/administration

The described invention relates to all routes of administration including intramuscular, subcutaneous, sublingual, intravenous, intraperitoneal, intranasal, intratracheal, topical, intradermal, intramucosal, intracavernous, intrarectal, into a sinus, gastrointestinal, intraductal, intrathecal, intraventricular, intrapulmonary, into an abscess, intraarticular, subpericardial, into an axilla, into the pleural space, intradermal, intrabuccal, transmucosal, transdermal, via inhalation, and via subcutaneous injection.

Administering as used herein includes in vivo administration, as well as administration directly to tissue ex vivo. Generally, compositions may be administered systemically, i.e., orally, buccally, parenterally, or rectally, in single or multi-dosage unit formulations containing nontoxic pharmaceutically acceptable carriers, adjuvants, and vehicles as desired.

According to some embodiments, the route of delivery is via oral administration. The compositions of the described invention may be in a form suitable for oral use, for example, as tablets, troches, lozenges, aqueous or oily suspensions, dispersible powders or granules, emulsions, hard or soft capsules or syrups or elixirs. As used herein, the terms "oral" or "'orally" refer to the introduction into the body by mouth whereby absorption occurs in one or more of the following areas of the body: the mouth, stomach, small intestine, lungs (also specifically referred to as inhalation), and the small blood vessels under the tongue (also specifically referred to as sublingually). Compositions intended for oral use may be prepared according to any known method, and such compositions may contain one or more agents selected from the group consisting of sweetening agents, flavoring agents, coloring agents, and preserving agents in order to provide pharmaceutically elegant and palatable preparations. Tablets may contain the active ingredient(s) in admixture with non-toxic pharmaceutically-acceptable excipients which are suitable for the manufacture of tablets. These excipients may be. for example, inert diluents, such as calcium carbonate, sodium carbonate, lactose, calcium phosphate or sodium phosphate; granulating and disintegrating agents, for example, com starch or alginic acid; binding agents, for example, starch, gelatin or acacia; and lubricating agents, for example, magnesium stearate.-stearic acid or talc. The tablets may be uncoated or they may be coated by known techniques, for example, to delay disintegration and absorption in the gastrointestinal tract and thereby provide a sustained action over a longer period, to protect the composition from oxidation or photodegradation; or for controlled release. For example, a time delay material, such as glyceryl monostearate or glyceryl distearate, may be employed.

Compositions of the described invention also may be formulated for oral use as hard gelatin capsules, where the active ingredient(s) is(are) mixed with an inert solid diluent, for example, calcium carbonate, calcium phosphate or kaolin, or soft gelatin capsules wherein the active ingredient(s) is (are) mixed with water or an oil medium, for example, peanut oil, liquid paraffin, or olive oil.

The compositions of the described invention may be formulated as aqueous suspensions wherein the active ingredient(s) is (are) in admixture with excipients suitable for the manufacture of aqueous suspensions. Such excipients are suspending agents, for example, sodium carboxymethylcellulose, methylcellulose, hydroxy- propylmethylcellulose, sodium alginate, polyvinylpyrrolidone, gum tragacanth. and gum acacia; dispersing or wetting agents may be a naturally-occurring phosphatide such as lecithin., or condensation products of an alkylene oxide with fatty acids, for example, polyoxyethylene stearate, or condensation products of ethylene oxide with long chain aliphatic alcohols, for example, heptadecaethyleneoxycetanol. or condensation products of ethylene oxide with partial esters derived from fatty acids and a hexitol such as polyoxyethylene sorbitol monooleate. or condensation products of ethylene oxide with partial esters derived from fatty acids and hexitol anhydrides, for example polyethylene sorbitan monooleate. The aqueous suspensions also may contain one or more coloring agents, one or more flavoring agents, and one or more sweetening agents, such as sucrose or saccharin.

Compositions of the described invention may be formulated as oily suspensions by suspending the active ingredient in a vegetable oil, for example arachis oil. olive oil. sesame oil or coconut oil, or in a mineral oil. such as liquid paraffin. The oily suspensions may contain a thickening agent, for example, beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Compositions of the described invention may be formulated in the form of dispersible powders and granules suitable for preparation of an aqueous suspension by the addition of water. The active ingredient in such powders and granules is provided in admixture with a dispersing or wetting agent, suspending agent, and one or more preservatives. Suitable dispersing or wetting agents and suspending agents are exemplified by those already mentioned above. Additional excipients, for example, sweetening, flavoring and coloring agents also may be present.

The compositions of the invention also may be in the form of an emulsion. An emulsion is a two-phase system prepared by combining two immiscible liquid carriers, one of which is disbursed uniformly throughout the other and consists of globules that have diameters equal to or greater than those of the largest colloidal particles. The globule size is critical and must be such that the system achieves maximum stability. Usually, separation of the two phases will not occur unless a third substance, an emulsifying agent, is incorporated. Thus, a basic emulsion contains at least three components, the two immiscible liquid carriers and the emulsifying agent, as well as the active ingredient.

Most emulsions incorporate an aqueous phase into a non-aqueous phase (or vice versa).

However, it is possible to prepare emulsions that arc basically non-aqueous, for example, anionic and cationic surfactants of the non-aqueous immiscible system glycerin and olive oil. Thus, the compositions of the invention may be in the form of an oil-in-water emulsion. The oily phase may be a vegetable oil, for example, olive oil or arachis oil, or a mineral oil. for example a liquid paraffin, or a mixture thereof Suitable emulsifying agents may be naturally-occurring gums, for example, gum acacia or gum tragacanlh, naturally-occurring phosphatides, for example soy bean., lecithin, and esters or partial esters derived from fatty acids and hexitol anhydrides, for example sorbitan monooleate, and condensation products of the partial esters with ethylene oxide, for example, polyoxyethylene sorbitan monooleate. The emulsions also may contain sweetening and flavoring agents.

The compositions of the invention also may be formulated as syrups and elixirs. Syrups and elixirs may be formulated with sweetening agents, for example, glycerol, propylene glycol, sorbitol or sucrose. Such formulations also may contain a demulcent, a preservative, and flavoring and coloring agents. Demulcents are protective agents employed primarily to alleviate irritation, particularly mucous membranes or abraded tissues. Λ number of chemical substances possess demulcent properties. These substances include the alginates, mucilages, gums, dextrins, starches, certain sugars, and polymeric polyhydric glycols. Others include acacia, agar, benzoin, carbomer, gelatin, glycerin, hydroxyethyl cellulose, hydroxypropyl cellulose, hydroxypropyl methylcellulose, propylene glycol, sodium alginate, tragacanth, hydrogels and the like.

For buccal administration, the compositions of the described invention may take the form of tablets or lozenges formulated in a conventional manner.

There are three general methods of tablet preparation: the wet-granulation method: the dry-granulation method; and direct compression. The method of preparation and the added ingredients arc selected to give the tablet formulation the desirable physical characteristics allowing the rapid compression of tablets. After compression, the tablets must have a number of additional attributes such as appearance, hardness, disintegration ability, appropriate dissolution characteristics, and uniformity, which also are influenced both by the method of preparation and by the added materials present in the formulation.

According to some embodiments, the tablet is a compressed tablet (CT). Compressed tablets are solid dosage forms formed with pressure and contain no special coating. Generally, they are made from powdered, crystalline, or granular materials, alone or in combination with binders, disintegrants, controlled-release polymers, lubricants, diluents and colorants.

According to some embodiments, the tablet is a sugar-coated tablet. These are compressed tablets containing a sugar coating. Such coatings may be colored and are beneficial in covering up active substances possessing objectionable tastes or odors and in protecting materials sensitive to oxidation.

According to some embodiments, the tablet is a film-coated tablet. These Compressed tablets are covered with a thin layer or film of a water-soluble material.

Numerous polymeric substances with film-forming properties may be used.

According to some embodiments, the tablet is an enteric-coated tablet. These Compressed tablets are coated with substances that resist solution in gastric fluid but disintegrate in the intestine.

According to some embodiments, the tablet is a multiple compressed tablet. These tablets are made by more than one compression cycle. Layered tablets are prepared by compressing additional tablet granulation on a previously compressed granulation. The operation may be repeated to produce multilayered tablets of two or three layers. Press-coated tablets (dry-coated) are prepared by feeding previously compressed tablets into a special tableting machine and compressing another granulation layer around the preformed tablets.

According to some embodiments, the tablet is a controlled-release tablet. Compressed tablets can be formulated to release the active slowly over a prolonged period of time. Hence, these dosage forms have been referred to as prolonged-release or sustained-release dosage forms.

According to some embodiments, the tablet is a tablet for solution. These Compressed tablets may be used to prepare solutions or to impart given characteristics to solutions.

According to some such embodiments, the tablet is an effervescent tablet. In addition to the active, these tablets contain sodium bicarbonate and an organic acid such as tartaric acid or citric acid. In the presence of water, these additives react, liberating carbon dioxide that acts as a disintegrator and produce effervescence.

According to some embodiments, the tablet is a compressed suppository or insert.

According to some embodiments, the tablet is a buccal and or sublingual tablet. These arc small, flat, oval tablets intended for buccal administration and that by inserting into the buccal pouch may dissolve or erode slowly.

According to some embodiments, the tablet is a molded tablet or tablet triturate.

In some embodiments, the tablet comprises a compressed core comprising at least one component of the described formulation; and a membrane forming composition. Formulations utilizing membrane forming compositions are known to those of skill in the art (see. for example. Remington's Pharmaceutical Sciences, 20th Ed., 2000). Such membrane forming compositions may include, for example, a polymer, such as, but not limited to, cellulose ester, cellulose ether, and cellulose ester-ether polymers, an amphophilic triblock copolymer surfactant, such as ethylene oxide-propylene oxide- ethylene oxide, and a solvent, such as acetone, which forms a membrane over the core. The compressed core may contain a bi-layer core including an active layer and a push layer.

According to some embodiments, when delivered orally, an effective amount of exogenous synthetic D-beta hydroxybutyrate may range from at least 500 mg to 100g, inclusive, i.e., at least 500 mg, at least 500 mg, 600 mg, 700 mg, 800 mg, 900 mg, 1 g, 2 g, 3 g, 4 g, 5 g, 6 g, 7 g, 8 g, 9 g, 10 g, 11 g, 12 g, 13 g, 14 g, 15 g, 16 g, 17 g, 18 g, 19g, 20 g, 21 g, 22 g, 23 g, 24 g, 25 g, 26 g, 27 g, 28 g, 29 g, 30 g, 31g, 32 g, 33 g, 34 g, 35 g, 36 g, 3 7g, 38 g, 39 g, 40 g, 41 g, 42 g, 43 g, 44 g, 45 g, 46 g, 47 g, 48 g, 49 g, 50 g, 51 g, 52 g, 53 g, 54 g, 55 g, 56 g, 57 g, 58 g, 59 g, 60 g, 61 g, 62 g, 63 g, 64 g, 65 g, 66 g, 67 g, 68 g, 69 g, 70 g, 71 g, 72 g, 73 g, 74 g, 75 g, 76 g, 77 g, 78 g, 79 g, 80 g, 81 g, 82 g, 83 g, 84 g, 85 g, 86 g , 87 g, 88 g, 89 g, 90 g, 91 g, 92 g, 93 g, 94 g, 95 g, 96 g, 97 g, 98 g, 9 9g, or 100 g, or from 10g-50 g, inclusive, i.e., at least 10 g, 11 g, 12 g, 13 g, 14 g, 15 g, 16 g, 17 g, 18 g, 19 g, 20 g, 21 g, 22 g, 23 g, 24 g, 25 g, 26 g, 27 g, 28 g, 29 g, 30 g, 31g, 32 g, 33 g, 34 g, 35 g, 36 g, 3 g, 38 g, 39 g, 40 g, 41 g, 42 g, 43 g, 44 g, 45 g, 46 g, 47 g, 48 g, 49 g, 50 g.

**Intranasal delivery of the dosage form.**

According to some embodiments, delivery is targeted to the brain by intranasal delivery.

FIG. 4 is an illustration in cross section showing internal nasal anatomy. The human nasal cavity is divided into two halves by the nasal septum, a central partition of bone and cartilage; each symmetrical half opens at the face via the nostrils and connects with the mouth at the nasopharynx. The nasal cavity is subdivided into the nasal vestibule, inferior turbinate, middle turbinate, superior turbinate, olfactory region, frontal sinus, sphenoidal sinus and the cribriform plate of the ethmoid bone. [Javia, A. et al., Polymers in nasal drug delivery: an overview. Ch. 11 in Applications of Polymers in Drug Delivery, 2d ed. Misra, A. and Shahiwala, A. Eds. Elsevier (2021) pp. 306-323].

The nasal vestibule has an area of about 0.6 cm$^2$ and is present on the anterior part of the nasal cavity, just inside the nostrils. Vibrissae, also known as nasal hairs, are present in this area, and they act as a filter unit for inhaled particles. This nasal portion is shielded by a stratified squamous epithelium and keratinized epithelium with sebaceous glands [Id., citing Marttin, E. et al Nasal mucociliary clearance as a factor in nasal drug delivery. Adv. Drug Deliv. Rev. (1998) 29: 13-38; Illum, L. Nasal drug delivery - possibilities, problems and solutions. J. Controlled Re. (2003) 87: 187-98]. These characteristics are necessary for resistance against toxic environmental substances but also affect the absorption of actives [Kimbell, JS et al. Correlation of regional formaldehyde flux predictions with the distribution of formaldehyde-induced squamous metaplasia in F344 rat nasal passages. Mutat. Res. Fund. Mol. Mech. Mutagen (1997) 380: 143-54].

The atrium is present midway between the nasal vestibule and the respiratory region. Its anterior section is a stratified squamous epithelium and the posterior area is pseudostratified columnar cells presenting microvilli [Id., citing Marttin, E. et al Nasal mucociliary clearance as a factor in nasal drug delivery. Adv. Drug Deliv. Rev. (1998) 29: 13-38].

In humans, the olfactory region (approximately 15 cm$^2$ ) is positioned on the superior turbinate opposite the septum, just below the cribriform plate of the ethmoid bone, which separates the cranial cavity from the nasal cavity, and accounts for about 5% of the total area of the nasal cavity [Id., citing Illum, L. Is nose-to-brain transport of drugs in man a reality? (2004) J. Pharm. Pharmacol. (2004) 56: 3-17] but is of interest in delivering substances to the CNS as it has a highly vascularized epithelial layer, bypasses the BBB, and has connectivity with the cerebrospinal fluid (CSF) and CNS [Id., citing Hanson, LR and Frey, WH Intranasal delivery bypasses the blood-brain barrier to target therapeutic agents to the central nervous system and treat neurodegenerative disease. (2008) BMC Neurosci. 9: S5; Illum, L. Nasal drug delivery - possibilities, problems and solutions. J. Controlled Re. (2003) 87: 187-98].

The blood-brain barrier (BBB) is located at the level of the cerebral microvasculature and is critical for maintaining central nervous system (CNS) homeostasis. Although the BBB restricts the entry of potentially neurotoxic substances into the brain, it also presents a major obstacle to the delivery of therapeutics into the CNS for disease treatment. The BBB exhibits a low rate of pinocytosis and possesses tight junctions (TJ) which form a seal between opposing endothelial membranes [Lockhead, JJ and Thorne, RG. "Intranasal delivery of biologics to the central nervous system." Adv. Drug Deliv Revs. (2012) 64: 614-28, citing Reese, TS and Karnovsky, MJ. J. Cell Biol. (1967) 34: 207-17]. The presence of TJ at the BBB creates a high transendothelial electrical resistance of 1500-2000 $\Omega \cdot cm2$ compared to 3-30 $\Omega \cdot cm2$ across most peripheral microvessels [Id., citing Crone, C. and Olesen, SP. Brain Res. (1982) 241: 49-55; Butt, AM et al. J. Physiol (1990) 429: 47-62]. This high resistance is associated with very low permeability, i.e. the BBB greatly restricts paracellular diffusion of solutes from the blood into the brain. Typically, only small, lipophilic molecules appreciably cross the normal, healthy BBB via transcellular passive diffusion, although some limited transport of certain peptides and peptide analogs has been reported [Id., citing Banks, WA. MBMC Neurol. (2009) 9 (Suppl. 1): S3]. Essential nutrients such as glucose or iron gain entry into the CNS through specific transporters such as the glucose transporter 1 or receptors such as the transferrin receptor [Id., citing Dick, AP et al. Proc. Natl Acad. Sci. USA (1984) 81: 7233-37; Jeffries, WA, et al. Nature (1984) 312: 162-3]. Receptors and transporters for gastrointestinal hormones involved in regulating metabolism are expressed at the BBB in order to convey information between the CNS and periphery [Id., citing Banks, WA. Regul. Pept. (20008) 149: 11-14]. In addition to its low paracellular permeability and low rate of pinocytosis, the BBB also expresses a high number of drug transporters (e.g. P-glycoprotein) which further restrict brain entry of many endogenous and exogenous substances that would otherwise be predicted to cross the BBB based on molecular weight (MW) and lipophilicity considerations. [Id].

The precise pathways and mechanisms by which a drug travels from the nasal epithelium to various regions of the CNS have not been fully elucidated. Substances can cross the olfactory membrane through three mechanisms: transport along olfactory and trigeminal nerve primary neurons of the olfactory epithelium and transport to the olfactory bulb by intracellular axonal transport; the substance permeates across the olfactory sustentacular epithelial cells, either by transcellular or paracellular mechanisms followed by uptake into the CNS; and pinocytosis by the olfactory neurons with successive possible distribution into more distant brain tissue [Kimbell, JS et al. Correlation of regional formaldehyde flux predictions with the distribution of formaldehyde-induced squamous metaplasia in F344 rat nasal passages. Mutat. Res. Fund. Mol. Mech. Mutagen (1997) 380: 143-54, citing Illum, L. Is nose-to-brain transport of drugs in man a reality? (2004) J. Pharm. Pharmacol. (2004) 56: 3-17].

The olfactory lobe provides a pathway for substances entering the CNS and peripheral circulation. A substance can enter the CNS by an intraneuronal pathway, which transports actives via axons, which require hours to days for the substances to reach the different regions of the brain. The extraneuronal pathway transports substances via perineural channels, which requires a few minutes for the substance to reach the parenchymal tissue or CSF [Singh, AK et al. Nasal cavity, a promising , a promising transmucosal platform for drug delivery and research approaches from nasal to brain targeting. J. Drug Deliv. Thera. (2012) 2]. Accordingly, nasal delivery has many advantages, such as fast absorption, rapid action, and low risk of overdose, over other routes of delivery [Id., citing Arora, P. et al. Permeability issues in nasal drug delivery. DrugDiscov. Today (2002) 7: 967-75, Ugwoke, ML et al. The biopharmaceutical aspects of nasal mucoadhesive drug delivery. J. Pharm. Pharmacol. (2001) 53: 3-22; Romeo, V. et al. Effects of physicochemical properties and other factors on systemic nasal drug delivery. Adv. Drug Deliv. Rev. (1998) 29: 89.

The respiratory epithelium is the major lining of the human nasal cavity and is essential for cleaning of the nasal mucosa by the nasal mucociliary clearance NMCC). The respiratory area is approximately 130 $cm^2$ [Id., citing Pires, A, et al. Intranasal drug delivery: how, why and what for? J. Pharm. Pharm. Sci. (2009) 12: 288-311]. It is composed of five types of cell: ciliated columnar cells, nonciliated columnar cells, basal cells (approximately 80%) , neurosecretory cells in the basement membrane, and goblet cells (approximately 20%). These cells help to avoid drying of mucosa by trapping moisture.

The nasopharynx is present in the posterior region; it has ciliated cells in the upper part and squamous epithelium in the lower part. About 20-40 ml of watery mucus per day are secreted by the goblet cells and the seromucous glands [Id., citing Quraishi, M. et al. The rheology of nasal mucus: a review. Clin. Otolaryngol. Allied Sci. (1998) 23: 403-13]. The nasopharynx consists of nasal associated lymphoid tissue [Id., citing Illum, L. Nanoparticulate systems for nasal delivery of drugs: a real improvement over simple systems" J. Pharm. Sci. (2007) 96: 473-83], which is situated immediately under the nasal mucosa. [Id., citing Stanley, A. et al. Characterization of ovine nasal-associated lymphoid tissue and identification of M cells in the overlying follicle-associated epithelium. J. Comp. Pathol. (2001) 125" 262-70]]. Substances administered by the nasal route may get absorbed wither into the systemic circulation or into the brain, or in many cases into both. Absorption is controlled by many factors, including cerebral influx of chemicals, active-metabolizing enzymes in the olfactory mucosa, the olfactory bulb and the brain itself.

**Biological barriers in nasal absorption**

Numerous studies have demonstrated intranasal (IN) drug delivery to effectively treat animal models of CNS diseases. Most of these studies did not show pharmacokinetic data which clearly demonstrated brain uptake of the nasally administered compound but rather presented pharmacodynamic data showing a positive effect following IN delivery of a substance in an animal model. [Lockhead, JJ et al. "Intranasal delivery of biologics to the central nervous system. Adv. Drug Deliv. Rev. (2012) 64 7]: 614-28]. This makes it difficult to ascertain whether the API bypassed the BBB via direct nose-to-brain pathways to enter the CNS, crossed the BBB to enter the CNS, or perhaps exerted its effects by acting on the brain endothelium.

*Mucus.* The first step of absorption by the nasal pathways is the passage through the mucus membrane. Lipophilic actives can easily cross biological membranes via the transcellular route as they are capable of partitioning into the lipid (bilayer) of the cell membrane. Thus, a lipophilic active can rapidly enter both the CSF and bloodstream after nasal administration; polar actives can only cross the mucous layer but are unable to cross the lipid layer. [[Javia, A. et al., Polymers in nasal drug delivery: an overview. Ch. 11 in Applications of Polymers in Drug Delivery, 2d ed. Misra, A. and Shahiwala, A. Eds. Elsevier (2021) pp. 306-323, citing Kimbell, JS et al. Correlation of regional formaldehyde flux predictions with the distribution of formaldehyde-induced squamous metaplasia in F344 rat nasal passages. Mutat. Res. Fund. Mol. Mech. Mutagen (1997) 380: 143-54., citing Sakane, T. et al. The transport of a drug to the cerebrospinal fluid directly from the nasal cavity: the relation to the lipophilicity of the drug. Chem. Pharm. Bull. (1991) 39: 2456-58 ].

*Nasal Mucociliary Clearance (NMCC).* The normal half-life time of mucociliary transit in humans is about 12-15 minutes [Id., citing Marttin, E. et al Nasal mucociliary clearance as a factor in nasal drug delivery. Adv. Drug Deliv. Rev. (1998) 29: 13-38]. The main components involved in nasal mucociliary clearance (NMCC) are the density of the cilia, periciliary fluid, and the composition of the mucus. The rate of NMCC is extremely variable in different areas and under different physiological conditions. Mucociliary activity is regulated by several factors, such as temperature, intracellular calcium and cyclic adenosine monophosphate (cAMP) levels, and by extracellular ATP, age, sex, posture, sleep, exercise, and common environmental pollutants that may affect the ciliary beat frequency and production of mucus resulting in increased mucociliary clearance rate. Disease conditions also can affect the NMCC [Id., citing Turker, S. et al. Nasal route and drug delivery systems. Pharm World Sci. (2004) 26: 137-42].

Absorption of a substance by the nasal mucosa depends on the time period for which the substance remains in contact with the nasal mucosa. Thus disturbance in the NMCC limits active absorption. To maximize active transport to the brain, the NMCC needs to increased ensuring prolonged contact time of the active at the absorption site [Id., citing Charlton, S. et al Evaluation of bioadhesive polymers as delivery systems for nose to brain delivery: in vitro characterization studies. J. Controlled Rel. (2007) 118: 225-34]. Efforts made for increasing the contact/residence time of the formulations in the nasal mucosa include the polymer properties such as mucoadhesiveness, pH sensitivity, and thermal gelation. [Id., citing Schaefer, ML et al. Trigeminal collaterals in the nasal epithelium and olfactory bulb: a potential route for direct modulation of olfactory information by trigeminal stimuli. J. comp. Neurol. (2002) 444: 221-26]

*Enzymic barrier.* The enzymic barrier of the nasal mucosa is mainly responsible for decreasing the bioavailability of peptides and proteins across the nasal mucosa. This barrier can be overcome by using enzyme inhibitors, absorption enhancers (e.g., bile salt, cyclodextrin, glycocholate) and peptide/protease inhibitors.

*P-glycoprotein efflux transporters.* P-glycoproteins (P-gps) are glycosylated membrane proteins present in the human nasal respiratory mucosa. [Id., citing Wioland, M-A et al. CFTR, MDR1 and MRP1 immunolocalization in normal human nasal respiratory mucosa. J. Histochem. Cytochem. (2000) 48: 1215-22]. Co-administration of a P-gp inhibitor as part of a nasal formulation increases the permeability of nasally administered active [Graff, CL and Pollack, GM. P-glycoprotein attenuates brain uptake of substrates after nasal instillation. Pharm. Res. (2003) 20: 1225-30 ].

*Physicochemical characteristics of the active.* The physicochemical characteristics of an active ingredient, which includes molecular weight, solubility, dissolution rate, charge, partition coefficient, acid dissociation constant (pKa), particle size and polymorphism, affect active absorption.

Substances of molecular weight less than 300 Da will easily permeate through aqueous channels of the membrane. For compounds having a molecular weight >300 Da, the rate of permeation is significantly affected. [Yamamoto, A. et al Absorption of water-soluble compounds with different molecular weights and [Asu1.7]-eel calcitonin from various mucosal administration sites. J. Controlled Rel. (2001) 76: 363-74].

Biological membrane permeability is mainly influenced by its lipophilicity [Id., citing Corbo, DC et al. Characterization of the barrier properties of mucosal membranes. J. Pharm. Sci. (1990) 79: 202-6], Low molecular weight lipophilic actives are absorbed quite efficiently across the nasal epithelium [Id., citing Hinchcliffe, M. and Illum, L. Intranasal insulin delivery and therapy. Adv. Drug Deliv. Rev. (1999) 35: 199-234].

Particles of different size accumulate in different areas of the respiratory tract, for example particles > 10 $\mu$m accumulate in the respiratory area via respiration; particles < 5 $\mu$m are inhaled and reach the lungs; and those < 0.5 $\mu$m are exhaled [Sanders, P. et al. Inhalers in healthy subjects and asthmatic patients. STP Pharma Sci. (1997) 7: 3000-76].

Dissolution is another rate-limiting step in nasal absorption, as nasal absorption will only take place after an active's dissolution. Thus, for nasal powder and suspension dosage forms, the dissolution rate is an important parameter. After nasal administration, if dissolution is slow, the particles will be cleared from the airway with a subsequent reduction in bioavailability [Romeo, V. et al Effects of physicochemical properties and other factors on systemic nasal drug delivery. Adv. Drug Deliv. Rev. (1998) 29: 39].

*Physicochemical properties of the formulation.*

*pH*: Because the nasal absorption and permeation of the active substance are often affected by the pKa value and pH of the formulation, the general rule is that pH therefore should be selected in the range between 4.5 and 6.5. [Javia, A. et al., Polymers in nasal drug delivery: an overview. Ch. 11 in Applications of Polymers in Drug Delivery, 2d ed. Misra, A. and Shahiwala, A. Eds. Elsevier (2021) pp. 306-323].

*Viscosity:* Although increasing the viscosity of the formulation may extend contact/residence time of the formulation in the nasal cavity, which may increase the permeation, highly viscous formulations may alter the regular functions of the nasal cavity, including mucociliary clearance and/or ciliary beat frequency. [Id.]

*Osmolarity.* More often, isotonic solutions/ formulations are administered in the nasal cavity for shrinkage of the nasal epithelium to increase the permeation of the substance due to structural changes. Isotonic solutions also inhibit ciliary activity. {Id.}

*Buffer capacity.* An acceptable buffer capacity of the formulation may be needed to maintain the pH. [Id.]

*Active concentration, dose and dose volume.* The higher the active concentration, the higher the absorption/permeation. The upper limit is 25 mg/dose. Dose volume ideally is in the range of 0.05-0.15 mL/dose, inclusive. [Id.]

*Polymers.* Polymers, such as cellulose derivatives, polyacrylates, starch, gelatin, phospholipids, chitosan, poly-N-alkyl acrylamide/poly-N-isopropylacrylamide, cyclodextrin, poloxamer and methylcellulose have proven effective in improving the intranasal absorption of therapeutic agents. [Id.]

*Starch microspheres.* Bioadhesive starch microspheres absorb water from nasal mucosa and help in paracellular transport. [Id., citing Illum, L. et al. Bioadhesive starch microspheres and absorption enhancing agents act synergistically to enhance the nasal absorption of polypeptides. Int. J. Pharm. (2001) 222: 109-19].

*Cyclodextrin.* Active solubility can be increased by encapsulating it in carriers, such as cyclodextrin. Intranasal delivery to the CNS along olfactory pathways can be specifically enhanced by using meta-cyclodextrin. [Id., citing Marttin, E. et al. Efficacy, safety and mechanism of cyclodextrins as absorption enhancers in nasal delivery of peptide and protein drugs. J. Drug Target (1998) 6: 17-36; Merkus, F. et al. Cyclodextrins in nasal drug delivery. Adv. Drug Deliv. Rev. (1999) 36: 41-57].

The disadvantage of a dosage form that is a solution, which suffers from NMCC, can be overcome by the combination of mucoadhesive and thermoreversible polymers.

*Thermoresponsive polymers.* A thermoresponsive polymer has a hydrophobic-hydrophilic balance in their structure, and they respond to small changes in temperature around the critical temperature by either chain collapse or chain expansion. Examples of thermoreversible polymers include gelatin, carrageenan, methylcellulose, hydroxypropyl methylcellulose (HPMC), xyloglucan, poly (N-isopropylacrylamide-c-acrylic acid), poly(N-isopropylacrylamide (PNIPAAm)/polyethylene oxide (PEO), poloxamer (Pluronic) and PEO/polylactic acid-co-glycolic acid (PLGA) [Id., citing Sharma, N. et al. Mucoadhesive thermoreversible nasal delivery system. J. Pharm. Res. (2010) 3: 991-97]. The factors affecting the thermoreversible properties of the formulation are concentration of the polymer, molecular weight, transition temperature, hydration value, polymer morphology, crystal state and polymerophism of polymers and phase separation of polymers. [Id., citing Wanka, G. et al. The aggregation behavior of poly-(oxyethylene)-poly-(oxypropylene)-poly-(oxyethylene) block copolymers in aqueous solution. Colloid Polym. Sci. (19990) 268: 101-17]. Methylcellulose and HPMC form a gel by the hydrophobic interaction between the methoxy substitutions. At lower temperature, there is little polymer-polymer interaction; as the temperature is raised, polymers lose their water of hydration and become a gel with low viscosity. After adequate dehydration of the polymer, association between the polymer-polymer occurs, and the total polymer structure behaves as an infinite gel network.

*pH sensitive polymers.* Polymers sensitive to pH have a low viscosity under normal ambient conditions but gel upon nasal administration as a result of the change in pH within the local nasal mucosa. [Id., citing Shaikh, RP et al. A review of multi-responsive membranous systems for rate-modulated drug delivery. AAPS PharmaSciTech. (2010) 11: 441-59]. This enhances nasal residence time and nasal absorption. These polymers are also descried as polyelectrolytes containing ionizable weak acid or basic moieties attached to a hydrophobic backbone in their structures. Polyacidic polymers with acidic groups remain unswollen at low pH, as the acidic groups of the polymer will be in protonated and unionized form. When the pH of the polymer environment is increased, a negatively charged polymer will swell. In contrast, polybasic polymers swell at a lower pH, as the basic group of the polymer will be in an ionized form. Examples of weak polyacidic pH sensitive anionic polymers are polycarboxylic acids, such as polyacrylic acid (PAA) or polymethacrylic acid and polysulfonamides [Id., citing Park, SY and Bae, YH, Novel pH-sensitive polymers containing sulfonamide groups. Macromol. Rapid Commun. (1999) 20: 269-73]. Depending on the electron-withdrawing nature of the substituent on the nitrogen, the pKa of weak polyacids varies between 3 and 11. Examples of cationic pH-sensitive polyelectrolytes include poly (N, N-dialkylaminoethylmethacrylates), polylysine, polyethyleneimine, and chitosan.

*Mucoadhesive polymer.* The nasal absorption of a substance can be improved by extending the retention of particles in the nasal mucosa. In nasal active delivery, mucoadhesion involves interaction between mucus secreted by the submucosal glands and the mucoadhesive polymer [Id., citing Salamat-Miller, N. et al. The use of mucoadhesive polymers in buccal drug delivery. Adv. Drug Deliv. Rev. (2005) 57: 1666-91]. The sequential steps of mucoadhesion include (1) absorption of water from the mucous layer leading to wetting and swelling of the polymer; (2) on swelling of the polymer, hydrogen bonds between the polymer chains are dissociated, leading to decreased polymer-polymer interaction and increased polymer-water interaction; the free polymer chains subsequently penetrate the mucus [Id., citing Ugwoke, ML et al Nasal mucoadhesive drug delivery: background, applications, trends and future perspectives. Adv. Drug Deliv. Rev. (2005) 57: 1640-65], and then interact with protein chains present in the nasal mucosa. Thus the formulation is retained in the nasal cavity, leading to an increased active concentration gradient across the epithelium. [Id., citing Jimenez-Castellanos, MR et al. Mucoadhesive drug delivery systems. Drug. Dev. Ind. Pharm. (1993) 19: 143-94]. Exemplary mucoadhesive polymers include chitosan, alginate, and cellulose or its derivatives.

*Ion-responsive polymers.* In this category, the sol-to-gel transition is due to the presence of mono/divalent cations such as Na+, K+, Ca2+ and Mg2+. Most wisely used polymers in this category are naturally occurring anionic polymers. Gellan gum, carrageenan, pectin, sodium alginate and xyloglucan have the property of cationic-induced gelation. [Id.]

According to some embodiments, for intranasal delivery, the maximum amount is about 25 mg/dose administered as frequently as tolerated, e.g. every 2 minutes, 5 minutes, 10 minutes, 15 minutes, 20 minutes, 30 minutes, 40 minutes 50 minutes, or 60 minutes for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 3;8 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours ,61 hours, 62 hours, 63 hr, 64 hr, 65 hr, 66 hr, 67 hr, 68 hr, 69 hr, 70 hr, 71 hr, 72 hours etc.

According to some embodiments, and based on HED from the mouse studies reported in the Examples below in para. [00635], the minimum daily dose for a 60 kg human (132 lb) is 250 mg/day. According to some embodiments, the minimum daily dose for a 90 kg human (200 lb) is 730 mg/day.

According to some embodiments, a dose time curve is determined so that a maximum 25 mg/dose delivered intranasally at a given frequency as tolerated, e.g., every 2 minutes, 5 minutes, 10 minutes, 15 minutes, 20 minutes, 30 minutes , 40 minutes, 50 minutes, or 60 minutes, for at least 1 hour, 2 hours, 3 hours, 4 hours, 5 hour, 6 hours, 7 hours, 8 hours, 9 hours, 10 hours, 11 hours, 12 hours, 13 hours,14 hours, 15 hours, 16 hours, 17 hours, 18 hours, 19 hours, 20 hours, 21 hours, 22 hours, 23 hours, or 24 hours, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. achieves the desired effective amount.

According to some embodiments, intranasal delivery within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before participating in an event where an acquired brain injury is a known risk and for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after participating in an event where an acquired brain injury is a known risk is neuroprotective. According to some embodiments, the event is a military operation. According to some embodiments, the event is a sports event. According to some embodiments, the sports event is, for example, hockey, football, soccer, baseball, polo, rugby, horseback riding, auto racing, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. Other applications include emergency medicine, for example use by EMTs or emergency departments in the context of such emergencies as domestic violence, falls, car crashes, etc.

According to some embodiments, integration of the D-beta-hydroxybutyrate into polymeric carriers can achieve controlled release, including delayed release, and sustained release.

According to some embodiments, the D-beta hydroxybutyrate binds HCA2.

According to some embodiments, the composition when delivered intranasally may elevate brain D-beta hydroxybutyrate levels, e.g., to about 0.1-3 mmol/L, or about 0.1 - 1 mmol/L for at least 6 hr to at least 48 hr, inclusive, i.e., at least 6 hr, at least 7 hr, at least 8 hr, at last 9 hr, at least 10 hr, at least 11 hr, at least 12 hr, at least 13 hr, at least 14 hr, at least 15 hr, at least 16 hr, at least 17 hr, at least 18 hr, at least 19 hr, at least 20 hr, at least 21 hr, at least 22 hr, at least 23 hr, at least 24 hr, at least 25 hr, at least 26 hr, at least 27 hr, at least 28 hr, at least 29 hr, at least 30 hr, at least 31 hr, at least 32 hr, at least 33 hr, at least 34 hr, at least 35 hr, at least 36 hr, at least 37 hr, at least 38 hr, at least 39 hr, at least 40 hr, at least 41 hr, at least 42 hr, at least 43 hr, at least 44 hr, at least 45 hr, at least 46 hr, at least 47 hr, at least 48 hr.

According to another aspect, the present disclosure provides a method for promoting healing of the brain after a traumatic brain injury comprising neuroinflammation comprising formulating a composition containing an active ingredient as a suspension of particles comprising a biodegradable polymer containing an effective amount of the active ingredient. administering to a subject the composition comprising the active ingredient, wherein the active ingredient is D-beta hydroxybutyrate; and targeting the composition to the brain; wherein healing comprises reducing neuroinflammation.

According to some embodiments, the administering is orally or intranasally.

According to some embodiments, when delivered orally, an effective amount of exogenous synthetic D-beta hydroxybutyrate may range from at least 500 mg to 100 g, inclusive, i.e., at least 500 mg, at least 500 mg, 600 mg, 700 mg, 800 mg, 900 mg, 1 g, 2 g, 3 g, 4 g, 5 g, 6 g, 7 g, 8 g, 9 g, 10 g, 11 g, 12 g, 13 g, 14 g, 15 g, 16 g, 17 g, 18 g, 19g, 20 g, 21 g, 22 g, 23 g, 24 g, 25 g, 26 g, 27 g, 28 g, 29 g, 30 g, 31g, 32 g, 33 g, 34 g, 35 g, 36 g, 3 7g, 38 g, 39 g, 40 g, 41 g, 42 g, 43 g, 44 g, 45 g, 46 g, 47 g, 48 g, 49 g, 50 g, 51 g, 52 g, 53 g, 54 g, 55 g, 56 g, 57 g, 58 g, 59 g, 60 g, 61 g, 62 g, 63 g, 64 g, 65 g, 66 g, 67 g, 68 g, 69 g, 70 g, 71 g, 72 g, 73 g, 74 g, 75 g, 76 g, 77 g, 78 g, 79 g, 80 g, 81 g, 82 g, 83 g, 84 g, 85 g, 86 g, 87 g, 88 g, 89 g, 90 g, 91 g, 92 g, 93 g, 94 g, 95 g, 96 g, 97 g, 98 g, 9 9g, or 100 g, or from 10g-50 g, inclusive, i.e., at least 10 g, 11 g, 12 g, 13 g, 14 g, 15 g, 16 g, 17 g, 18 g, 19 g, 20 g, 21 g, 22 g, 23 g, 24 g, 25 g, 26 g, 27 g, 28 g, 29 g, 30 g, 31g, 32 g, 33 g, 34 g, 35 g, 36 g, 3 g, 38 g, 39 g, 40 g, 41 g, 42 g, 43 g, 44 g, 45 g, 46 g, 47 g, 48 g, 49 g, 50 g.

According to some embodiments, for intranasal delivery, the minimum effective amount is a maximum of about 25 mg/dose administered as frequently as tolerated, e.g. every 2 minutes, 5 minutes, 10 minutes, 15 minutes, 20 minutes, 30 minutes, 40 minutes, 50 minutes, for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 3;8 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours ,61 hours, 62 hours, 63 hr, 64 hr, 65 hr, 66 hr, 67 hr, 68 hr, 69 hr, 70 hr, 71 hr, 72 hours etc. to achieve a minimum daily dose ranging from about 85 mg/day (e.g., for a child weighing about 45 lb.) to about 800 mg/day (e.g., for an adult) inclusive, i.e., at least about 85 mg/day; about 95 mg/day; about 105 mg/day, about 115 mg/day; about 125 mg/day;

about 135 mg/day; about 145 mg/day; about 155 mg/day; about 165 mg/day; about 175 mg/day; about 185 mg/day; about 195 mg/day; about 205 mg/day, about 215 mg/day; about 225 mg/day; about 235 mg/day; about 245 mg/day; about 255 mg/day; about 265 mg/day; about 275 mg/day; about 285 mg/day; about 295 mg/day; about 305 mg/day, about 315 mg/day; about 325 mg/day; about 335 mg/day; about 345 mg/day; about 355 mg/day; about 365 mg/day; about 375 mg/day; about 385 mg/day; about 395 mg/day; about 405 mg/day, about 415 mg/day; about 425 mg/day; about 435 mg/day; about 445 mg/day; about 455 mg/day; about 465 mg/day; about 475 mg/day; about 485 mg/day; about 495 mg/day; about 505 mg/day, about 515 mg/day; about 525 mg/day; about 535 mg/day; about 545 mg/day; about 555 mg/day; about 565 mg/day; about 575 mg/day; about 585 mg/day; about 595 mg/day; about 605 mg/day, about 615 mg/day; about 625 mg/day; about 635 mg/day; about 645 mg/day; about 655 mg/day; about 665 mg/day; about 675 mg/day; about 685 mg/day; about 695 mg/day; about 705 mg/day, about 715 mg/day; about 725 mg/day; about 735 mg/day; about 745 mg/day; about 755 mg/day; about 765 mg/day; about 775 mg/day; about 785 mg/day; about 795 mg/day; or about 800 mg/day.

According to some embodiments, and based on human equivalent dose (HED) from the mouse studies reported below in para. [00635], the minimum daily dose for a 60 kg human (132 lb) is 250 mg/day. According to some embodiments, the minimum daily dose for a 90 kg human (200 lb) is 730 mg/day.

According to some embodiments, a dose time curve is determined so that a maximum 25 mg/dose delivered intranasally at a given frequency tolerated, e.g., every 2 minutes, 5 minutes, 10 minutes, 15 minutes, 20 minutes, 30 minutes , 40 minutes, 50 minutes, or 60 minutes, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. achieves the desired effective amount.

According to some embodiments, intranasal delivery every 2 minutes, 5 minutes, 10 minutes, 15 minutes, 20 minutes, 30 minutes, 40 minutes, 50 minutes, or 60 minutes for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. before participating in an event where an acquired brain injury is a known risk and for at least 1 day, at least 2 days, at least 3 days, at least 4 days, at least 5 days, at least 6 days, at least 7 days, at least 8 days, least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after participating in an event where an acquired brain injury is a known risk is neuroprotective. According to some embodiments, the event is a military operation. According to some embodiments, the event is a sports event. According to some embodiments, the sports event is , for example, hockey, football, soccer, baseball, polo, rugby, horseback riding, autoracing, motorcycling, skiing, gymnastics, mountain climbing, Basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. Other applications include emergency medicine, for example use by EMTs or emergency departments in the context of such emergencies as domestic violence, falls, car crashes, etc.

According to some embodiments, the composition when delivered intranasally may elevate brain D-beta hydroxybutyrate levels, e.g., to approximately about 0.1-3 mmol/L, inclusive, or about 0.1 - 1 mmol/L, inclusive, for at least 6 hr to at least 48 hr, inclusive, i.e., at least 6 hr, at least 7 hr, at least 8 hr, at last 9 hr, at least 10 hr, at least 11 hr, at least 12 hr, at least 13 hr, at least 14 hr, at least 15 hr, at least 16 hr, at least 17 hr, at least 18 hr, at least 19 hr, at least 20 hr, at least 21 hr, at least 22 hr, at least 23 hr, at least 24 hr, at least 25 hr, at least 26 hr, at least 27 hr, at least 28 hr ,at least 29 hr, at least 30 hr, at least 31 hr, at least 32 hr, at least 33 hr, at least 34 hr, at least 35 hr, at least 36 hr, at least 37 hr, at least 38 hr, at least 39 hr, at least 40 hr, at least 41 hr, at least 42 hr, at least 43 hr, at least 44 hr, at least 45 hr, at least 46 hr, at least 47 hr, at least 48 hr .

According to some embodiments, the beta-hydroxybutyrate is soluble in water.

According to some embodiments, size of the particles is greater than 10 nm in diameter, greater than 15 nm in diameter; greater than 20 nm in diameter, greater than 30 nm in diameter, greater than 40 nm in diameter, greater than 50 nm in diameter, greater than 60 nm in diameter, greater than 70 nm in diameter, greater than 80 nm; in diameter, greater than 90 nm in diameter, greater than 100 nm, greater than 200 nm, greater than 300 nm, greater than 400 nm, greater than 500 nm, greater than 600 nm, greater than 700 nm, greater than 800 nm, greater than 900 nm, greater than 1000 nm, greater than 2000 nm, greater than 3000 nm, greater than 4000 nm, greater than 5000 nm, greater than 6000 nm, greater than 7000 nm, greater than 8000 nm, greater than 9000 nm, greater than 10,000 nm, etc. [See, e.g., Ohta, S.et al. Investigating the optimum size of nanoparticles for their delivery into the brain assisted by focused ultrasound-induced blood brain barrier opening. Sci. Reports (2020) article 18220].

According to some embodiments, the D-beta hydroxybutyrate is dispersed throughout the particles; or the particles are impregnated with the D-beta hydroxybutyrate; or the particles comprise a matrix and the matrix comprises the D-beta hydroxybutyrate.

According to some embodiments, a fraction of the D-beta hydroxybutyrate is adsorbed or weakly bound to the surface of the microparticles and contributes to a rapid initial release or burst release.

According to some embodiments, the particles are of any order release kinetics, including zero order release, first order release, second order release, delayed release, sustained release, immediate release, long term release, or a combination thereof.

According to some embodiments, release of the D-beta hydroxybutyrate from the particles is through diffusion, erosion or both.

According to another aspect, the present disclosure provides a method for promoting brain health comprising formulating a composition containing a suspension of particles comprising a biodegradable polymer containing an effective amount of the agent, and. administering to a subject the composition comprising the active ingredient, wherein the agent is synthetic D-beta hydroxybutyrate; and targeting the composition to the brain, wherein, burst release and controlled release of the synthetic D- beta-hydroxybutyrate f may lead to an effective amount of D-beta hydroxybutyrate levels in the brain.

According to some embodiments, the administering is orally or intranasally.

According to some embodiments, when delivered orally, the effective amount of exogenous synthetic D-beta hydroxybutyrate may range from at least 500 mg to 100 g, inclusive, i.e., at least 500 mg, at least 500 mg, 600 mg, 700 mg, 800 mg, 900 mg, 1 g, 2 g, 3 g, 4 g, 5 g, 6 g, 7 g, 8 g, 9 g, 10 g, 11 g, 12 g, 13 g, 14 g, 15 g, 16 g, 17 g, 18 g, 19g, 20 g, 21 g, 22 g, 23 g, 24 g, 25 g, 26 g, 27 g, 28 g, 29 g, 30 g, 31g, 32 g, 33 g, 34 g, 35 g, 36 g, 3 7g, 38 g, 39 g, 40 g, 41 g, 42 g, 43 g, 44 g, 45 g, 46 g, 47 g, 48 g, 49 g, 50 g, 51 g, 52 g, 53 g, 54 g, 55 g, 56 g, 57 g, 58 g, 59 g, 60 g, 61 g, 62 g, 63 g, 64 g, 65 g, 66 g, 67 g, 68 g, 69 g, 70 g, 71 g, 72 g, 73 g, 74 g, 75 g, 76 g, 77 g, 78 g, 79 g, 80 g, 81 g, 82 g, 83 g, 84 g, 85 g, 86 g, 87 g, 88 g, 89 g, 90 g, 91 g, 92 g, 93 g, 94 g, 95 g, 96 g, 97 g, 98 g, 9 9g, or 100 g, or from 10g-50 g, inclusive, i.e., at least 10 g, 11 g, 12 g, 13 g, 14 g, 15 g, 16 g, 17 g, 18 g, 19 g, 20 g, 21 g, 22 g, 23 g, 24 g, 25 g, 26 g, 27 g, 28 g, 29 g, 30 g, 31g, 32 g, 33 g, 34 g, 35 g, 36 g, 3 g, 38 g, 39 g, 40 g, 41 g, 42 g, 43 g, 44 g, 45 g, 46 g, 47 g, 48 g, 49 g, or 50 g.

According to some embodiments, when delivered intranasally, a maximum dose of 25 mg/dose is administered as frequently as tolerated, e.g. every 2 minutes, 5 minutes, 10 minutes, 15 minutes, 20 minutes, 30 minutes, 40 minutes, or 50 minutes for at least 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 3;8 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours ,61 hours, 62 hours, 63 hr, 64 hr, 65 hr, 66 hr, 67 hr, 68 hr, 69 hr, 70 hr, 71 hr, or 72 hours etc. to achieve a minimum daily dose ranging from about 85 mg/day (e.g., for a child weighing about 45 lb.) to about 800 mg/day (e.g., for an adult) inclusive, i.e., at least about 85 mg/day; about 95 mg/day; about 105 mg/day, about 115 mg/day; about 125 mg/day; about 135 mg/day; about 145 mg/day; about 155 mg/day; about 165 mg/day; about 175 mg/day; about 185 mg/day; about 195 mg/day; about 205 mg/day, about 215 mg/day; about 225 mg/day; about 235 mg/day; about 245 mg/day; about 255 mg/day; about 265 mg/day; about 275 mg/day; about 285 mg/day; about 295 mg/day; about 305 mg/day, about 315 mg/day; about 325 mg/day; about 335 mg/day; about 345 mg/day; about 355 mg/day; about 365 mg/day; about 375 mg/day; about 385 mg/day; about 395 mg/day; about 405 mg/day, about 415 mg/day; about 425 mg/day; about 435 mg/day; about 445 mg/day; about 455 mg/day; about 465 mg/day; about 475 mg/day; about 485 mg/day; about 495 mg/day; about 505 mg/day, about 515 mg/day; about 525 mg/day; about 535 mg/day; about 545 mg/day; about 555 mg/day; about 565 mg/day; about 575 mg/day; about 585 mg/day; about 595 mg/day; about 605 mg/day, about 615 mg/day; about 625 mg/day; about 635 mg/day; about 645 mg/day; about 655 mg/day; about 665 mg/day; about 675 mg/day; about 685 mg/day; about 695 mg/day; about 705 mg/day, about 715 mg/day; about 725 mg/day; about 735 mg/day; about 745 mg/day; about 755 mg/day; about 765 mg/day; about 775 mg/day; about 785 mg/day; about 795 mg/day; or about 800 mg/day.

According to some embodiments, and based on HED from the mouse studies reported below in para. [00635], the minimum daily dose for a 60 kg human (132 lb) is 250 mg/day. According to some embodiments, the minimum daily dose for a 90 kg human (200 lb) is 730 mg/day.

According to some embodiments, a dose time curve is determined so that a maximum 25 mg/dose delivered intranasally at a given frequency as tolerated, e.g., every 2 minutes, 5 minutes, 10 minutes, 15 minutes, 20 minutes, 30 minutes , 40 minutes, 50 minutes, or 60 minutes, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. achieves the desired effective amount.

According to some embodiments, intranasal delivery within 1 minute; within 5 minutes; within 10 minutes; within 15 minutes; within 30 minutes; within 45 minutes; within 1 hour, within 2 hours, within 3 hours, within 4 hours, within 5 hours,

within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before participating in an event where an acquired brain injury is a known risk and for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after participating in an event where an acquired brain injury is a known risk is neuroprotective. According to some embodiments, the event is a military operation. According to some embodiments, the event is a sports event. According to some embodiments, the sports event is, for example, hockey, football, soccer, baseball, polo, rugby, horseback riding, autoracing, motorcycling, skiing, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. Other applications include emergency medicine, for example use by EMTs or emergency departments in the context of such emergencies as domestic violence, falls, car crashes, etc.

According to some embodiments, the composition when delivered intranasally may elevate brain D-beta hydroxybutyrate levels, e.g., to approximately about 0.1-3 mmol/L, inclusive, or about 0.1 - 1 mmol/L, inclusive, for at least 6 hr to at least 48 hr, inclusive, i.e., at least 6 hr, at least 7 hr, at least 8 hr, at last 9 hr, at least 10 hr, at least 11 hr, at least 12 hr, at least 13 hr, at least 14 hr, at least 15 hr, at least 16 hr, at least 17 hr, at least 18 hr, at least 19 hr, at least 20 hr, at least 21 hr, at least 22 hr, at least 23 hr, at least 24 hr, at least 25 hr, at least 26 hr, at least 27 hr, at least 28 hr, at least 29 hr, at least 30 hr, at least 31 hr, at least 32 hr, at least 33 hr, at least 34 hr, at least 35 hr, at least 36 hr, at least 37 hr, at least 38 hr, at least 39 hr, at least 40 hr, at least 41 hr, at least 42 hr, at least 43 hr, at least 44 hr, at least 45 hr, at least 46 hr, at least 47 hr, at least 48 hr.

According to some embodiments, size of the particles is greater than 10 nm in diameter, greater than 15 nm in diameter; greater than 20 nm in diameter, greater than 30 nm in diameter, greater than 40 nm in diameter, greater than 50 nm in diameter, greater than 60 nm in diameter, greater than 70 nm in diameter, greater than 80 nm; in diameter, greater than 90 nm in diameter, greater than 100 nm, greater than 200 nm, greater than 300 nm, greater than 400 nm, greater than 500 nm, greater than 600 nm, greater than 700 nm, greater than 800 nm, greater than 900 nm, greater than 1000 nm, greater than 2000 nm, greater than 3000 nm, greater than 4000 nm, greater than 5000 nm, greater than 6000 nm, greater than 7000 nm, greater than 8000 nm, greater than 9000 nm, greater than 10,000 nm, or etc.[See, e.g., Ohta, S.et al. Investigating the optimum size of nanoparticles for their delivery into the brain assisted by focused ultrasound-induced blood brain barrier opening. Sci. Reports (2020) article 18220].

According to some embodiments, the D-beta hydroxybutyrate is dispersed throughout the particles; or the particles are impregnated with the D-beta hydroxybutyrate; or the particles comprise a matrix and the matrix comprises the D-beta hydroxybutyrate.

According to some embodiments, a fraction of the D-beta hydroxybutyrate is adsorbed or weakly bound to the surface of the microparticles and contributes to a rapid initial release or burst release.

According to some embodiments, the particles are of any order release kinetics, including zero order release, first order release, second order release, delayed release, sustained release, immediate release, long term release, or a combination thereof.

According to some embodiments, release of the D-beta hydroxybutyrate from the particles is through diffusion, erosion or both.

**Pharmaceutical compositions**

According to another aspect, the present disclosure provides a pharmaceutical composition comprising a pharmaceutically acceptable carrier and a therapeutic amount of an API, wherein the API is a synthetic D-beta-hydroxybutyrate (BHB), wherein targeted delivery is to the olfactory region of the nasal cavity, and wherein the targeted delivery achieves a therapeutically effective amount of the API in the brain.

According to some embodiments, the pharmaceutical composition comprises the active formulated as a solution, a suspension or a dispersion. According to some embodiments, the API is formulated as a solution. According to some embodiments, the API is formulated as a suspension. According to some embodiments, the API is formulated as a suspension, wherein the suspension comprises a particle size distribution of a population of particles comprising the API. According to some embodiments, the API is formulated as a dispersion. According to some embodiments, the API is formulated as a dispersion, wherein the dispersion comprises a particle size distribution of a population of particles comprising the API.

According to some embodiments, the pharmaceutical composition comprises a liquid spray formulation or a dry powder formulation.

According to some embodiments, the pharmaceutical composition comprises a liquid spray formulation of the API. According to some embodiments, the pharmaceutical composition is delivered as a liquid spray comprising a droplet size distribution comprising droplets containing the API.

According to some embodiments, the pharmaceutical composition comprises a dry powder formulation. According to some embodiments, the dry powder formulation when aerosolized comprises a cloud of very fine particles comprising the API.

According to some embodiments, the formulation is formulated with excipients. According to some embodiments, the excipients maximize the solubility of the API. According to some embodiments, the excipients enhance adhesion of the API to the nasal mucosa in the olfactory region. According to some embodiments the enhanced adhesion enhances absorption of the API in the nasal mucosa.

According to some embodiments, nasal aerosols comprise the formulation, a container, a valve, an actuator, a dust cap, associated accessories and protective packaging, which together constitute the drug product. For administration by insufflation, for example, the pharmaceutical composition according to the described invention can be conveniently delivered in the form of an aerosol spray presentation from pressurized packs with the use of a suitable propellant, e.g., dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges of, e.g., gelatin for use in an insufflator can be formulated containing a powder mix of the active compound and a suitable powder base such as lactose or starch.

According to some embodiments, the formulation is filled in unit dose devices. According to some devices, the formulation is filled in multi-dose devices. According to some embodiments, exemplary devices include a spray pump and a metered dose inhaler. According to some embodiments, the liquid formulation is filled in a spray pump, an exemplary device for delivering the intranasal formulation. According to some embodiments, the dry powder formulation is filled in a dry powder device comprising a formulation of the active agent and a container closure system.

For example, a typical nasal spray formulation consists of the active agent suspended or dissolved in an aqueous medium, which is filled into a bottle with a metered spray pump. Pump actuation by the subject delivers drug-laden droplets into the nasal cavity. The role of the pump, which is an integral part of the whole assembly, is to atomize and deliver an accurate drug dose.

Most nasal spray pumps produce droplets in the range of from about 20 $\mu$ to about 120 $\mu$m, inclusive, i.e., about 20 $\mu$m, about 25 $\mu$m, about 30 $\mu$m, about 35 $\mu$m, about 40 $\mu$m, about 45 $\mu$m, about 50 $\mu$m, 55 $\mu$m, about 60 $\mu$m, about 65 $\mu$m, about 70 $\mu$m, about 75 $\mu$m, about 80 $\mu$m, about 85 $\mu$m, about 90 $\mu$m about 95 $\mu$m, about 100 $\mu$m, about 105 $\mu$m, about 110 $\mu$m, about 115 $\mu$m or about 120 $\mu$m containing a therapeutic amount of an API [Yu, G. et al. Fluid flow and particle diffusion in the human upper respiratory system. Aerosol Science and Technol. (1998) 28: 146-58], so that the droplet size permits droplets deposit in the olfactory region of the nasal cavity for delivery of the API to the brain. If the droplet size is too fine (<10 $\mu$m) there is a possibility that the droplets will pass through the nasal passages and deposit in the lungs. If the droplet size is too large, the spray may be trapped in the nostrils. Laser diffraction techniques can measure the size of droplets by measuring the intensity of light scattered by particles as a function of angle to determine the droplet size for a given device and formulation.

The properties of the emitted spray plume are considered important in assessing performance of the pump. According to some embodiments, pump performance is characterized by spray pattern and plume geometry of the plume emitted by the delivery device. During the very early life of an aqueous nasal spray plume, the formulation may exit the actuator orifice as a narrow stream that subsequently forms a relatively stable, fully developed, conical plume prior to separating from the orifice. The term "plume geometry" describes a side view of the aerosol cloud parallel to the axis of the plume. According to some embodiments, measurement of plume geometry comprises images taken from the side of the plume, perpendicular to the laser light sheet, which is aligned with the expected central plume axis. According to some embodiments, measurement of plume geometry is by flash illumination of the plume with photographic image capture. According to some embodiments, plume geometry is characterized by the spray angle and plume width.

According to some embodiments, the pharmaceutical composition comprises a formulation containing a distribution of particles containing the API According to some embodiments, the majority of the distribution of particles ranges in diameter from about 25 $\mu$m to about-40 $\mu$m, inclusive, i.e., about 25 $\mu$m, about 26 $\mu$m, about 27 $\mu$m about 28 $\mu$m, about 29 $\mu$m, about 30 $\mu$m, about 31 $\mu$m, about 32 $\mu$m, about 333 about 34 $\mu$m, about 35 $\mu$m, about 36 $\mu$m, about 37 $\mu$m, about 38 $\mu$m, about 39 $\mu$m or about 40 $\mu$m. According to some embodiments, the distribution of particles is depleted of smaller particles that would otherwise go into the lung.

According to some embodiments, a viscosity enhancer/mucoadhesive polymer, e.g., microcrystalline cellulose, sodium carboxymethylcellulose, hydroxypropyl methyl cellulose or a combination thereof, may be added to the formulation, as 0.1%, 0.2%, 0.3%, 1% or 2% of the formulation by weight or by volume to increase absorption of the API in the nasal mucosa of the olfactory region of the nasal cavity. According to some embodiments, the viscosity of each nasal spray formulation will be measured. According to some embodiments, the spray pattern and plume geometry of each nasal spray formulation will be measured. According to some embodiments, the droplet-size distribution of each nasal spray will be determined to determine a median (Dv50), a 10th (Dv10) and a 90th (Dv90) percentile of the cumulative undersize distribution.

According to some embodiments, for intranasal delivery, a maximum of about 25 mg/dose is administered as frequently as tolerated, e.g. every 2 minutes, 5 minutes, 10 minutes, 15 minutes, 20 minutes, 30 minutes, 40 minutes, 50 minutes, or 60 minutes, for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 3;8 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours ,61 hours, 62 hours, 63 hr, 64 hr, 65 hr, 66 hr, 67 hr, 68 hr, 69 hr, 70 hr, 71 hr, 72 hours etc. to achieve a minimum daily dose ranging from about 85 mg/day (e.g., for a child weighing about 45 lb.) to about 800 mg/day (e.g., for an adult) inclusive, i.e., at least about 85 mg/day; about 95 mg/day; about 105 mg/day, about 115 mg/day; about 125 mg/day; about 135 mg/day; about 145 mg/day; about 155 mg/day; about 165 mg/day; about 175 mg/day; about 185 mg/day; about 195 mg/day; about 205 mg/day, about 215 mg/day; about 225 mg/day; about 235 mg/day; about 245 mg/day; about 255 mg/day; about 265 mg/day; about 275 mg/day; about 285 mg/day; about 295 mg/day; about 305 mg/day, about 315 mg/day; about 325 mg/day; about 335 mg/day; about 345 mg/day; about 355 mg/day; about 365 mg/day; about 375 mg/day; about 385 mg/day; about 395 mg/day; about 405 mg/day, about 415 mg/day; about 425 mg/day; about 435 mg/day; about 445 mg/day; about 455 mg/day; about 465 mg/day; about 475 mg/day; about 485 mg/day; about 495 mg/day; about 505 mg/day, about 515 mg/day; about 525 mg/day; about 535 mg/day; about 545 mg/day; about 555 mg/day; about 565 mg/day; about 575 mg/day; about 585 mg/day; about 595 mg/day; about 605 mg/day, about 615 mg/day; about 625 mg/day; about 635 mg/day; about 645 mg/day; about 655 mg/day; about 665 mg/day; about 675 mg/day; about 685 mg/day; about 695 mg/day; about 705 mg/day, about 715 mg/day; about 725 mg/day; about 735 mg/day; about 745 mg/day; about 755 mg/day; about 765 mg/day; about 775 mg/day; about 785 mg/day; about 795 mg/day; or about 800 mg/day.

According to some embodiments, and based on human equivalent dose (HED) from the mouse studies reported below in para. [00635], the minimum daily dose for a 60 kg human (132 lb) is 250 mg/day. According to some embodiments, the minimum daily dose for a 90 kg human (200 lb) is 730 mg/day.

According to some embodiments, a dose time curve is determined so that a maximum 25 mg/dose delivered intranasally at a given frequency as tolerated, e.g., every 2 minutes, 5 minutes, 10 minutes, 15 minutes, 20 minutes, 30 minutes , 40 minutes, 50 minutes, or 60 minutes, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. achieves the desired effective amount.

According to some embodiments, intranasal delivery within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before participating in an event where an acquired brain injury is a known risk and for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after participating in an event where an acquired brain injury is a known risk is neuroprotective. According to some embodiments, the event is a military operation. According to some embodiments, the event is a sports event. According to some embodiments, the sports event is, for example, hockey, football, soccer, baseball, polo, rugby, horseback riding, autoracing, motorcycling, skiing, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. Other applications include emergency medicine, for example use by EMTs or emergency departments in the context of such emergencies as domestic violence, falls, car crashes, etc.

According to some embodiments, integration of the D-beta-hydroxybutyrate into polymeric carriers can achieve controlled release, including delayed release, and sustained release.

According to some embodiments, the D-beta hydroxybutyrate binds HCA2.

According to some embodiments, the composition when delivered intranasally may elevate brain D-beta hydroxybutyrate levels, e.g., to about 0.1-3 mmol/L, or about 0.1 - 1 mmol/L for at least 6 hr to at least 48 hr, inclusive, i.e., at least 6 hr, at least 7 hr, at least 8 hr, at last 9 hr, at least 10 hr, at least 11 hr, at least 12 hr, at least 13 hr, at least 14 hr, at least 15 hr, at least 16 hr, at least 17 hr, at least 18 hr, at least 19 hr, at least 20 hr, at least 21 hr, at least 22 hr, at least 23 hr, at least 24 hr, at least 25 hr, at least 26 hr, at least 27 hr, at least 28 hr ,at least 29 hr, at least 30 hr, at least 31 hr, at least 32 hr, at least 33 hr, at least 34 hr, at least 35 hr, at least 36 hr, at least 37 hr, at least 38 hr, at least 39 hr, at least 40 hr, at least 41 hr, at least 42 hr, at least 43 hr, at least 44 hr, at least 45 hr, at least 46 hr, at least 47 hr, or at least 48 hr.

**Methods.**

(1) **Pre-injury**

According to another aspect, the present disclosure provides a method for reducing risk of brain damage due to an acquired brain injury for a subject susceptible to, or otherwise at risk of, the acquired brain injury comprising administering a pharmaceutical composition comprising a pharmaceutically acceptable carrier and a formulation comprising a therapeutic amount of an API, wherein the API is a synthetic D-beta-hydroxybutyrate,

wherein the administering is intranasally (IN),

wherein the administering comprises targeted delivery to the olfactory region of the nasal cavity of the subject;

wherein the targeted delivery achieves therapeutically effective amounts of the API in the brain, and

wherein an effective amount of the pharmaceutical composition (i) eliminates or reduces the risk of the acquired brain injury or (ii) lessens the severity of the acquired brain injury, or (iii) delays the onset of the acquired brain injury; or (iv) a combination thereof.

According to some embodiments, the acquired brain injury is a traumatic brain injury. According to some embodiments, the traumatic brain injury comprises a concussion. According to some embodiments, the subject's risk of the acquired brain injury is increased by participating in an event where an acquired brain injury is a known risk. According to some embodiments, the event is a military operation. According to some embodiments, the event is a sports event. According to some embodiments, the sports event is, for example, hockey, football, soccer, baseball, polo, rugby, horseback riding, autoracing, motorcycling, skiing, gymnastics, mountain climbing, Basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. or (iv) a combination thereof. Other applications include emergency medicine, for example use by EMTs or emergency departments in the context of such emergencies as domestic violence, falls, car crashes, etc.

According to some embodiments, the administering within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before the event where an acquired brain injury is a known risk is neuroprotective.

According to some embodiments, the pharmaceutical composition comprises the active formulated as a solution, a suspension or a dispersion. According to some embodiments, the API is formulated as a solution. According to some embodiments, the API is formulated as a suspension. According to some embodiments, the API is formulated as a suspension, wherein the suspension comprises a particle size distribution of a population of particles comprising the API n. According to some embodiments, the API is formulated as a dispersion. According to some embodiments, the API is formulated as a dispersion, wherein the dispersion comprises a particle size distribution of a population of particles comprising the API.

According to some embodiments, the pharmaceutical composition comprises a liquid spray formulation or a dry powder formulation.

According to some embodiments, the pharmaceutical composition comprises a liquid spray formulation of the API. According to some embodiments, the pharmaceutical composition is delivered as a liquid spray comprising a droplet size distribution comprising droplets containing the API.

According to some embodiments, the pharmaceutical composition comprises a dry powder formulation. According to some embodiments, the aerosolized dry powder formulation comprises a cloud of very fine particles comprising the API.

According to some embodiments, the formulation is formulated with excipients. According to some embodiments, the excipients maximize the solubility of the API. According to some embodiments, the excipients enhance adhesion of the API to the nasal mucosa in the olfactory. According to some embodiments the enhanced adhesion enhances absorption of the API in the nasal mucosa.

According to some embodiments, nasal aerosols comprise the formulation, a container, a valve, an actuator, a dust cap, associated accessories and protective packaging, which together constitute the drug product. For administration by insufflation, for example, the pharmaceutical composition according to the described invention can be conveniently delivered in the form of an aerosol spray presentation from pressurized packs with the use of a suitable propellant, e.g., dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges of, e.g., gelatin for use in an insufflator can be formulated containing a powder mix of the active compound and a suitable powder base such as lactose or starch.

According to some embodiments, the formulation is filled in unit dose devices. According to some devices, the formulation is filled in multi-dose devices. According to some embodiments, exemplary devices include a spray pump and a metered dose inhaler. According to some embodiments, the liquid formulation is filled in a spray pump, an exemplary device for delivering the intranasal formulation. According to some embodiments, the dry powder formulation is filled in a dry powder device comprising a formulation of the active agent and a container closure system.

For example, a typical nasal spray formulation consists of the active agent suspended or dissolved in an aqueous medium, which is filled into a bottle with a metered spray pump. Pump actuation by the subject delivers drug-laden droplets into the nasal cavity. The role of the pump, which is an integral part of the whole assembly, is to atomize and deliver an accurate drug dose.

Most nasal spray pumps produce droplets in the range of from about 20 μ to about 120 μm, i.e., about 20 μm, about 25 μm, about 30 μm, about 35 μm, about 40 μm, about 45 μm, about 50 μm, 55 μm, about 60 μm, about 65 μm, about 70 μm, about 75 μm, about 80 μm, about 85 μm, about 90 μm about 95 μm, about 100 μm, about 105 μm, about 110 μm, about 115 μm or about 120 μm containing a therapeutic amount of the API [Yu, G. et al. Fluid flow and particle diffusion in the human upper respiratory system. Aerosol Science and Technol. (1998) 28: 146-58], so that the droplet size permits droplets deposit in the olfactory region of the nasal cavity for delivery of the API to the brain. If the droplet size is too fine (<10 μm) there is a possibility that the droplets will pass through the nasal passages and deposit in the lungs. If the droplet size is too large, the spray may be trapped in the nostrils. Laser diffraction techniques can measure the size of droplets by measuring the intensity of light scattered by particles as a function of angle to determine the droplet size for a given device and formulation.

The properties of the emitted spray plume are considered important in assessing performance of the pump. According to some embodiments, pump performance is characterized by spray pattern and plume geometry of the plume emitted by the delivery device. During the very early life of an aqueous nasal spray plume, the formulation may exit the actuator orifice as a narrow stream that subsequently forms a relatively stable, fully developed, conical plume prior to separating from the orifice. The term "plume geometry" describes a side view of the aerosol cloud parallel to the axis of the plume. According to some embodiments, measurement of plume geometry comprises images taken from the side of the plume, perpendicular to the laser light sheet, which is aligned with the expected central plume axis. According to some embodiments, measurement of plume geometry is by flash illumination of the plume with photographic image capture. According to some embodiments, plume geometry is characterized by the spray angle and plume width.

According to some embodiments, the pharmaceutical composition comprises a formulation containing a distribution of particles containing the API According to some embodiments, the majority of the distribution of particles ranges in diameter from about 25 μm to about-40 μm, i.e., about 25 μm , about 26 μm, about 27 μm about 28 μm, about 29 μm, about 30 μm, about 31 μm, about 32 μm, about 333 about 34 μm, about 35 μm, about 36 μm, about 37 μm, about 38 μm, about 39 μm or about 40 μm. According to some embodiments, the distribution of particles is depleted of smaller particles that would otherwise go into the lung.

According to some embodiments, a viscosity enhancer/mucoadhesive polymer, e.g., microcrystalline cellulose, sodium carboxymethylcellulose, or hydroxypropyl methyl cellulose, or a combination thereof, may be added to the formulation, as 0.1%, 0.2%, 0.3%, 1% or 2% of the formulation by weight or by volume to increase absorption of the API in the nasal mucosa of the olfactory region of the nasal cavity. According to some embodiments, the viscosity of each nasal spray formulation will be measured. According to some embodiments, the spray pattern and plume geometry of each nasal spray formulation will be measured. According to some embodiments, the droplet-size distribution of each nasal spray will be determined to determine a median (Dv50), a 10th (Dv10) and a 90th (Dv90) percentile of the cumulative undersize distribution.

According to some embodiments, for intranasal delivery, a maximum of about 25 mg/dose is administered as frequently as tolerated, e.g. every 2 minutes, 5 minutes, 10 minutes, 15 minutes, 20 minutes, 30 minutes, 40 minutes, 50 minutes, or 60 minutes, for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 3;8 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours, 61 hours, 62 hours, 63 hr, 64 hr, 65 hr, 66 hr, 67 hr, 68 hr, 69 hr, 70 hr, 71 hr, 72 hours etc. to achieve a minimum daily dose ranging from about 85 mg/day (e.g., for a child weighing about 45 lbs.) to about 800 mg/day (e.g., for an adult) inclusive, i.e., at least about 85 mg/day; about 95 mg/day; about 105 mg/day, about 115 mg/day; about 125 mg/day; about 135 mg/day; about 145 mg/day; about 155 mg/day; about 165 mg/day; about 175 mg/day; about 185 mg/day; about 195 mg/day; about 205 mg/day, about 215 mg/day; about 225 mg/day; about 235 mg/day; about 245 mg/day; about 255 mg/day; about 265 mg/day; about 275 mg/day; about 285 mg/day; about 295 mg/day; about 305 mg/day, about 315 mg/day; about 325 mg/day; about 335 mg/day; about 345 mg/day; about 355 mg/day; about 365 mg/day; about 375 mg/day; about 385 mg/day; about 395 mg/day; about 405 mg/day, about 415 mg/day; about 425 mg/day; about 435 mg/day; about 445 mg/day; about 455 mg/day; about 465 mg/day; about 475 mg/day; about 485 mg/day; about 495 mg/day; about 505 mg/day, about 515 mg/day; about 525 mg/day; about 535 mg/day; about 545 mg/day; about 555 mg/day; about 565 mg/day; about 575 mg/day; about 585 mg/day; about 595 mg/day; about 605 mg/day, about 615 mg/day; about 625 mg/day; about 635 mg/day; about 645 mg/day; about 655 mg/day; about 665 mg/day; about 675 mg/day; about 685 mg/day; about 695 mg/day; about 705 mg/day; about 715 mg/day; about 725 mg/day; about 735 mg/day; about 745 mg/day; about 755 mg/day; about 765 mg/day; about 775 mg/day; about 785 mg/day; about 795 mg/day; or about 800 mg/day.

According to some embodiments, and based on HED from the mouse studies reported below in para. [00635], the minimum daily dose for a 60 kg human (132 lb) is 250 mg/day. According to some embodiments, the minimum daily dose for a 90 kg human (200 lb) is 730 mg/day.

According to some embodiments, a dose time curve is determined so that a maximum 25 mg/dose delivered intranasally at a given frequency as tolerated, e.g., every 2 minutes, 5 minutes, 10 minutes, 15 minutes, 20 minutes, 30 minutes , 40 minutes, 50 minutes, or 60 minutes, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. achieves the desired effective amount.

According to some embodiments, intranasal delivery within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before an event where an acquired brain injury is a known risk is neuroprotective.

According to some embodiments, the event is a military operation. According to some embodiments, the event is a sports event. According to some embodiments, the sports event is, for example, hockey, football, soccer, baseball, polo, rugby, horseback riding, autoracing, motorcycling, skiing, gymnastics, mountain climbing, Basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. Other applications include emergency medicine, for example use by EMTs or emergency departments in the context of such emergencies as domestic violence, falls, car crashes, etc.

According to some embodiments, integration of the D-beta-hydroxybutyrate into polymeric carriers can achieve controlled release, including delayed release, and sustained release.

According to some embodiments, the D-beta hydroxybutyrate binds HCA2.

According to some embodiments, the composition when delivered intranasally may elevate brain D-beta hydroxybutyrate levels, e.g., to about 0.1-3 mmol/L, or about 0.1 - 1 mmol/L for at least 6 hr to at least 48 hr, inclusive, i.e., at least 6 hr, at least 7 hr, at least 8 hr, at last 9 hr, at least 10 hr, at least 11 hr, at least 12 hr, at least 13 hr, at least 14 hr, at least 15 hr, at least 16 hr, at least 17 hr, at least 18 hr, at least 19 hr, at least 20 hr, at least 21 hr, at least 22 hr, at least 23 hr, at least 24 hr, at least 25 hr, at least 26 hr, at least 27 hr, at least 28 hr ,at least 29 hr, at least 30 hr, at least 31 hr, at least 32 hr, at least 33 hr, at least 34 hr, at least 35 hr, at least 36 hr, at least 37 hr, at least 38 hr, at least 39 hr, at least 40 hr, at least 41 hr, at least 42 hr, at least 43 hr, at least 44 hr, at least 45 hr, at least 46 hr, at least 47 hr, or at least 48 hr.

(2) **Post-injury**

According to another aspect, the present disclosure provides a method for treating symptoms of a brain injury of a subject comprising neuroinflammation comprising administering a pharmaceutical composition comprising a pharmaceutically acceptable carrier and a formulation comprising a therapeutic amount of an API, wherein the API is a synthetic D-beta-hydroxybutyrate,

wherein the administering is intranasally (IN);

wherein the administering comprises targeted delivery to the olfactory region of the nasal cavity of the subject,

wherein the targeted delivery achieves a therapeutically effective amount of the API in the brain,

wherein the effective amount of the pharmaceutical composition accomplishes one or more therapeutic effects, including:

(a) reducing the severity of the acquired brain injury;

(b) limiting development of symptoms characteristic of the acquired brain injury being treated;

(c) limiting worsening of symptoms characteristic of the acquired brain injury being treated;

(d) limiting recurrence of the acquired brain injury in subjects that have previously had the acquired brain injury; or

(e) limiting recurrence of symptoms in subjects that were previously asymptomatic for the acquired brain injury.

According to some embodiments, the acquired brain injury is a traumatic brain injury. According to some embodiments, the traumatic brain injury comprises a concussion. According to some embodiments, the subject's risk of the acquired brain injury is increased by the subject's participation in an event where an acquired brain injury is a known risk. According to some embodiments, the event is a military operation. According to some embodiments, the event is a sports event. According to some embodiments, the sports event is, for example, hockey, football, soccer, baseball, polo, rugby, horseback riding, autoracing, motorcycling, skiing, mountain climbing, gymnastics, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. Other applications include emergency medicine, for example use by EMTs or emergency departments in the context of such emergencies as domestic violence, falls, car crashes, etc.

According to some embodiments, the administering is as frequently as tolerated, e.g. every 2 minutes, 5 minutes, 10 minutes, 15 minutes, 20 minutes, 30 minutes , 40 minutes, 50 minutes, or 60 minutes, for 1 hour, for 2 hours, for 3 hours, for 4 hours, for 5 hours, for 6 hours, for 7 hours, for 8 hours, for 9 hours, for 10 hours, for 11 hours, for 12 hours, for 13 hours, for 14 hours, for 15 hours, for 16 hours, for 17 hours, for 18 hours, for 19 hours, for 20 hours, for 21 hours, for 22 hours, for 23 hours, for 24 hours, within at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after the event.

According to some embodiments, the pharmaceutical composition comprises a liquid spray formulation or a dry powder formulation. According to some embodiments, the pharmaceutical composition comprises a liquid spray formulation. According to some embodiments, the pharmaceutical composition comprises a dry powder formulation. According to some embodiments, the formulation is formulated with excipients. According to some embodiments, the excipients maximize the solubility of the API. According to some embodiments, the excipients enhance adhesion of the API to the nasal mucosa. According to some embodiments the enhanced adhesion enhances absorption of the API in the nasal mucosa.

According to some embodiments, the pharmaceutical composition comprises the active formulated as a solution, a suspension or a dispersion. According to some embodiments, the API is formulated as a solution. According to some embodiments, the API is formulated as a suspension. According to some embodiments, the API is formulated as a suspension, wherein the suspension comprises a particle size distribution of a population of particles comprising the API n. According to some embodiments, the API is formulated as a dispersion. According to some embodiments, the API is formulated as a dispersion, wherein the dispersion comprises a particle size distribution of a population of particles comprising the API.

According to some embodiments, the pharmaceutical composition comprises a liquid spray formulation or a dry powder formulation.

According to some embodiments, the pharmaceutical composition comprises a liquid spray formulation of the API. According to some embodiments, the pharmaceutical composition is delivered as a liquid spray comprising a droplet size distribution comprising droplets containing the API.

According to some embodiments, the pharmaceutical composition comprises a dry powder formulation. According to some embodiments, the aerosolized dry powder formulation comprises a cloud of very fine particles comprising the API.

According to some embodiments, the formulation is formulated with excipients. According to some embodiments, the excipients maximize the solubility of the API. According to some embodiments, the excipients enhance adhesion of the API to the nasal mucosa in the olfactory. According to some embodiments the enhanced adhesion enhances absorption of the API in the nasal mucosa.

According to some embodiments, nasal aerosols comprise the formulation, a container, a valve, an actuator, a dust cap, associated accessories and protective packaging, which together constitute the drug product. For administration by insufflation, for example, the pharmaceutical composition according to the described invention can be conveniently delivered in the form of an aerosol spray presentation from pressurized packs with the use of a suitable propellant, e.g., dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges of, e.g., gelatin for use in an insufflator can be formulated containing a powder mix of the active compound and a suitable powder base such as lactose or starch.

According to some embodiments, the formulation is filled in unit dose devices. According to some embodiments, the formulation is filled in multi-dose devices. According to some embodiments, exemplary devices include a spray pump and a metered dose inhaler. According to some embodiments, the liquid formulation is filled in a spray pump, an exemplary device for delivering the intranasal formulation. According to some embodiments, the dry powder formulation is filled in a dry powder device comprising a formulation of the active agent and a container closure system.

For example, a typical nasal spray formulation consists of the active agent suspended or dissolved in an aqueous medium, which is filled into a bottle with a metered spray pump. Pump actuation by the subject delivers drug-laden droplets into the nasal cavity. The role of the pump, which is an integral part of the whole assembly, is to atomize and deliver an accurate drug dose.

Most nasal spray pumps produce droplets in the range of from about 20 $\mu$ to about 120 $\mu$m, i.e., about 20 $\mu$m, about 25 $\mu$m, about 30 $\mu$m, about 35 $\mu$m, about 40 $\mu$m, about 45 $\mu$m, about 50 $\mu$m, 55 $\mu$m, about 60 $\mu$m, about 65 $\mu$m, about 70 $\mu$m, about 75 $\mu$m, about 80 $\mu$m, about 85 $\mu$m, about 90 $\mu$m about 95 $\mu$m, about 100 $\mu$m, about 105 $\mu$m, about 110 $\mu$m, about 115 $\mu$m or about 120 $\mu$m containing a therapeutic amount of the API [Yu, G. et al. Fluid flow and particle diffusion in the human upper respiratory system. Aerosol Science and Technol. (1998) 28: 146-58], so that the droplet size permits droplets deposit in the olfactory region of the nasal cavity for delivery of the API to the brain. If the droplet size is too fine (<10 $\mu$m) there is a possibility that the droplets will pass through the nasal passages and deposit in the lungs. If the droplet size is too large, the spray may be trapped in the nostrils. Laser diffraction techniques can measure the size of droplets by measuring the intensity of light scattered by particles as a function of angle to determine the droplet size for a given device and formulation.

The properties of the emitted spray plume are considered important in assessing performance of the pump. According to some embodiments, pump performance is characterized by spray pattern and plume geometry of the plume emitted by the delivery device. During the very early life of an aqueous nasal spray plume, the formulation may exit the actuator orifice as a narrow stream that subsequently forms a relatively stable, fully developed, conical plume prior to separating from the orifice. The term "plume geometry" describes a side view of the aerosol cloud parallel to the axis of the plume. According to some embodiments, measurement of plume geometry comprises images taken from the side of the plume, perpendicular to the laser light sheet, which is aligned with the expected central plume axis. According to some embodiments, measurement of plume geometry is by flash illumination of the plume with photographic image capture. According to some embodiments, plume geometry is characterized by the spray angle and plume width.

According to some embodiments, the pharmaceutical composition comprises a formulation containing a distribution of particles containing the API According to some embodiments, the majority of the distribution of particles ranges in diameter from about 25 µm to about-40 µm, i.e., about 25 µm , about 26 µm, about 27 µm about 28 µm, about 29 µm, about 30 µm, about 31 µm, about 32 µm, about 333 about 34 µm, about 35 µm, about 36 µm, about 37 µm, about 38 µm, about 39 µm or about 40 µm. According to some embodiments, the distribution of particles is depleted of smaller particles that would otherwise go into the lung.

According to some embodiments, a viscosity enhancer/mucoadhesive polymer, e.g., microcrystalline cellulose, sodium carboxymethylcellulose, hydroxypropyl methyl cellulose, or a combination thereof, may be added to the formulation, as 0.1%, 0.2%, 0.3%, 1% or 2% of the formulation by weight or by volume to increase absorption of the API in the nasal mucosa of the olfactory region of the nasal cavity. According to some embodiments, the viscosity of each nasal spray formulation will be measured. According to some embodiments, the spray pattern and plume geometry of each nasal spray formulation will be measured. According to some embodiments, the droplet-size distribution of each nasal spray will be determined to determine a median (Dv50), a 10th (Dv10) and a 90th (Dv90) percentile of the cumulative undersize distribution.

According to some embodiments, for intranasal delivery, a maximum of about 25 mg/dose is administered as frequently as tolerated, e.g. every 2 minutes, 5 minutes, 10 minutes, 15 minutes, 20 minutes, 30 minutes , 40 minutes, 50 minutes, or 60 minutes for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 3;8 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours ,61 hours, 62 hours, 63 hr, 64 hr, 65 hr, 66 hr, 67 hr, 68 hr, 69 hr, 70 hr, 71 hr, 72 hours, or etc. after the acquired brain injury to achieve a minimum daily dose ranging from about 85 mg/day (e.g., for a child weighing about 45 lbs.) to about 800 mg/day (e.g., for an adult) inclusive, i.e., at least about 85 mg/day; about 95 mg/day; about 105 mg/day, about 115 mg/day; about 125 mg/day; about 135 mg/day; about 145 mg/day; about 155 mg/day; about 165 mg/day; about 175 mg/day; about 185 mg/day; about 195 mg/day; about 205 mg/day, about 215 mg/day; about 225 mg/day; about 235 mg/day; about 245 mg/day; about 255 mg/day; about 265 mg/day; about 275 mg/day; about 285 mg/day; about 295 mg/day; about 305 mg/day, about 315 mg/day; about 325 mg/day; about 335 mg/day; about 345 mg/day; about 355 mg/day; about 365 mg/day; about 375 mg/day; about 385 mg/day; about 395 mg/day, about 405 mg/day, about 415 mg/day; about 425 mg/day; about 435 mg/day; about 445 mg/day; about 455 mg/day; about 465 mg/day; about 475 mg/day; about 485 mg/day; about 495 mg/day; about 505 mg/day, about 515 mg/day; about 525 mg/day; about 535 mg/day; about 545 mg/day; about 555 mg/day; about 565 mg/day; about 575 mg/day; about 585 mg/day; about 595 mg/day; about 605 mg/day, about 615 mg/day; about 625 mg/day; about 635 mg/day; about 645 mg/day; about 655 mg/day; about 665 mg/day; about 675 mg/day; about 685 mg/day; about 695 mg/day; about 705 mg/day, about 715 mg/day; about 725 mg/day; about 735 mg/day; about 745 mg/day; about 755 mg/day; about 765 mg/day; about 775 mg/day; about 785 mg/day; about 795 mg/day; or about 800 mg/day.

According to some embodiments, and based on HED from the mouse studies reported below in para. [00635], the minimum daily dose for a 60 kg human (132 lb) is 250 mg/day. According to some embodiments, the minimum daily dose for a 90 kg human (200 lb) is 730 mg/day.

According to some embodiments, a dose time curve is determined so that a maximum 25 mg/dose delivered intranasally at a given frequency as tolerated, e.g., every 2 minutes, 5 minutes, 10 minutes, 15 minutes, 20 minutes, 30 minutes , 40 minutes, 50 minutes, or 60 minutes, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, or etc. achieves the desired effective amount.

According to some embodiments, intranasal delivery within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, or etc. of the acquired brain injury is neuroprotective.

According to some embodiments, integration of the D-beta-hydroxybutyrate into polymeric carriers can achieve controlled release, including delayed release, and sustained release.

According to some embodiments, the D-beta hydroxybutyrate binds HCA2.

According to some embodiments, the composition when delivered intranasally may elevate brain D-beta hydroxybutyrate levels, e.g., to about 0.1-3 mmol/L, or about 0.1 - 1 mmol/L for at least 6 hr to at least 48 hr, inclusive, i.e., at least 6 hr, at least 7 hr, at least 8 hr, at last 9 hr, at least 10 hr, at least 11 hr, at least 12 hr, at least 13 hr, at least 14 hr, at least 15 hr, at least 16 hr, at least 17 hr, at least 18 hr, at least 19 hr, at least 20 hr, at least 21 hr, at least 22 hr, at least 23 hr, at least 24 hr, at least 25 hr, at least 26 hr, at least 27 hr, at least 28 hr ,at least 29 hr, at least 30 hr, at least 31 hr, at least 32 hr, at least 33 hr, at least 34 hr, at least 35 hr, at least 36 hr, at least 37 hr, at least 38 hr, at least 39 hr, at least 40 hr, at least 41 hr, at least 42 hr, at least 43 hr, at least 44 hr, at least 45 hr, at least 46 hr, at least 47 hr, or at least 48 hr.

## (3) Pre and Post-injury

According to another aspect, the present disclosure provides a method for reducing risk of brain damage due to an acquired brain injury for a subject susceptible to, or otherwise at risk of, the acquired brain injury and for treating symptoms of the acquired brain injury once the brain injury has been acquired by the subject comprising

(a) administering a pharmaceutical composition comprising a pharmaceutically acceptable carrier and a formulation comprising a therapeutic amount of an API, wherein the API is a synthetic D-beta-hydroxybutyrate before an event wherein the subject's risk of acquiring and of experiencing a traumatic brain injury is increased by the subject's participation in an event where an acquired brain injury is a known risk.,

wherein the administering is intranasally (IN),

wherein the administering comprises targeted delivery to the olfactory region of the nasal cavity of the subject;

wherein the targeted delivery achieves an effective amount of the API in the brain, and

wherein an effective amount of the pharmaceutical composition (i) eliminates or reduces the risk of the acquired brain injury or (ii) lessens the severity of the acquired brain injury, or (iii) delays the onset of the acquired brain injury; or (iv) a combination thereof; and

(b) continuing the administering intranasally (IN) after the event;

wherein the effective amount of the pharmaceutical composition accomplishes one or more of the following therapeutic effects:

(i) reducing the severity of the acquired brain injury;

(ii) limiting development of symptoms characteristic of the acquired brain injury being treated;

(iii) limiting worsening of symptoms characteristic of the acquired brain injury being treated;

(iv) limiting recurrence of the acquired brain injury in subjects that have previously had the acquired brain injury; or

(v) limiting recurrence of symptoms in subjects that were previously asymptomatic for the acquired brain injury.

According to some embodiments, the acquired brain injury is a traumatic brain injury. According to some embodiments, the traumatic brain injury comprises a concussion. According to some embodiments, the event is a military operation. According to some embodiments, the event is a sports event. According to some embodiments, the sports event is, for example, hockey, football, soccer, baseball, polo, rugby, horseback riding, autoracing, motorcycling, skiing, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. Other applications include emergency medicine, for example use by EMTs or emergency departments in the context of such emergencies as domestic violence, falls, car crashes, etc.

According to some embodiments, the administering is within 1 hour of the event, i.e., within 10 minutes; within 20 minutes; within 30 minutes; within 40 minutes, within 50 minutes, within 60 minutes; or within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before the event where an acquired brain injury is a known risk and for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after the event.

According to some embodiments, the pharmaceutical composition comprises a liquid spray formulation or a dry powder formulation. According to some embodiments, the pharmaceutical composition comprises a liquid spray formulation. According to some embodiments, the pharmaceutical composition comprises a dry powder formulation. According to some embodiments, the formulation is formulated with excipients. According to some embodiments, the excipients maximize the solubility of the API. According to some embodiments, the excipients enhance adhesion of the API to the nasal mucosa. According to some embodiments the enhanced adhesion enhances absorption of the API in the nasal mucosa.

According to some embodiments, the pharmaceutical composition comprises the active formulated as a solution, a suspension or a dispersion. According to some embodiments, the API is formulated as a solution. According to some embodiments, the API is formulated as a suspension. According to some embodiments, the API is formulated as a suspension, wherein the suspension comprises a particle size distribution of a population of particles comprising the API n. According to some embodiments, the API is formulated as a dispersion. According to some embodiments, the API is formulated as a dispersion, wherein the dispersion comprises a particle size distribution of a population of particles comprising the API.

According to some embodiments, the pharmaceutical composition comprises a liquid spray formulation or a dry powder formulation.

According to some embodiments, the pharmaceutical composition comprises a liquid spray formulation of the API. According to some embodiments, the pharmaceutical composition is delivered as a liquid spray comprising a droplet size distribution comprising droplets containing the API.

According to some embodiments, the pharmaceutical composition comprises a dry powder formulation. According to some embodiments, the aerosolized dry powder formulation comprises a cloud of very fine particles comprising the API.

According to some embodiments, the formulation is formulated with excipients. According to some embodiments, the excipients maximize the solubility of the API. According to some embodiments, the excipients enhance adhesion of the API to the nasal mucosa in the olfactory. According to some embodiments the enhanced adhesion enhances absorption of the API in the nasal mucosa.

According to some embodiments, nasal aerosols comprise the formulation, a container, a valve, an actuator, a dust cap, associated accessories and protective packaging, which together constitute the drug product. For administration by insufflation, for example, the pharmaceutical composition according to the described invention can be conveniently delivered in the form of an aerosol spray presentation from pressurized packs with the use of a suitable propellant, e.g., dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges of, e.g., gelatin for use in an insufflator can be formulated containing a powder mix of the active compound and a suitable powder base such as lactose or starch.

According to some embodiments, the formulation is filled in unit dose devices. According to some devices, the formulation is filled in multi-dose devices. According to some embodiments, exemplary devices include a spray pump and a metered dose inhaler. According to some embodiments, the liquid formulation is filled in a spray pump, an exemplary device for delivering the intranasal formulation. According to some embodiments, the dry powder formulation is filled in a dry powder device comprising a formulation of the active agent and a container closure system.

For example, a typical nasal spray formulation consists of the active agent suspended or dissolved in an aqueous medium, which is filled into a bottle with a metered spray pump. Pump actuation by the subject delivers drug-laden droplets into the nasal cavity. The role of the pump, which is an integral part of the whole assembly, is to atomize and deliver an accurate drug dose.

Most nasal spray pumps produce droplets in the range of from about 20 $\mu$ to about 120 $\mu$m, i.e., about 20 $\mu$m, about 25 $\mu$m, about 30 $\mu$m, about 35 $\mu$m, about 40 $\mu$m, about 45 $\mu$m, about 50 $\mu$m, 55 $\mu$m, about 60 $\mu$m, about 65 $\mu$m, about 70 $\mu$m, about 75 $\mu$m, about 80 $\mu$m, about 85 $\mu$m, about 90 $\mu$m about 95 $\mu$m, about 100 $\mu$m, about 105 $\mu$m, about 110 $\mu$m, about 115 $\mu$m or about 120 $\mu$m containing a therapeutic amount of the API [Yu, G. et al. Fluid flow and particle diffusion in the human upper respiratory system. Aerosol Science and Technol. (1998) 28: 146-58], so that the droplet size permits droplets deposit in the olfactory region of the nasal cavity for delivery of the API to the brain. If the droplet size is too fine (<10 $\mu$m) there is a possibility that the droplets will pass through the nasal passages and deposit in the lungs. If the droplet size is too large, the spray may be trapped in the nostrils. Laser diffraction techniques can measure the size of droplets by measuring the intensity of light scattered by particles as a function of angle to determine the droplet size for a given device and formulation.

The properties of the emitted spray plume are considered important in assessing performance of the pump. According to some embodiments, pump performance is characterized by spray pattern and plume geometry of the plume emitted by the delivery device. During the very early life of an aqueous nasal spray plume, the formulation may exit the actuator orifice as a narrow stream that subsequently forms a relatively stable, fully developed, conical plume prior to separating from the orifice. The term "plume geometry" describes a side view of the aerosol cloud parallel to the axis of the plume. According to some embodiments, measurement of plume geometry comprises images taken from the side of the plume, perpendicular to the laser light sheet, which is aligned with the expected central plume axis. According to some embodiments, measurement of plume geometry is by flash illumination of the plume with photographic image capture. According to some embodiments, plume geometry is characterized by the spray angle and plume width.

According to some embodiments, the pharmaceutical composition comprises a formulation containing a distribution of particles containing the API According to some embodiments, the majority of the distribution of particles ranges in diameter from about 25 $\mu$m to about-40 $\mu$m, inclusive, i.e., about 25 $\mu$m, about 26 $\mu$m, about 27 $\mu$m about 28 $\mu$m, about 29 $\mu$m, about 30 $\mu$m, about 31 $\mu$m, about 32 $\mu$m, about 333 about 34 $\mu$m, about 35 $\mu$m, about 36 $\mu$m, about 37 $\mu$m, about 38 $\mu$m, about 39 $\mu$m or about 40 $\mu$m. According to some embodiments, the distribution of particles is depleted of smaller particles that would otherwise go into the lung.

According to some embodiments, a viscosity enhancer/mucoadhesive polymer, e.g., microcrystalline cellulose, sodium carboxymethylcellulose, hydroxypropyl methyl cellulose, or a combination thereof, may be added to the formulation, as 0.1%, 0.2%, 0.3%, 1% or 2% of the formulation by weight or by volume to increase absorption of the API in the nasal mucosa of the olfactory region of the nasal cavity. According to some embodiments, the viscosity of each nasal spray formulation will be measured. According to some embodiments, the spray pattern and plume geometry of each nasal spray formulation will be measured. According to some embodiments, the droplet-size distribution of each nasal spray will be determined to determine a median (Dv50), a 10th (Dv10) and a 90th (Dv90) percentile of the cumulative undersize distribution.

According to some embodiments, for intranasal delivery, a maximum of about 25 mg/dose is administered as frequently as tolerated, e.g. every 2 minutes, 5 minutes, 10 minutes, 15 minutes, 20 minutes, 30 minutes , 40 minutes, 50 minutes, or 60 minutes, for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 3;8 hours, 39 hours, 40 hours, 41 hours, 42 hours, 43 hours, 44 hours, 45 hours, 46 hours, 47 hours, 48 hours, 49 hours, 50 hours, 51 hours, 52 hours, 53 hours, 54 hours, 55 hours, 56 hours, 57 hours, 58 hours, 59 hours, 60 hours ,61 hours, 62 hours, 63 hr, 64 hr, 65 hr, 66 hr, 67 hr, 68 hr, 69 hr, 70 hr, 71 hr, 72 hours etc. to achieve a minimum daily dose ranging from about 85 mg/day (e.g., for a child weighing 45 lbs.) to about 800 mg/day (e.g., for an adult) inclusive, i.e., at least about 85 mg/day; about 95 mg/day; about 105 mg/day, about 115 mg/day; about 125 mg/day; about 135 mg/day; about 145 mg/day; about 155 mg/day; about 165 mg/day; about 175 mg/day; about 185 mg/day; about 195 mg/day; about 205 mg/day, about 215 mg/day; about 225 mg/day; about 235 mg/day; about 245 mg/day; about 255 mg/day; about 265 mg/day; about 275 mg/day; about 285 mg/day; about 295 mg/day; about 305 mg/day, about 315 mg/day; about 325 mg/day; about 335 mg/day; about 345 mg/day; about 355 mg/day; about 365 mg/day; about 375 mg/day; about 385 mg/day; about 395 mg/day; about 405 mg/day, about 415 mg/day; about 425 mg/day; about 435 mg/day; about 445 mg/day; about 455 mg/day; about 465 mg/day; about 475 mg/day; about 485 mg/day; about 495 mg/day; about 505 mg/day, about 515 mg/day; about 525 mg/day; about 535 mg/day; about 545 mg/day; about 555 mg/day; about 565 mg/day; about 575 mg/day; about 585 mg/day; about 595 mg/day; about 605 mg/day, about 615 mg/day; about 625 mg/day; about 635 mg/day; about 645 mg/day; about 655 mg/day; about 665 mg/day; about 675 mg/day; about 685 mg/day; about 695 mg/day; about 705 mg/day, about 715 mg/day; about 725 mg/day; about 735 mg/day; about 745 mg/day; about 755 mg/day; about 765 mg/day; about 775 mg/day; about 785 mg/day; about 795 mg/day; or about 800 mg/day.

According to some embodiments, and based on HED from the mouse studies reported below in para. [00635], the minimum daily dose for a 60 kg human (132 lb) is 250 mg/day. According to some embodiments, the minimum daily dose for a 90 kg human (200 lb) is 730 mg/day.

According to some embodiments, a dose time curve is determined so that a maximum 25 mg/dose delivered intranasally at a given frequency as tolerated, e.g., every 2 minutes, 5 minutes, 10 minutes, 15 minutes, 20 minutes, 30 minutes , 40 minutes, 50 minutes, or 60 minutes, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. achieves the desired effective amount.

According to some embodiments, intranasal delivery within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before a sporting event and for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after participating in an event where an acquired brain injury is a known risk is neuroprotective. According to some embodiments, the event is a military operation. According to some embodiments, the event is a sports event. According to some embodiments, the sports event is, for example, hockey, football, soccer, baseball, polo, rugby, horseback riding, autoracing, motorcycling, skiing, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing. Other applications include emergency medicine, for example use by EMTs or emergency departments in the context of such emergencies as domestic violence, falls, car crashes, etc.

According to some embodiments, integration of the D-beta-hydroxybutyrate into polymeric carriers can achieve controlled release, including delayed release, and sustained release.

According to some embodiments, the D-beta hydroxybutyrate binds HCA2.

According to some embodiments, the composition when delivered intranasally may elevate brain D-beta hydroxybutyrate levels, e.g., to about 0.1-3 mmol/L, or about 0.1 - 1 mmol/L for at least 6 hr to at least 48 hr, inclusive, i.e., at least 6 hr, at least 7 hr, at least 8 hr, at last 9 hr, at least 10 hr, at least 11 hr, at least 12 hr, at least 13 hr, at least 14 hr, at least 15 hr, at least 16 hr, at least 17 hr, at least 18 hr, at least 19 hr, at least 20 hr, at least 21 hr, at least 22 hr, at least 23 hr, at least 24 hr, at least 25 hr, at least 26 hr, at least 27 hr, at least 28 hr ,at least 29 hr, at least 30 hr, at least 31 hr, at least 32 hr, at least 33 hr, at least 34 hr, at least 35 hr, at least 36 hr, at least 37 hr, at least 38 hr, at least 39 hr, at least 40 hr, at least 41 hr, at least 42 hr, at least 43 hr, at least 44 hr, at least 45 hr, at least 46 hr, at least 47 hr, or at least 48 hr.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, exemplary methods and materials have been described. All publications mentioned herein are incorporated herein by reference to disclose and described the methods and/or materials in connection with which the publications are cited.

It must be noted that as used herein and in the appended claims, the singular forms "a", "and", and "the" include plural references unless the context clearly dictates otherwise.

The publications discussed herein are provided solely for their disclosure prior to the filing date of the present application and each is incorporated by reference in its entirety. Nothing herein is to be construed as an admission that the present invention is not entitled to antedate such publication by virtue of prior invention. Further, the dates of publication provided may be different from the actual publication dates which may need to be independently confirmed.

EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

**Example 1. Pharmacokinetic Study of Test Article Administered to Mice.**

1. The purpose of this study is to determine tissue levels of a test article after dosing in mice.

2. *Regulatory Compliance.* This study is exploratory in nature and is not intended to comply in accordance with U.S. FDA Good Laboratory Practice (GLP) Regulations for Nonclinical Laboratory Studies, Code of Federal Regulations, Title 21 Part 58. However, this study was conducted in accordance with the test facility standard operating procedures (SPOs), the World Health Organization Quality Practices in Basic Biomedical Research guidelines, and in compliance with all state and federal regulations, including USDA Animal Welfare Act 9 CFR Parts 1-3. Federal Register 39129, July 22, 1993.

3. *Test system*

Number of animals: 39

Species/strain or breed: C57BL/6J

Age/Wt. at Arrival: 7 weeks

Sex: Female

*4. Environment and Husbandry*

Upon arrival the animals were housed 3 to 5 per cage in polycarbonate cages with wood chip bedding and suspended food and water bottles. The mice were housed either in shoebox cages (static airflow, approximately 0.045 m2 floor space) with filter tops or in individually ventilated pie cages (passive airflow, approximately 0.045-0.048 m2 floor space.

The mice were acclimated for 10 days prior to being placed in the study. An attending veterinarian was on site or on call during the live phase of the study. No concurrent medications were given.

During the acclimation and study periods, the animals were housed in a laboratory environment with temperatures ranging from 19 C to 25C and relative humidity of 30% to 70%. Automatic timers provided 12 hrs of light and 12 hrs of dark. The animals were allowed access ad libitum to Envigo Teklad 8640 diet and fresh municipal tap water.

5. *Study Design*

On study day 0 animals were randomized into treatment groups based on body weight. Following enrollment, treatment was initiated and continued as indicated below. Body weights were recorded following enrollment. Sample collection time points are outlined below On study day 1, animals were necropsied as specified in Section 6.6 below.

5.1 *Moribund or found dead animals.* If animals are found dead no samples will be taken. For animals needing to be euthanized, regardless of reason, samples will be taken as they would at necropsy (See Section 6.6). Animals on health assessment may be given SC fluids as well as hydrogel and food on the bottom of the cage.

6. *Study Group Designations*

| Group | N | Treatment | Dose Level (mg/kg) | Dose Vol. (mouse) | Dose Conc. (mg/ml) | Dose Route | Regimen |
|---|---|---|---|---|---|---|---|
| 1 | 3 | Vehicle | 0 | 20 μl* | 0 | IN | Once |
| 2 | 18 | R-(-)-3-hydroxybutyric acid sodium salt (BHB) (Sigma) | 30 | 20 μl | 29.1 | IN | Once |

(continued)

| Group | N | Treatment | Dose Level (mg/kg) | Dose Vol. (mouse) | Dose Conc. (mg/ml) | Dose Route | Regimen |
|---|---|---|---|---|---|---|---|
| 3 | 18 | R-3'-hydroxybutyl-®-3'-hydroxylbutyrate (BHB ester) (Ambeed) | 15 | 20 $\mu$l | 14.56 | IN | Once |
| *10 $\mu$l per nostril | | | | | | | |

### 7. *Test article and vehicle information*

#### 7.1 *unformulated test article storage conditions*

BHB: ambient, lightly closed in dessicator

BHB ester: 4 C under blanket of nitrogen or argon in glass ampule

#### 7.2 *Vehicle: sterile PBS*

#### 7.3 *Test article formulation instructions and calculations*

BHB salt is strongly hygroscopic, work accordingly; Make fresh daily; maintain at 4 C until use later the same day

BHB ester: ester is liquid, 95% pure and must be formulated in an oxygen free environment.

#### 7.4 *Dosing formulations and vehicle storage and* stability

Not stable; discard after use

#### 7.5 *Disposition of test articles following dosing: discard.*

#### 7.6 *Necropsy information*

*Method of euthanasia:* bleed to exsanguinate followed by cervical dislocation.
*Time points:* N=3 at each time point; 0 (vehicle); 10 min; 30 min; 1, 2, 3 and 4h

#### *Necropsy sample collection:*

Terminal blood samples were collected via cardiac blood draw and processed for plasma (K2 EDTA, 300-400 uL/animal. Plasma samples were stored at -80 C for shipment on dry ice for bioanalysis. From each animal, the entire brain was collected, flash frozen, and stored at -80 C for shipment on dry ice to bioanalysis.

### 8. *Histology Processing*

#### 8.1 *Histopathologic evaluation*

Histopathology data for samples will be assessed for appropriate parameters (as determined by pathologist) and entered into an appropriate Microsoft Office application. Current methodology for histopathologic evaluation will be reported in detail in study report. Photos in the report will be representative images of the group means.
9. *Statistical analysis methods.* From numerical data generated, arithmetic means and standard errors were calculated. Significance for all tests was set at $p \leq 0.5$.

**Results Study MPK-GBS-1**

*Summary*

A study was performed to collect plasma for pharmacokinetic monitoring after a single dose of R-(-)-3-hydroxybutyric acid sodium salt ("BHB") or R-3'-hydroxybutyl (R)-3'-hydroxybutyrate ("BHB ester") in female C57BL/6 mice. Mice aged 7 weeks were randomized by bod weight into treatment groups on study day 0, then were dosed once via intranasal route (IN) with vehicle (sterile PBS), BHB (30 mg/kg) (group 1) or BHB ester (15 mg/kg) (group 2). Vehicle control animals were euthanized immediately after dosing, and groups 2 and 3 were euthanized 10 min, 30 min, 1 h , 2 h, 3h, or 4h after dosing (N=3 per time-point) for terminal blood collection. Plasma Samples were shipped to the analytical laboratory for bioanalysis.
The live portion of the study was completed successfully including animal weighing, dosing and biological sample collection. All animals survived to the scheduled termination.

**Test System Identification**

Female C57BL/6 mice (N=39) aged 7 weeks were obtained from the Jackson Laboratory (Bar Harbor, Maine). The mice weighted approximately 16 to 22 grams (mean of approximately 19 g) at enrollment on study day 0.
The animals were identified by color-coded dots at base of tail delineating group and animal number. After randomization, all cages were labeled with protocol number, group number and animal numbers with the appropriate color-coding.
Analytical methods are shown in Table 1:

| Module | Manufacturer | Model |
|---|---|---|
| LC | Sciex | Exion |
| Autosampler | Sciex | AD Multiplate |
| MS Detection | AB Sciex | 7500 Triple Quad_10 |

**Ultraperformance liquid chromatography (UPLC) method**

Column: Restek ARC-18 (2.1 x 100 mm, 2.7 um)

Elution: Gradient, 0.8 mL/min

Mobile phase A: 0.1% formic acid in water

Mobile Phase B: 0.1% formic Acid in Acetonitrile

The UPLC gradient is shown below in Table 2

| UPLC Gradient | | | |
|---|---|---|---|
| Total Time (Min) | Flow Rate (mL/min) | A (%) | B (%) |
| Initial | 0.800 | 99 | 1 |
| 0.10 | 0.800 | 99 | 1 |
| 0.50 | 0.800 | 90 | 10 |
| 1.00 | 0.800 | 90 | 10 |
| 1.01 | 0.800 | 99 | 1 |
| 1.50 | 0.800 | 99 | 1 |
| Injection volume | 3 $\mu$L | | |

(continued)

| UPLC Gradient | | | |
|---|---|---|---|
| **Total Time (Min)** | **Flow Rate (mL/min)** | **A (%)** | **B (%)** |
| Column Temperature: | 40 C | | |

## Liquid Chromatography

The liquid chromatography setup is shown in Table 3.
Acquisition method: BHB (LC); BHB_Neg. (MS)

### Table 3

| Analyte Peak Name | BHB | Analyte Peak Area | | | Calibration Range (Absolute concentration range | |
|---|---|---|---|---|---|---|
| Analyte Retention Ttime (min) | 0.524 | LLOQ= | 79807 | Counts | 0.500 | Ug/mL |
| Analyte mass ranges(Aamu) | 103.00/59.00 Da | ULOQ | 15011062 | Counts | 100 | Ug/mL |
| | | | | | | |
| **Intl Standard Peak Name** | **BHB-13C4** | **Dilution factor** | | | **Calibration Range Qualified Sample Range** | |
| Intl Retention Time (min) | 0.513 | Minimum | 1 | Fold | 0.500 | ug/mL |
| Intl Mass Ranges (amu) | 107.00/61.00 Da | Maximum | 1 | Fold | 100 | Ug/mL |

## Bioanalytical Prep

### Plasma for BHB

Samples: 20 μL sample + 180 μL IS (BHB-13C4 500 ng/mL in CAN), mix at 350 rpm for 5 min. Centrifuge 10 min at 3000 rpm. Transfer 20 μL supernatant to deep well plate to 180 μL of water, vortex to mix. Inject via LC-MS/MS

### Brain for BHB

Homogenization: 3:1 70% IPA: Brain tissue, add zirconium beads, homogenize for 1 min in bead beater

Samples: 50 μL sample + 150 μL IS (BHB-13C4 500 ng/mL in CAN), mix at 350 rpm for 5 min. Centrifuge 10 min at 3000 rpm. Transfer 20 μL supernatant to deep well plate to 180 μL of water; vortex to mix. Inject via LC-MS/MS

**MS Detection and Calibration for BHB in Mouse plasma/PBS is shown in** <u>Table 4</u> **below.**
Peak name: BHB-13C4
Use as Internal Standard
Q1/Q3 Masses: 107/61.00 Da
Peak name: BHB
Internal standard: Tolbutamide
Q1/Q3 Masses: 103.00/59 Da

| Fit | Linear | Weighting 1/x |
|---|---|---|
| Slope | 0.215 | |
| Intercept | 0.113 | |
| Correlation Coefficient | 0.9999 | |

(continued)

| Fit | Linear | Weighting 1/x |
|---|---|---|
| Use Area | | |

MS detection and calibration for BHB in Mouse Brain/PBS is shown in Table 5 below.
Peak Name: BHB-13C4
Use as Internal Standard
Peak Name: BHB
Internal Standard: Tolbutamide
Q1/Q3 Masses: 103.00/59.00 Da

| Fit | Linear | Weighting 1/x |
|---|---|---|
| Slope | 0.710 | |
| Intercept | 0.00662 | |
| Correlation Coefficient | 0.9997 | |
| Use Area | | |

Table 6 below shows body weight and dose calculation data for study MPK-GBS-1.

**Table 6**

| TREATMENT GROUP | | |
|---|---|---|
| **Group 1**<br>**Vehicle,**<br>**IN, 1X (day 0)** | **Body Wt (g)** | **Dose Vol. 10 ul (ml) per nostril** |
| 1 | 19.45 | 0.01 |
| 2 | 18.71 | 0.01 |
| 3 | 19.21 | 0.01 |
| Mean<br>SE | 19.12<br>0.22 | |
| **Group 2**<br>**R-(-)-3-hydroxybutyric acid sodium salt (BHB) (30 mg/kg,**<br>**IN, 1x Day 0** | **Body Wt (g)** | **Dose Vol. (20 ul) (ml)** |
| 1 | 19.33 | 0.02 |
| 2 | 18.81 | 0.02 |
| 3 | 19.20 | 0.02 |
| 4 | 20.38 | 0.02 |
| 5 | 20.15 | 0.02 |
| 6 | 18.53 | 0.02 |
| 7 | 19.68 | 0.02 |
| 8 | 21.18 | 0.02 |
| 9 | 19.44 | 0.02 |
| 10 | 21.19 | 0.02 |
| 11 | 20.05 | 0.02 |
| 12 | 20.64 | 0.02 |
| 13 | 18.21 | 0.02 |

(continued)

| Group 2<br>R-(-)-3-hydroxybutyric acid sodium salt (BHB) (30 mg/kg, IN, 1x Day 0) | Body Wt (g) | Dose Vol. (20 ul) (ml) |
|---|---|---|
| 14 | 20.35 | 0.02 |
| 15 | 20.08 | 0.02 |
| 16 | 19.22 | 0.02 |
| 17 | 18.02 | 0.02 |
| 18 | 18.-04 | 0.02 |
| Mean<br>SE | 19.58<br>0.04 | |
| Group 3<br>R-3'-hydroxybutyl (R)-3'-hydroxybutyrate (BHB ester) (15 mg/kg) | Body Wt (g) | Dose Vol. 20 ul (ml) |
| 1 | 20.73 | 0.02 |
| 2 | 19.48 | 0.02 |
| 3 | 21.96 | 0.02 |
| 4 | 18.69 | 0.02 |
| 5 | 16.48 | 0.02 |
| 6 | 18.74 | 0.02 |
| 7 | 18.31 | 0.02 |
| 8 | 19.04 | 0.02 |
| 9 | 19.05 | 0.02 |
| 10 | 18.68 | 0.02 |
| 11 | 18.05 | 0.02 |
| 12 | 21.38 | 0.02 |
| 13 | 19.87 | 0.02 |
| 14 | 20.89 | 0.02 |
| 15 | 18.54 | 0.02 |
| 16 | 18.62 | 0.02 |
| 17 | 18.35 | 0.02 |
| 18 | 18.58 | 0.02 |
| Mean<br>SE | 19.24<br>0.32 | |

**FIG. 5A** is a graph of beta-hydroxybutyrate (BHB) concentration ($\mu$g/mL, y axis) in plasma on Day 14 over time (h) (x axis) following intranasal administration to C57BL/6J mice for Group 1 (vehicle), Group 2 (30 mg/kg BHB salt); Group 3 (BHB ester (15 mg/kg). **FIG. 5B** is a graph of beta-hydroxybutyrate (BHB) concentration ($\mu$g/mL, y axis) in brain on Day 14 over time (h) (x axis) following intranasal administration to C57BL/6J mice for Group 1 (vehicle), Group 2 (30 mg/kg BHB salt); Group 3 (BHB ester (15 mg/kg).

**FIG. 6A** is a graph of beta-hydroxybutyrate (BHB) concentration(ug/g, y axis) in plasma on Day 14 over time (h)(x axis) following intranasal administration to C57BL/6J mice for Group 1 (vehicle), Group 2 (30 mg/kg BHB salt); Group 3 (BHB ester (15 mg/kg). **FIG. 6B** is a graph of beta-hydroxybutyrate (BHB) concentration(ug/g, y axis) in brain on Day 14 over time (h)(x axis) following intranasal administration to C57BL/6J mice for Group 1 (vehicle), Group 2 (30 mg/kg BHB salt); Group 3 (BHB ester (15 mg/kg).

**FIG. 7** is a graph showing the endogenous baseline level of beta-hydroxybutyrate (y-axis) over time (x-axis) when BHB is administered as 15 mg/kg and 30 mg/kg. Note that the endogenous baseline in **FIG. 7** is the same as the vehicle line in **FIG. 5A** (about 19 μg/mL).

**Table 7** below shows the ratio of plasma : brain BHB over time for each group:

| | Ratio, Plasma To Brain Over Time | | | | | |
|---|---|---|---|---|---|---|
| Group | 0 h | 0.17 h | 0.5 h | 1 h | 2 h | 3h |
| 1 | 14.5 | | | | | |
| | 16.1 | | | | | |
| | 20.0 | | | | | |
| | | | | | | |
| 2 | | 9.22 | 14.0 | 7.69 | 10.8 | 11.0 |
| | | 12.1 | 6.40 | 5.71 | 9.13 | 8.4 |
| | | 12.4 | 10.5 | 9.76 | 7.48 | 14.0 |
| | | | | | | |
| 3 | | 10.2 | 7.70 | 9.91 | 6.12 | 6.03 |
| | | 7.48 | 8.11 | 7.32 | 13.3 | 8.43 |
| | | 8.92 | 9.88 | 7.33 | 12.1 | 10.3 |

**Conclusion:** Both the sodium salt of BHB (30 mg/kg) and the ester of BHB (15 mg/kg) when administered intranasally in mice reach the brain. BHB concentration peaks quickly and generally declines over time to about 50% of maximum within 1 hr in plasma and brain. Plasma levels are approximately ten fold higher than brain levels.

**Example 2. Assessment of BHB test article in the weight drop model of TBI in female mice.**

**Test System.**

Number of animals: 120 (100 + 20 extra for TBI deaths0

Species/Strain or Breed: C57BL/6J

Vendor: Jackson Labs

Age/Wt at Arrival: 11 weeks at arrival

Gender: Female

Age/Wt at Study Initiation: 12 weeks

Acclimation: 7 days

Housing: 3-4 animals/cage

**Study Design.** Animals will arrive in the facility and will have access to food and water ad libitum. On study day 0, animals will be randomized based on bodyweight. Animals will be briefly anesthetized with isoflurane and subjected to traumatic brain injury (TBI) or sham injury as listed under Injury Induction. Animals will be weighed and undergo behavior testing (Beam Walking) and necropsied according to the Necropsy section.

**Injury Induction**

On days of TBI or sham, animals will be anesthetized with 2.5% isoflurane. They will then be placed individually in the prone position in a foam bed with their heads directly under a Plexiglas tube. A brass weight (80g) will be dropped once through the tube from a specific height (0.8m) and will strike the head directly creating a midline impact injury. Animals will be monitored for the presence of the forearm fencing response as a sign of moderate TBI. Animals will be placed in a clean cage on a heating pad for recovery, and righting reflex recovery times will be recorded for all animals. Sham animals will undergo all the same procedures but will not be subjected to TBI. Animals will be given 0.1 mg/kg SC buprenorphine directly after TBI to reduce acute pain associated with the injury and directly after sham procedures for the purpose of experimental matching. Animals will be checked daily, per the IACUC-approved post-operative monitoring plan. Moribund animals, animals that have skull fractures or are exhibiting signs of limb dysfunction (i.e., dragging the leg) will not be included in the study and euthanized.

*Behavioral assay -* Beam Walking (Luong, TN et al. Assessment of motor balance and coordination in mice using the balance beam." J. Vis. Exp. (2011) 49: 2376) Fine motor coordination and balance can be assessed by the beam walking assay, which is a popular test for analyzing mice's gait in an environment that challenges their ability to balance themselves. The goal of this test is for the mouse to stay upright and walk across an elevated narrow beam to a safe platform. Performance on the beam is quantified by measuring the time it takes for the mouse to traverse the beam and the number of paw slips that occur in the process. This task is particularly useful for detecting subtle deficits in motor skills and balance that may not be detected by other motor tests, such as the Rotarod.

This test takes place over 3 consecutive days: 2 days of training and 1 day of testing. On training days, each mouse crosses the 12 mm beam 3 times and then the 6 mm beam 3 times. Beams of wider widths (i.e. 28 mm) can be used for mice that are not able to cross the narrower ones or for larger mice. Mice are placed at one end of a beam and the time required to cross to the escape box at the other end (80 cm away) is measured by the motion detectors. The timer is started by the nose of the mouse entering the center 80 cm, and stopped when the animal reaches the end of the 80 cm. The mice rest for 10 min in their home cages between training sessions on the two beams.

On the test day, times to cross each beam are recorded. Two successful trials in which the mouse did not stall on the beam are averaged. Video recordings can be used for finer analysis of slipping and other observable motor deficits. An alternative way to assess beam walking is by a neurological scoring system, which rates the inability to cross the beam as a score of "1" and the ability to traverse the beam normally with both paws on the beam surface and fewer than two foot slips as a "7." A slip is defined as the foot coming off the top of the beam. In this indexing system, a score of "3" would correspond to crossing the beam by dragging limbs. An animal that scores 1 on two trials is not retested.

## Moribund or Found Dead Animals

If animals are found dead no samples will be taken. For animals needing to be euthanized, regardless of reason, samples will be taken as they would at necropsy. Animals on health assessment may be given SC fluids as well as hydrogel and food on the bottom of the cage.

Study Group Designations are shown in Table 8 below.

### Table 8

| Group | N | Disease | Treatment* | Treatment Start | Treatment End |
|---|---|---|---|---|---|
| 1 | 20 | Sham | Saline-Saline | ~30m | 14d |
| 2 (blue) | 20 | TBI | Saline-Saline | ~30m | 14d |
| 3 (red) | 20 | TBI | Pretreatment-Saline | ~30m | 14d |
| 4 (green) | 20 | TBI | Saline-Post-treatment | ~30m | 14d |
| 5 (violet) | 20 | TBI | Pre-treatment + Post-treatment | ~30m | 14d |
| *Intranasal (20 μl) R-(-)-3-hydroxybutyric acid sodium salt | | | | | |

*Loss of righting time is defined as an* observation of the time required for the animal to regain consciousness after TBI (typically >1 min for TBI animals and < 30 seconds for shams). The animal is placed in the supine position directly after TBI or sham, and the animal is timed until it rights itself and is ambulatory on all 4 paws. This is another measure of injury severity that can be used for posthoc correlations.

## Necropsy Information

*Sacrifice Schedule:*

a. Day 0 - animals 1-5, all groups, 4h post TBI

b. Day 1 - animals 6-10, all groups, 24 h post-TBI

c. Day 14 - animals 11-20, all groups, 14d post TBI

*Method of euthanasia:* anesthesia by 3% isoflurane followed by cardiac puncture for blood isolation and bilateral pneumothorax.

*Plasma collection method:* Centrifuge plasma samples (EDTA tubes) at 2200 x g (not RPM) for 15 minutes at room temperature. Observe separation of blood cells and plasma, with plasma layer on top. Draw off only the plasma layer and take care not to disturb buffy coat when aliquoting by leaving some plasma behind and avoiding the cell layer. Aliquot samples immediately into appropriately labeled tubes and then place them in a -80 °C freezer. Note: Plasma samples do not need to clot and should be centrifuged immediately after collection.

**Table 9** Necropsy sample collection: whole blood

| Type | Process For | Anti-Coag | Gr/An | Final Volume | Storage Condition | Disposition |
|------|-------------|-----------|-------|--------------|-------------------|-------------|
| WB | Plasma | K2EDTA | All | ~400 ml Plasma Split into 2 tubes (200 tubes) | -80 | Tube 1: 100ml ELISA<br><br>Tube 2: >250ml Quanterix |

Necropsy sample collection: brain tissue

| Necropsy Tissue Sample Collection: | | | | |
|------|-------|---------|-------------------|-------------|
| Type | Gr/An | Details | Storage Condition | Disposition |
| Cortex | All | Flash freeze in LN2 (100 tubes) | -80 | Ex Vivo |
| Hippocampus | All | Flash freeze in LN2 (100 tubes) | -80 | Ex Vivo |

**Table 10** shows the planned sample analysis:

| Analysis | Sample/Tissue | Details/Notes |
|----------|---------------|---------------|
| Glial fibrillary acidic protein (GFAP) | Plasma, Cortex, Hippocampus<br>All groups, Animals 1-15 (225 samples) | ELISA |
| (Ubiquitin C-terminal hydrolase L1 (UCH-L1) | Plasma, Cortex, Hippocampus<br>All groups, Animals 1-15 (225 samples) | ELISA |
| Luminex Mouse Inflammatory Panel | Plasma, Cortex, Hippocampus<br>All groups, Animals 1-15 (225 samples) | Luminex Kit Number: MCYTOMAG-70K 4 analytes: Il-1b, IL-6, IL-10, TNFa |
| Neurofilament Light (NfL) Chain | Plasma<br>All groups, Animals 11-15 (25 samples) | Outsourced to Quanterix |

*Histology Processing.* Images will not be scored or quantitated. They will only be checked for qualitative differences between groups 1 and 2 that are expected.

*Statistical Analysis Methods:* N/A

*RESULTS*

**FIG. 8** shows results of the beam-walk behavioral assay. It shows bar graph plots in which the number of foot faults is plotted against the sample study group of C57BL/6J female mice, 20 animals per group. 20 µl of a standard stock solution of the test compound R-(-)-3-hydroxybutyric acid sodium salt (Sigma, 50 mg/kg) (BHB) prepared fresh daily was administered daily intranasally from Day 0 immediately post-TBI through day 14. Saline was added to the compound to the appropriate concentration.

*Human equivalent doses (HEDs):* 50 mg/kg in a mouse is equivalent to about 4 mg/kg in a human, resulting in a dose of about 250 mg/day in a 60 kg human (or about 132 lbs.) and about 365 mg/day in a 90 kg human (or about 200 lbs.). For the human equivalent dose of 100 mg/kg in a mouse, these numbers are multiplied by a factor of 2 to obtain the human equivalent dose, i.e., 100 mg/kg in a mouse is equivalent to about 8 mg/kg in a human, resulting in a dose of about 500 mg/day in a 60 kg human (or about 132 lbs.) and about 730 mg/day in a 90 kg human (or about 200 lbs.).

Study groups were (1) sham injury, treated with vehicle only before and after sham TBI; 2) TBI injury, treated with vehicle only before and after TBI injury; (3) pretreated with BHB IN before TBI injury, and then treated with saline post-TBI injury;(4) pretreated with saline before TBI injury and then treated with BHB post-TBI injury; and (5) pretreated with BHB IN before TBI injury and then treated with BHB post-TBI injury; and (6) pretreated with 2X dose BHB IN before TBI injury and then treated with BHB post TBO-injury.

Sham injured mice remained sure-footed. TBI mice at day 1 showed the highest number of foot faults; by day 3 the number of foot faults in this group decreased by about 50%. The pretreated, post-treated, pre and post-treated, and 2x pre- and post-treated groups all showed improvement in beam walking at day 1 and at day 3. At day 3 all treated groups were significantly improved, with best results in the post and 2x pre + post groups.

**Conclusion:** IN delivery of BHB after TBI in mice decreased foot faults, a behavioral measure of the TBI, as soon as day 1 after injury, with the greatest decrease in post-treatment and 2x pre and post groups at day 3.

**Neurofilament light chain.** Preliminary NfL plasma results were not significant (data not shown).

**Luminex Mouse Inflammatory Panel. FIG. 9A - FIG. 9C** show pg/µg total protein of IL-1β **(FIG. 9A),** IL-6 **(FIG. 9B)** and IL-10 **(FIG. 9C)** expressed in the cortex in each study group from **FIG. 8.** Results for TNF-α were not significant (data not shown). The data at the 24 h and 14 day time points in **FIG. 9A, FIG. 9B** and **FIG. 9C** show that treatment decreased expression of IL-1β, IL-6 and II-10. While IL-10 is generally thought of as an antiinflammatory cytokine, it also may promote fibrotic processes [Steen, EH et al. Adv. Wound Care (New Rochelle) (2020) 9 (4): 184-98, citing O'Garra, A. et al. Immunol. Rev. (20008) 223: 114-31]. The data for the pre + post groups are consistent with the behavioral data.

While the present invention has been described with reference to the specific embodiments thereof it should be understood by those skilled in the art that various changes may be made and equivalents may be substituted without departing from the true spirit and scope of the invention. In addition, many modifications may be made to adopt a particular situation, material, composition of matter, process, process step or steps, to the objective spirit and scope of the present invention. All such modifications are intended to be within the scope of the claims appended hereto.
Additional aspects of the present invention:

1. A composition comprising an active ingredient that is encapsulated in suspension of particles comprising a biodegradable polymer containing an effective amount of the active agent, wherein the particles are configured to bypass the blood brain barrier, wherein the active ingredient is synthetic D-beta hydroxybutyrate, and wherein targeted delivery is to the brain.

2. The composition according to 1,

a. wherein size of the particles is greater than 10 nm in diameter, greater than 15 nm in diameter; greater than 20 nm in diameter, greater than 30 nm in diameter, greater than 40 nm in diameter, greater than 50 nm in diameter, greater than 60 nm in diameter, greater than 70 nm in diameter, greater than 80 nm; in diameter, greater than 90 nm in diameter, greater than 100 nm in diameter, greater than 200 nm in diameter, greater than 300 nm in diameter, greater than 400 nm in diameter, greater than 500 nm in diameter, greater than 600 nm in diameter, greater than 700 nm in diameter, greater than 800 nm in diameter, greater than 900 nm in diameter, greater than 1000 nm in diameter, greater than 2000 nm in diameter, greater than 3000 nm in diameter, greater than 4000 nm in diameter, greater than 5000 nm in diameter, greater than 6000 nm in diameter, greater than 7000 nm in diameter, greater than 8000 nm in diameter, greater than 9000 nm in diameter, or greater than 10,000 nm in diameter; or.

b. wherein the synthetic D-beta hydroxybutyrate is dispersed throughout the particles; or the particles are impregnated with the D-beta hydroxybutyrate; or the particles comprise a matrix and the matrix comprises the D-beta hydroxybutyrate; or

c. wherein integration of the synthetic D-beta-hydroxybutyrate into polymeric carriers can achieve controlled release, including delayed release, and sustained release; or.

d. wherein the polymer comprises a mucoadhesive polymer, a thermoresponsive polymer or both; or

e. wherein a fraction of the synthetic D-beta hydroxybutyrate is adsorbed or weakly bound to the surface of the particles and contributes to a rapid initial release or burst release; or

f. wherein the particles are of any order release kinetics, including zero order release, first order release, second order release, delayed release, sustained release, immediate release, long term release, or a combination thereof; or

g. wherein release of the synthetic D-beta hydroxybutyrate from the particles is through diffusion, erosion or both; or

h. wherein the composition is formulated for intranasal delivery.

3. The composition according to 2, wherein the mucoadhesive polymer includes chitosan, alginate, and a cellulose or a derivative thereof.

4. The composition according to 3, wherein the thermoreversible polymer includes gelatin, carrageenan, methylcellulose, hydroxypropyl methylcellulose (HPMC), xyloglucan, poly (N-isopropylacrylamide-c-acrylic acid), poly(N-isopropylacrylamide (PNIPAAm)/polyethylene oxide (PEO), poloxamer (Pluronic) or PEO/polylactic acid-co-glycolic acid (PLGA).

5. The composition according to 3, wherein the polymer is selected from a cellulose derivative, a polyacrylate, starch, gelatin, a phospholipid, chitosan, poly-N-alkyl acrylamide/poly-N-isopropylacrylamide, cyclodextrin, a poloxamer and methylcellulose.

6. The composition according to 2, wherein when formulated for intranasal delivery, a maximum amount of about 25 mg/dose is administered to achieve a minimum daily dose ranging from about 85 mg/day to about 800 mg/day, inclusive.

7. A method for promoting brain health comprising (a) formulating a composition containing an active ingredient as a suspension of particles comprising a biodegradable polymer containing an effective amount of the active ingredient; (b) administering to a subject the composition comprising the active ingredient, wherein the active ingredient is D-beta hydroxybutyrate; and (c) targeting the composition to the brain, wherein burst release and controlled release of the D-beta-hydroxybutyrate can lead to effective D-beta hydroxybutyrate levels in the brain.

8. The method according to 7,

a. wherein size of the particles is greater than 10 nm in diameter, greater than 15 nm in diameter; greater than 20 nm in diameter, greater than 30 nm in diameter, greater than 40 nm in diameter, greater than 50 nm in diameter, greater than 60 nm in diameter, greater than 70 nm in diameter, greater than 80 nm; in diameter, greater than 90 nm in diameter, greater than 100 nm in diameter, greater than 200 nm in diameter, greater than 300 nm in diameter, greater than 400 nm in diameter, greater than 500 nm in diameter, greater than 600 nm in diameter, greater than 700 nm in diameter, greater than 800 nm in diameter, greater than 900 nm in diameter, greater than 1000 nm in diameter, greater than 2000 nm in diameter, greater than 3000 nm in diameter, greater than 4000 nm in diameter, greater than 5000 nm in diameter, greater than 6000 nm in diameter, greater than 7000 nm in diameter, greater than 8000 nm in diameter, greater than 9000 nm in diameter, or greater than 10,000 nm in diameter; or

b. wherein the synthetic D-beta hydroxybutyrate is dispersed throughout the particles; or the particles are impregnated with the D-beta hydroxybutyrate; or the particles comprise a matrix and the matrix comprises the D-beta hydroxybutyrate; or

c. wherein integration of the synthetic D-beta-hydroxybutyrate into polymeric carriers can achieve controlled release, including delayed release, and sustained release; or

d. wherein the polymer comprises a mucoadhesive polymer, a thermoresponsive polymer or both; or

e. wherein a fraction of the synthetic D-beta hydroxybutyrate is adsorbed or weakly bound to the surface of the particles and contributes to a rapid initial release or burst release; or

f. wherein the particles are of any order release kinetics, including zero order release, first order release, second order release, delayed release, sustained release, immediate release, long term release, or a combination thereof; or

g. wherein release of the synthetic D-beta hydroxybutyrate from the particles is through diffusion, erosion or both; or

h. wherein the composition is formulated for oral delivery or intranasal delivery.

9. The method according to 8, wherein the mucoadhesive polymer includes chitosan, alginate, and a cellulose or a derivative thereof.

10. The method according to 9, wherein the thermoreversible polymer includes gelatin, carrageenan, methylcellulose, hydroxypropyl methylcellulose (HPMC), xyloglucan, poly (N-isopropylacrylamide-c-acrylic acid), poly(N-isopropylacrylamide (PNIPAAm)/polyethylene oxide (PEO), poloxamer (Pluronic) or PEO/polylactic acid-co-glycolic acid (PLGA).

11. The method according to 9, wherein the polymer is selected from a cellulose derivative, a polyacrylate, starch, gelatin, a phospholipid, chitosan, poly-N-alkyl acrylamide/poly-N-isopropylacrylamide, cyclodextrin, a poloxamer and methylcellulose.

12. The method according to 8, wherein when formulated for intranasal delivery, a maximum amount of about 25 mg/dose is administered to achieve a minimum daily dose ranging from about 85 mg/day to about 800 mg/day inclusive.

13. A method for reducing risk of brain damage due to an acquired brain injury for a subject susceptible to, or otherwise at risk of, the acquired brain injury comprising administering a pharmaceutical composition comprising a pharmaceutically acceptable carrier and a formulation comprising a therapeutic amount of an API, wherein the API is a synthetic D-beta-hydroxybutyrate,

wherein the administering is intranasally (IN),

wherein the administering comprises targeted delivery to the olfactory region of the nasal cavity of the subject;

wherein the targeted delivery achieves an effective amount of the API in the brain, and

wherein the effective amount of the pharmaceutical composition

(i) eliminates or reduces the risk of the acquired brain injury or

(ii) lessens the severity of the acquired brain injury, or

(iii) delays the onset of the acquired brain injury; or

(iv) a combination thereof.

14. The method according to 13,

(a) wherein the acquired brain injury is a traumatic brain injury; or

(b) wherein the administering is neuroprotective; or

(c) wherein the subject's risk of the acquired brain injury is increased by participating in an event where an acquired brain injury is a known risk; or

(d) wherein the administering is a maximum amount of 25 mg/dose within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, etc. before the event; or

(e) wherein the pharmaceutical composition comprises a liquid spray formulation or a dry powder formulation; or

(f) wherein the pharmaceutical composition comprises the active formulated as a solution, a suspension or a dispersion; or

(g) wherein a minimum daily dose of the pharmaceutical composition contains from about 85 mg/day to about 800 mg/day, inclusive, of the API.

15. The method according to 14, wherein the traumatic brain injury comprises a concussion.

16. The method according to 14, wherein the event is a military operation or a sports event.

17. The method according to 16, wherein the sports event is hockey, football, soccer, baseball, polo, rugby, horseback riding, a car race, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing.

18. The method according to 14, wherein

(1). the pharmaceutical composition is delivered as a liquid spray comprising a droplet size distribution comprising droplets containing the API; or

(2). the dry powder formulation when aerosolized comprises a cloud of very fine particles comprising the API; or

(3). the formulation is formulated with excipients.

19. The method according to 18, wherein the droplet size distribution of the liquid spray ranges from about 20 μ to about 120 μm, inclusive containing a therapeutic amount of the API.

20. The method according to 18, wherein the distribution of particles is depleted of smaller particles that would otherwise go into the lung.

21. A method for treating symptoms of a brain injury of a subject comprising neuroinflammation comprising administering a pharmaceutical composition comprising a pharmaceutically acceptable carrier and a formulation comprising a therapeutic amount of an API, wherein the API is a synthetic D-beta-hydroxybutyrate,

wherein the administering is intranasally (IN);

wherein the administering comprises targeted delivery to the olfactory region of the nasal cavity of the subject,

wherein the targeted delivery achieves an effective amount of the API in the brain,

wherein the effective amount of the pharmaceutical composition accomplishes one or more therapeutic effects including:

(i) reducing the severity of the acquired brain injury; or

(ii) limiting development of symptoms characteristic of the acquired brain injury being treated; or

(iii) limiting worsening of symptoms characteristic of the acquired brain injury being treated; or

(iv) limiting recurrence of the acquired brain injury in subjects that have previously had the acquired brain injury; or

(v) limiting recurrence of symptoms in subjects that were previously asymptomatic for the acquired brain injury; or

(vi) a combination thereof.

22. The method according to 21,

(a) wherein the acquired brain injury is a traumatic brain injury; or

(b) wherein the administering is neuroprotective; or

.(c) wherein the subject's risk of the acquired brain injury is increased by participating in an event where an acquired brain injury is a known risk; or

(d) wherein the administering is a maximum amount of 25 mg/dose within 1 minute; within 5 minutes; within 10 minutes; within 15 minutes; within 30 minutes; within 45 minutes; within 1 hour, within 2 hours, within 3 hours, within 4 hours, within 5 hours, within 6 hours, within 7 hours, within 8 hours, within 9 hours, within 10 hours, within 11 hours, within 12 hours, within 13 hours, within 14 hours, within 15 hours, within 16 hours, within 17 hours, within 18 hours, within 19 hours, within 20 hours, within 21 hours, within 22 hours, within 23 hours, within 24 hours, within 1 day, within 2 days, within 3 days, within 4 days, within 5 days, within 6 days, within 7 days, within 8 days, within 9 days, within 10 days, within 11 days, within 12 days, within 13 days, within 14 days, within 15 days, within 16 days, within 17 days, within 18 days, within 19 days, within 20 days, within 21 days, of the brain injury and for at least 1 hour, 2 hours, 3 hours, 4 hours, 5 hours, 6 hours, 7 hours, 8 hours, 9 hours, 10 hours, 11 hours, 12 hours, 13 hours, 14 hours, 15 hours ,16 hours, 17 hours, 18 hours, 19 hours, 20 hours, 21 hours, 22 hours, 23 hours, at least 24 hours, at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, etc. after the event where the acquired brain injury is acquired; or

(e) wherein the pharmaceutical composition comprises a liquid spray formulation or a dry powder formulation; or

(f) wherein the pharmaceutical composition comprises the active formulated as a solution, a suspension or a dispersion. or

(g) wherein a minimum daily dose of the pharmaceutical composition contains from about 85 mg/day to about 800 mg/day, inclusive, of the API.

23. The method according to 22, wherein the traumatic brain injury comprises a concussion.

24. The method according to 22, wherein the event is a military operation or a sports event.

25. The method according to 24, wherein the sports event is hockey, football, soccer, baseball, polo, rugby, horseback riding, a car race, gymnastics, mountain climbing, basketball, mixed martial arts, Brazilian Jiu-jitsu, Muay Thai, taekwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing

26. The method according to 22, wherein

(1). the pharmaceutical composition is delivered as a liquid spray comprising a droplet size distribution comprising droplets containing the API; or

(2). the dry powder formulation when aerosolized comprises a cloud of very fine particles comprising the API; or

(3). the formulation is formulated with excipients.

27. The method according to 26, wherein the droplet size distribution of the liquid spray ranges from about 20 $\mu$ to about 120 $\mu$m, inclusive containing a therapeutic amount of the API.

28. A method for reducing risk of an acquired brain injury for a subject susceptible to, or otherwise at risk of, the acquired brain injury and for treating symptoms of the acquired brain injury after the brain injury has been acquired by the subject, wherein the subject's risk of the acquired brain injury is increased by participating in an event where an acquired brain injury is a known risk; comprising

(a) administering a pharmaceutical composition comprising a pharmaceutically acceptable carrier and a formulation comprising a therapeutic amount of an API, wherein the API is a synthetic D-beta-hydroxybutyrate before an event,

wherein the administering is intranasally (IN) and neuroprotective,

wherein the administering comprises targeted delivery to the olfactory region of the nasal cavity of the subject;

wherein the targeted delivery achieves an effective amount of the API in the brain, and

wherein the effective amount of the pharmaceutical composition

(i) eliminates or reduces the risk of the acquired brain injury or

(ii) lessens the severity of the acquired brain injury, or

(iii) delays the onset of the acquired brain injury; or

(iv) a combination thereof;
and

(b) continuing the administering intranasally (IN) after acquiring the brain injury at the event,

wherein the effective amount of the pharmaceutical composition accomplishes one or more therapeutic effects comprising:

(i') reducing the severity of the acquired brain injury;

(ii') limiting development of symptoms characteristic of the acquired brain injury being treated;

(iii') limiting worsening of symptoms characteristic of the acquired brain injury being treated;

(iv') limiting recurrence of the acquired brain injury in subjects that have previously had the acquired brain injury; or

(v') limiting recurrence of symptoms in subjects that were previously asymptomatic for the acquired brain injury;

(vi') or a combination thereof.

29. The method according to 28,

(a) wherein the acquired brain injury is a traumatic brain injury; or

(b) wherein the pharmaceutical composition comprises a liquid spray formulation or a dry powder formulation; or

(c) wherein the pharmaceutical composition comprises the API formulated as a solution, a suspension or a dispersion; or

(d) wherein a minimum daily dose of the pharmaceutical composition contains from about 85 mg/day to about 800 mg/day, inclusive, of the API.

30. The method according to 29, wherein the traumatic brain injury comprises a concussion.

31. The method according to 29, wherein the event is a military operation or a sports event.

32. The method according to 31, wherein the sports event is hockey, football, soccer, baseball, polo, rugby. horseback riding, a car race, gymnastics, mountain climbing, basketball, mixed martial arts, Brazilian Jiu-jitsu, Muay Thai, tae kwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing.

33. The method according to 29,

wherein the administering is a maximum amount of 25 mg/dose every 2 minutes, 5 minutes, 10 minutes, 20 minutes, 30 minutes; 40 minutes, 50 minutes, or 60 minutes, for at least 1 hour, for 2 hours, for 3 hours, for 4 hours, for 5 hours, for 6 hours, for 7 hours, for 8 hours, for 9 hours, for 10 hours, for 11 hours, for 12 hours, for 13 hours, for 14 hours, for 15 hours, for 16 hours, for 17 hours, for 18 hours, for 19 hours, for 20 hours, for 21 hours, for 22 hours, for 23 hours, for 24 hours, within at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days before the event; and

a maximum of 25 mg/dose every 2 minutes, 5 minutes, 10 minutes, 20 minutes, 30 minutes; 40 minutes, 50 minutes, or 60 minutes for at least 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, or at least 24 hours, for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, after the brain injury is acquired.

34. The method according to 29, wherein

(1) the pharmaceutical composition is delivered as a liquid spray comprising a droplet size distribution comprising droplets containing the API; or

(2). the dry powder formulation when aerosolized comprises a cloud comprising a distribution of fine particles comprising the API; or

(3) the formulation is formulated with excipients.

35. The method according to 34, wherein the droplet size distribution of the liquid spray ranges from about 20 $\mu$ to about 120 $\mu$m, inclusive containing a therapeutic amount of the API.

36. The method according to 34, wherein the distribution of particles is depleted of smaller particles that would otherwise go into the lung.

37. A pharmaceutical composition for use in reducing risk of brain damage due to an acquired brain injury in a subject susceptible to, or otherwise at risk of, the acquired brain injury, said composition comprising a pharmaceutically acceptable carrier and a formulation comprising a therapeutic amount of an API, wherein the API is a synthetic D-beta-hydroxybutyrate, wherein the treatment comprises targeted delivery of a maximum amount of 25 mg/dose to the olfactory region of the nasal cavity of the subject (a) before the subject participates in an event where an acquired brain injury is a known risk; (b) after the subject participates in the event for use in treating symptoms of the acquired brain injury of the subject comprising neuroinflammation; or both.

38. The pharmaceutical composition for use according to 37,

wherein the targeted delivery achieves an effective amount of the API in the brain of the subject; and

wherein the effective amount of the pharmaceutical composition

(i) limits development of symptoms characteristic of the acquired brain injury; and/or

(ii) limits worsening of symptoms characteristic of the acquired brain injury; and/or

(iii) limits recurrence of the acquired brain injury in subjects that have previously had the acquired brain injury; and/or

(v) limits recurrence of symptoms in subjects that were previously asymptomatic for the acquired brain injury;

39. The pharmaceutical composition for use according to 37,

(a) wherein the acquired brain injury is a traumatic brain injury; and/or

(b) wherein the brain damage comprises neuroinflammation; and/or

(b) wherein the event is a sports event; and/or

(c) wherein the event is a military operation; and/or

(d) wherein the administering is neuroprotective; and/or

(e) wherein the pharmaceutical composition comprises the API formulated as a solution, a suspension or a dispersion; and/or

(f) wherein a minimum daily dose of the pharmaceutical composition contains from about 85 mg/day to about 800 mg/day, inclusive, of the API.

40. The pharmaceutical composition for use according to 39, wherein the traumatic brain injury comprises a concussion.

41. The pharmaceutical composition for use according to 39, wherein the sports event is hockey, football, soccer, baseball, polo, rugby, horseback riding, autoracing, motorcycling, skiing, gymnastics, mountain climbing, basketball, mixed martial arts, Brazilian Jiu-jitsu, Muay Thai, tae kwondo, kickboxing, boxing, wrestling, lacrosse, softball, cheerleading, volleyball, beach volleyball, alpine skiing, water skiing, wakeboarding, snowboarding, skateboarding, kayaking, handball, cycling, mountain biking, barrel racing, bull riding, BASE jumping, skydiving, paragliding, tennis, squash, bicycle motocross (BMX), motocross, surfing, bobsled, luge, skeleton, broomball, hurling, camogie, cricket, diving, field hockey, figure skating, speed skating, sailing, ski jumping, ultimate frisbee, water polo, or windsurfing.

**Claims**

1. A pharmaceutical composition for use in treating traumatic brain injury (TBI) in a subject in need thereof comprising a therapeutically effective amount of a composition comprising D-beta-hydroxybutyrate formulated for intranasal delivery.

2. The pharmaceutical composition for use according to claim 1, wherein:

the subject comprises neuroinflammation;
the pharmaceutical composition comprises a pharmaceutically acceptable carrier and a formulation comprising a therapeutic amount of an API, wherein the API is a synthetic D-beta-hydroxybutyrate; and
the pharmaceutical composition is administered intranasally (IN), wherein the administration comprises targeted delivery to the olfactory region of the nasal cavity of the subject, wherein the targeted delivery achieves a therapeutically effective amount of the API in the brain.

3. The pharmaceutical composition for use according to claim 2, wherein an effective amount of the pharmaceutical composition accomplishes one or more therapeutic effects including:

(i) reducing the severity of the acquired brain injury;
(ii) limiting development of symptoms characteristic of the acquired brain injury being treated;
(iii) limiting worsening of symptoms characteristic of the acquired brain injury being treated;
(iv) limiting recurrence of the acquired brain injury in subjects that have previously had the acquired brain injury;
(v) limiting recurrence of symptoms in subjects that were previously asymptomatic for the acquired brain injury; or
(vi) a combination thereof.

4. The pharmaceutical composition for use according to any one of claims 1-3, wherein the pharmaceutical composition is administered to a subject after the subject sustains an external mechanical force to the head, optionally wherein the pharmaceutical composition is administered within 1 hour, within 2 hours, within 3 hours, within 4 hours, within 5 hours , within 6 hours, within 7 hours, within 8 hours, within 9 hours, within 10 hours, within 11 hours, within 12 hours, or within 24 hours after the subject sustains an external mechanical force to the head.

5. The pharmaceutical composition for use according to any one of claims 1-4, wherein the D-beta-hydroxybutyrate is a salt or an ester of D-beta-hydroxybutyrate.

6. The pharmaceutical composition for use according to any one of claims 1-5, wherein the pharmaceutical composition is formulated as a spray or as a dry powder.

7. The pharmaceutical composition for use according to any one of claims 1-6, wherein the pharmaceutical composition is administered multiple times.

8. A pharmaceutical composition for use in prophylactically limiting the development of symptoms characteristic of a traumatic brain injury (TBI) in a subject in need thereof, comprising a therapeutically effective amount of a composition comprising D-beta-hydroxybutyrate formulated for intranasal delivery.

9. The pharmaceutical composition for use according to claim 8, wherein the pharmaceutical composition is for use in a method for reducing risk of brain damage due to the acquired brain injury for a subject susceptible to, or otherwise at risk of, the acquired brain injury.

10. The pharmaceutical composition for use according to claim 8 or 9, wherein:

the pharmaceutical composition comprises a pharmaceutically acceptable carrier and a formulation comprising a therapeutic amount of an API, wherein the API is a synthetic D-beta-hydroxybutyrate; and
the pharmaceutical composition is administered intranasally (IN), wherein the administration comprises targeted delivery to the olfactory region of the nasal cavity of the subject and the targeted delivery achieves therapeutically effective amounts of the API in the brain.

11. The pharmaceutical composition for use according to claim 10, wherein an effective amount of the pharmaceutical composition:

(i) eliminates or reduces the risk of the acquired brain injury or
(ii) lessens the severity of the acquired brain injury, or
(iii) delays the onset of the acquired brain injury; or
(iv) a combination thereof.

12. The pharmaceutical composition for use according to any one of claims 8-11, wherein the pharmaceutical composition is administered to the subject before the subject engages in an activity that elevates the risk of the subject sustaining a TBI, optionally wherein the pharmaceutical composition is administered within 1 hour, within 2 hours, within 3 hours, within 4 hours, within 5 hours, within 6 hours, within 7 hours, within 8 hours, within 9 hours, within 10 hours, within 11 hours, within 12 hours, or within 24 hours before the activity.

13. The pharmaceutical composition for use according to any one of claims 8-12, wherein the D-beta-hydroxybutyrate is a salt or an ester of D-beta-hydroxybutyrate.

14. The pharmaceutical composition for use according to any one of claims 8-13, wherein the pharmaceutical composition is formulated as a spray or as a dry powder.

15. The pharmaceutical composition for use according to any one of claims 8-14, wherein the pharmaceutical composition is administered multiple times.

Traumatic
Insult

Primary Injuries
• Biomechanical damage
• Injury to neuro−axonal structures
• Mechanical stress/deformation
• Stretching, compressing, and tearing of cells

*Time: microseconds ⟶ seconds*

Secondary Injuries
• Excitotoxicity
• Calcium overload
• Impaired glucose metabolism
• Oxidative stress
• Mitochondrial dysfunction
• Inflammation

*Time: minutes ⟶ weeks*

Post Traumatic
Energy Crisis

Neuropathologies
• Neurodegeneration
• Cognitive dysfunction
• Neuropsychiatric comorbidities

*Time: weeks ⟶ years*

FIG.1

FIG.2A

FIG.2B

FIG.3

FIG.4

Plasma BHB

FIG.5A

Brain BHB

FIG.5B

FIG.6A

FIG.6B

FIG.7

EP 4 631 364 A2

FIG.8

**IL-1β Cortex**
$F_{(5,72)} = 7.12,\ p<0.001$

FIG.9A

**IL-6 Cortex**
*Intx term; 1-way ANOVA at each time point*

FIG.9B

**IL-10 Cortex**
$F_{(5,72)} = 8.53,\ p<0.001$

FIG.9C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63316845 A

**Non-patent literature cited in the description**

- **ROBSON MC et al.** *Curr Probl Surg*, 2001, vol. 38, 72-140
- **VELNAR T et al.** *The Journal of International Medical Research*, 2009, vol. 37, 1528-1542
- **ALONSO JE et al.** *Surg Clin North Am*, 1996, vol. 76, 879-903
- **WEYRICH AS** ; **ZIMMERMAN GA**. *Trends Immunol*, September 2004, vol. 25 (9), 489-495
- **SINGER AF** ; **CLARK RA**. *N Engl J Med*, 02 September 1999, vol. 341 (10), 738-746
- **HELDIN, C.** ; **WESTERMARK B.** The molecular and cellular biology of wound repair. Plenum Press, 1996, 249-273
- 3. Repair, Regeneration and Fibrosis. **SEPHEL, G.C.** ; **WOODWARD, S.C.** Wolters Kluwyer Health, /Lippincott Williams & Wilkins. 2008, vol. 71
- **BROWN, E.** *Phagocytosis, Bioessays*, 1995, vol. 17, 109-117
- **RICHES, D.** ; **IN CLARK R.** The molecular and cellular biology of wound repair. Plenum Press, 95-141
- **RAPPOLEE, D. et al.** *Science*, 1988, vol. 241, 708-712
- **LEIBOVICH, S** ; **ROSS, R.** *Am J Pathol*, 1975, vol. 78, 1-100
- **PALADINI, R. et al.** *J. Cell Biol*, 1996, vol. 132, 381-397
- **GOLIGER, J.** *Paul, D. Mol Biol Cell*, 1995, vol. 6, 1491-1501
- **GABBIANI, G. et al.** *J Cell Biol*, 1978, vol. 76, 561-568
- **CLARK, R.** *J Invest Dermatol*, 1990, vol. 94, 128S-134S
- **PILCHER, B. et al.** *J Cell Biol*, 1997, vol. 137, 1445-1457
- **BUGGE, T. et al.** *Cell*, 1996, vol. 87, 709-719
- **MIGNATTI, P. et al.** The molecular and cellular biology of wound repair. Plenum Press, 1996, 427-474
- Epidermal Growth Factor and Transforming Growth Factor-α. **NANNEY, L.** ; **KING, L.** The molecular and cellular biology of wound repair. Plenum Press, 1996, 171-194
- **WERNER, S. et al.** *Science*, 1994, vol. 266, 819-822
- Modulation of Wound Repair by Members of the Fibroblast Growth Factor family. **ABRAHAM, J.** ; **KLAGSBURN, M.** The molecular and cellular biology of wound repair. Plenum Press, 1996, 195-248
- **CLARK R. et al.** *J. Invest Dermatol*, 1982, vol. 79, 264-269
- Wound Healing and Wound Infection: Theory and Surgical Practice. Appleton-Century-Crofts, 1980
- Transforming Growth Factor-1. **ROBERTS, A.** ; **SPORN, M**. The molecular and cellular biology of wound repair. Plenum Press, 1996, 275-308
- **GRAY, A. et al.** *J Cell Sci*, 1993, vol. 104, 409-413
- **XU, J.** ; **CLARK, R.** *J Cell Biol*, 1996, vol. 132, 239-149
- **ROBSON, M. et al.** *Lancet*, 1992, vol. 339, 23-25
- **STEED, D.** *J Vasc Surg*, 1995, vol. 21, 71-78
- **ROBSON, M. et al.** *Ann Surg*, 1992, vol. 216, 401-406
- **CLARK, R. et al.** *J. Invest Dermatol*, 1982, vol. 79, 264-269
- **GREILING, D.** ; **CLARK R.** *J. Cell Sci*, 1997, vol. 110, 861-870
- **XU, J.** ; **CLARK, R.** *J Cell Sci*, 1996, vol. 132, 239-249
- **CLARK, R. et al.** *J Cell Sci*, vol. 108, 1251-1261
- Proteinases and Tissue Remodeling. **MIGNATTI, P. et al.** The molecular and cellular biology of wound repair. Plenum Press, 1996, 427-474
- **VAALAMO, M. et al.** *J Invest Dermatol*, 1997, vol. 109, 96-101
- **CLARK, R. et al.** *J Cell Sci*, 1995, vol. 108, 1251-1261
- **WELCH, M. et al.** *J. Cell Biol*, 1990, vol. 110, 133-145
- Angiogenesis. **MADRI, J. et al.** The molecular and cellular biology of wound repair. Plenum Press, 1996, 355-371

- **FOLKMAN, J.** ; **D'AMORE**. *P, Cell*, 1996, vol. 87, 1153-1155
- **BROWN, L. et al.** *J Exp Med*, 1992, vol. 176, 1375-1379
- **NISSEN, N. et al.** *Am J Pathol*, 1998, vol. 152, 1445-1552
- **CLARK, R. et al.** *J. Exp Med*, 1982, vol. 156, 646-651
- **BROOKS, P. et al.** *Science*, 1994, vol. 264, 569-571
- **PINTUCCI, G. et al.** *Semin Thromb Hemost*, 1996, vol. 22, 517-524
- **ILAN, N. et al.** *J Cell Sci*, 1998, vol. 111, 3621-3631
- **FOLKMAN, J.** Angiogenesis and angiogenesis inhibition: an overview. *EXS*, 1997, vol. 79, 1-8
- **WELCH, M. et al.** *J Cell Biol*, 1990, vol. 110, 133-145
- The role of the myofibroblast in wound healing and fibrocontractive diseases. **DESMOULIERE, A.** ; **GABBIANI, G.** The molecular and cellular biology of wound repair. Plenum Press, 1996, 391-423
- **MONTESANO, R.** ; **ORCI**. *Proc Natl Acad Sci USA*, 1988, vol. 85, 4894-4897
- **CLARK, R. et al.** *J Clin Invest*, 1989, vol. 84, 1036-1040
- **SCHIRO, J. et al.** *Cell*, 1991, vol. 67, 403-410
- **WOODLEY, D. et al.** *J Invest Dermatol*, 1991, vol. 97, 580-585
- **MADLENER, M. et al.** *Exp Cell Res*, 1998, vol. 242, 201-210
- **MONTINE, TJ et al.** *Acta Neuropathol.*, 2012, vol. 123 (1), 1-11
- **SELKOE, DJ**. *Science*, 2002, vol. 298 (5594), 789-91
- **SPIRES-JONES, TL** ; **HYMAN, BT.** *Neuron*, 2014, vol. 82 (4), 756-71
- **HARDY, J.** ; **SELKOE, DJ.** *Science*, 2002, vol. 297 (5580), 353-6
- **MONCKE-BUCHNER, E. et al.** *Nucleic Acid Res.*, 2002, vol. 30 (16), e83
- **VERBER, NS et al.** *Front. Neurol.*, 2019, vol. 10, 291
- **WOOLEY, SC** ; **JONATHAN**. *SK. Phys. Med. Rehabil. Clin. N. Am.*, 2008, vol. 19, 607-17
- **NILANTHA DE SILVA, R. et al.** *Pract. Neurol.*, 2019, vol. 19 (3), 196-207
- **NOWINSKI, CJ et al.** *Front. Neurol.*, 2022, vol. 13, 938163
- Pharmacology and Therapeutics. **MINTZER, S.** Principles to Practice. Elsevier, Inc., 2009, 663-83
- SF. Genetics of epilepsy syndromes. **ID., CITING BERKOVIC**. Epilepsy: A comprehensive Textbook. Philadelphia: Lippincott-Raven, 1997
- **PRINCE, D.A.** ; **FUTAMACHI, KT.** Intracellular recordings in chronic focal epilepsy. *Brain Res.*, 1968, vol. 11, 681-4
- **DICHTER, M.** ; **SPENCER, WA.** Penicillin-induced interictal discharges from the cat hippocampus. I. Characteristics and topographical features. *J. Neurophysiol.*, 1969, vol. 32, 649-62
- **TIAN, GF et al.** An astrocytic basis of epilepsy. *Nature Med.*, 2005, vol. 11, 973-81
- **TEASDALE, G.** ; **JENNETT, B.** *Lancet*, 1974, 81-84
- **TEASDALE, G.** ; **JENNETT, B.** *Acta Neurochir.*, 1976, vol. 34, 45-55
- **PRINS, ML** ; **MATSUMOTO, JH.** The collective therapeutic potential of cerebral ketone metabolism in traumatic brain injury. *J. Lipid Res.*, 2014, vol. 55 (12), 2450-7
- **FINEMAN I. et al.** Concussive brain injury is associated with a prolonged accumulation of calcium: a 45Ca autoradiographic study. *Brain Res.*, 1993, vol. 624, 94-102
- **KATAYAMA Y. et al.** Massive increases in extracellular potassium and the indiscriminate release of glutamate following concussive brain injury. *J. Neurosurg.*, 1999, vol. 73, 889-900
- **YOSHINO A. et al.** Dynamic changes in local cerebral glucose utilization following cerebral conclusion in rats: evidence of a hyper- and subsequent hypometabolic state. *Brain Res.*, 1991, vol. 561, 106-119
- **BERGSNEIDER M. et al.** Cerebral hyperglycolysis following severe traumatic brain injury in humans: a positron emission tomography study. *J. Neurosurg.*, 1997, vol. 86, 241-251
- **PRINS, M. et al.** Mapping cerebral glucose metabolism during spatial learning: interactions of development and traumatic brain injury. *J. Neurotrauma.*, 2001, vol. 18, 31-46
- **THOMAS, S. et al.** Cerebral metabolic response to traumatic brain injury sustained early in development: a 2-deoxy-D-glucose autoradiographic study. *J. Neurotrauma.*, 2000, vol. 17, 649-665
- **HOVDA, DA et al.** Long-term changes in metabolic rates for glucose following mild, moderate, and severe concussive head injuries in adult rats. *Soc. Neurosci.*, 1994, vol. 20, 845
- **BITNIK, BL et al.** Upregulation of pentose phosphate pathway and preservation of tricarboxylic acid cycle flux after experimental brain injury. *J. Neurotrauma*, 2005, vol. 22, 1052-1065
- **HALL, ED et al.** Brain hydroxyl radical generation in acute experimental head injury. *J. Neurochem.*, 1993, vol. 60, 588-594
- **ALTHAUS, JS et al.** The use of salicylate hydroxylation to detect hydroxyl radical generation in ischemic and traumatic brain injury. Reversal by tirilazad mesylate (U-74006F). *Mol. Chem. Neuropathol.*, 1993, vol. 20, 147-162

- **MARKLUND, N. et al.** Effects of the nitrone radical scavengers PBN and S-PBN on in vivo trapping of reactive oxygen species after traumatic brain injury in rats. *J. Cereb. Blood Flow Metab.*, 2001, vol. 21, 1259-1267
- **SEN, S. et al.** Oxypurinol inhibits free radical release from the cerebral cortex of closed head injured rats. *Neurosci. Lett.*, 1993, vol. 162, 117-120
- **SENS, S. et al.** Alpha-phenyl-tert-butyl-nitrone inhibits free radical release in brain concussion. *Free Radic. Biol. Med*, 1994, vol. 16, 685-691
- **SHELINE, C. et al.** Zinc-induced cortical neuronal death: contribution of energy failure attributable to loss of NAD(+) and inhibition of glycolysis. *J. Neurosci.*, 2000, vol. 20, 3139-3146
- **SINGH, IN et al.** Time course of post-traumatic mitochondrial oxidative damage and dysfunction in a mouse model of focal traumatic brain injury: implications for neuroprotective therapy. *J. Cereb. Blood Flow Metab.*, 2006, vol. 26, 1407-1418
- **LEE, S. et al.** Evidence for energy failure following irreversible traumatic brain injury. *Ann. N. Y. Acad. Sci.*, 1999, vol. 893, 337-340
- **RUSSO, M.** ; **MCGAVERN, DB**. Inflammatory neuroprotection following traumatic brain injury. *Science*, 2016, vol. 353 (6301), 783-6
- **CORPS, KN. et al.** *JAMA Neurol.*, 2015, vol. 72, 355-62
- **LOU, N. et al.** *Proc. Natl Acad. Sci. USA*, 2016, vol. 113, 1074-9
- **STIRLING, DP et al.** *J. Neurosci.*, 2009, vol. 29, 753-64
- **ROTH, TL et al.** *Nature*, 2014, vol. 505, 223-8
- **SHECHTER, R. et al.** *Immunity*, 2013, vol. 38, 555-69
- **WALSH, JT et al.** *J. Clin. Invest.*, 2015, vol. 125, 699-714
- **RAPOSO, C. et al.** *J. Neurosci.*, 2014, vol. 34, 10141-55
- **ALBERTS, B. et al.** Molecular Biology of the Cell. Garland Publishing, 1983, vol. 2, 67-75
- **BRYANT, NJ et al.** *Nat. Rev. Mol. Cell Biol.*, 2002, vol. 3 (4), 267-77
- **DEFRONZO, RA.** *Med. Clin. N. Am.*, 2004, vol. 88, 787-835
- **GERICH, JE**. *Diabetes Obes. Metab.*, 2000, vol. 2
- **DRUCKER** ; **NAUK**. *Lancet*, 2006, vol. 368, 1696-1705
- **DRUCKER**. *Cell Metab.*, 2006, vol. 3, 153-165
- **GERSHUNI, VM et al.** *Curr. Nutr. Rep.*, 2018, vol. 7 (3), 97-106
- **SATO, K. et al.** Insulin, Ketone bodies and mitochondrial energy transduction. *FASEB J.*, 1995, vol. 9, 651-58
- **VOLEK, JS** ; **PHINNEY SD.** The Art and Science of Low Carbohydrate Living. Beyond Obesity, LLC

- **BOUGH, KJ et al.** Mitochondrial biogenesis in the anticonvulsant mechanism of the ketogenic diet. *Ann. Neurol.*, 2006, vol. 60, 223-35
- **AHOLA-ERKKILA, S. et al.** Ketogenic diet slows down mitochondrial myopathy progression in mice. *Hum. Mol. Genet.*, 2010, vol. 19, 1974-84
- **NEWMAN, JC** ; **VERLDIN E.** Beta-hydroxybutyrate: much more than a metabolite. *Diabetes Res. Clin. Pract.*, 2014, vol. 106, 173-81
- **LAFFEL, L.** Ketone Bodies: a review of physiology, pathophysiology and application of monitoring to diabetes. *Diabetes Metab. Res. Rev.*, 1999, vol. 15, 412-26
- **VEECH, RL et al.** Ketone bodies, potential therapeutic uses. *IUBMB Life*, 2001, vol. 51, 241-47
- **WESTMAN, EC et al.** Low-carbohydrate nutrition and metabolism. *Am. J. Clin. Nutr.*, 2007, vol. 86, 276-84
- **VOLEK, JS et al.** Carbohydrate restriction has a more favorable impact on the metabolic syndrome than a low-fat diet. *Lipids*, 2009, vol. 44, 297-309
- **VOLEK, JS** ; **FEINMAN, RD.** Carbohydrate restriction improves the features of Metabolic syndrome. *Metabolic Syndrome may be defined by the response to carbohydrate restriction. Nutr. Metab.*, 2005, vol. 2, 31
- **VOLEK JS et al.** Dietary carbohydrate restriction induces a unique metabolic state positively affecting atherogenic dyslipidemia, fatty acid partitioning, and metabolic syndrome. *Prog Lipid Res*, 2008, vol. 47, 307-318
- **PRINS, ML** ; **MATSUMOTO**. JH. *J. Lipid Res.*, 2014, vol. 55, 2450-55
- **PAN J. W. et al.** Measurement of beta-hydroxybutyrate in acute hyperketonemia in human brain. *J. Neurochem.*, 2001, vol. 79, 539-544
- **WHITE, H. et al.** Effect of a hypertonic balanced ketone solution on plasma, CSF and brain beta-hydroxybutyrate levels and acid-base status. *Intensive Care Med.*, 2013, vol. 39, 727-733
- **PAN, J. et al.** Human brain beta-hydroxybutyrate and lactate increase in fasting-induced ketosis. *J. Cereb. Blood Flow Metab.*, 2000, vol. 20, 1502-1507
- **CLARKE, :K. et al.** Kinetics, safety and tolerability of (R)-3-hydroxybutyl (R)-3-hydroxybutyrate in healthy adult subjects. *Regul. Toxicol. Pharmacol.*, 2012, vol. 63, 401-408
- **DELFT, R. et al.** Blood beta-hydroxybutyrate correlates better with seizure reduction due to ketogenic diet than do ketones in the urine. *Seizure.*, 2010, vol. 19, 36-39
- **MADISON, LL et al.** The hypoglycemic action of ketones. II. Evidence for a stimulatory feedback of ketones on the pancreatic beta cells. *J. Clin. Invest.*, 1964, vol. 43, 408-415

- **OFFERMANNS, S.** ; **SCHWANINGER, M.** Nutritional or pharmacological activation of HCA2 ameliorates neuroinflammation. *Trends in Molec. Med.*, 2015, vol. 21 (40), 345-55
- **OFFERMANNS, S. et al.** *Pharmacol. Rev.*, 2011, vol. 63, 269-90
- **SOGA, T. et al.** Molecular identification of nicotinic acid receptor. *Biochem. Biophys. Res. Commun.*, 2003, vol. 303, 364-69
- **TUNARU, S. et al.** PUMA-G and HM74 are receptors for nicotinic acid and mediate its anti-lipolytic effect. *Nat. Med.*, 2003, vol. 9, 352-55
- **WISE, A. et al.** Molecular identification of high and low affinity receptors for nicotinic acid. *J. Biol. Chem.*, 2003, vol. 278, 9869-74
- **TAGGART, AK et al.** D-β-hydroxybutyrate inhibits adipocyte lipolysis via the nicotinic aid receptor PUMA-G. *J. Biol. Chem.*, 2005, vol. 280, 26649-52
- **BENYO, Z. et al.** GPR109A (PUMA-G/HM74A) mediates nicotinic acid-induced flushing. *J. Clin. Invest.*, 2005, vol. 115, 3634-40
- **KOSTYLINN, G. et al.** Neutrophil apoptosis mediated by nicotinic acid receptors (GPR109A). *Cell Death Differ.*, 2005, vol. 15, 134-42
- **KOSTYLINA, G. et al.** Neutrophil apoptosis mediated by nicotinic acid receptors (GPR109A). *Cell Death Differ.*, 2008, vol. 15, 134-42
- **MAXIEJEWSKI-LENOIR, D. et al.** Langerhans cells release prostaglandin D2 in response to nicotinic acid. *J. Invest. Dermatol.*, 2006, vol. 126, 2637-46
- **RAHMAN, M. et al.** The β-hydroxybutyrate receptor HCA2 activates a neuroprotective subset of macrophages. *Nat. Commun*, 2014, vol. 5, 3944
- **SCHAUB, A. et al.** PUMA-G, an IFN-γ-inducible gene in macrophages is a novel member of the seven transmembrane spanning receptor superfamily. *Eur. J. Immunol.*, 2001, vol. 31, 3714-25
- **SINGH, N. et al.** Activation of Gpr109a, receptor for niacin and the commensal metabolite butyrate, suppresses colonic inflammation and carcinogenesis. *Immunity*, 2014, vol. 40, 128-39
- **LUKASOVA, M. et al.** Nicotinic acid (niacin): new lipid-independent mechanisms of action and therapeutic potentials. *Trends Pharmacol. Sci.*, 2011, vol. 32, 700-7
- **RAHMAN, M. et al.** The β-hydroxybutyrate receptor HCA2 activates a neuroprotective subset of macrophages. *Nat. Commun.*, 2014, vol. 5, 3944
- **LEVY, RG et al.** Ketogenic diet and other dietary treatments for epilepsy. *Cochrane Database Syt. Rev.*, vol. 20120 (3), CD001903
- **NEWMAN, JC** ; **VERDIN, E.** Ketone bodies as signaling metabolites. *Trends Endocrinol. Metab.*, 2014, vol. 25, 42-52
- **WON, Y-J et al.** β-hydroxybutyrate modulates N type calcium channels in rat sympathetic neurons by acting as an agonist for the G protein coupled receptor FFAS. *J. Neurosci.*, 2013, vol. 33, 19314-25
- **KRATOFIL, RM et al.** Monocyte Conversion During Inflammation and Injury. *Arteriosclerosis, Thrombosis and Vascular Biology*, 2017, vol. 37 (1), 35-42
- **GEISSMANN, F. et al.** Blood monocytes consist of two principal subsets with distinct migratory properties. *Immunity*, 2003, vol. 19, 71-82
- **NEWMAN JC** ; **VERDIN, E.** β-hydroxybutyrate: a signaling metabolite. *Ann. Rev. Nutrition*, 2017, vol. 37, 51-76
- **NEWMAN, JC** ; **VERDIN, E**. β-Hydroxybutyrate. *Annu. Rev. Nutr.*, 2017, vol. 37, 51-76
- **CHRIETT, S. et al.** Prominent action of butyrate over β-hydroxybutyrate as histone deactylase inhibitor, transcriptional modulator and anti-inflammatory molecule. *Sci. Reports*, 2019, vol. 9 (1), 742
- **NEWMAN, JC** ; **VERDIN**. E β-Hydroxybutyrate. *Annu. Rev. Nutr.*, 2017, vol. 37, 51-76
- **LINCOLN, BC et al.** *Arch. Biochem. Biophys.*, 1987, vol. 259, 149-56
- **CUENOUD, B. et al.** Metabolism of exogenous D-beta-hydroxybutyrate, an energy substrate avidly consumed by the heart and kidney. *Frontiers Nutrition*, 2020, vol. 7, 13
- **WHITE, H. et al.** A systematic review of intravenous β-hydroxybutyrate use in humans - a promising future therapy?. *Frontiers Medicine*, 2021, vol. 8, 740374
- **PELLERIN, L. et al.** *J. Neurosci. Res.*, 2005, vol. 79, 55-64
- **BARTON, KM** ; **PALMER, SE**. *Curr. HIV/AIDS Rep.*, 2016, vol. 13, 77-84
- **TAKEN FROM DAINES, SA et al.** The therapeutic potential and limitations of ketones in traumatic brain injury. *Frontiers Neurology*, 2021, vol. 12, 723148
- **DAINES, SA et al.** The therapeutic potential and limitations of ketones in traumatic brain injury. *Frontiers Neurology*, 2021, vol. 12, 723148
- **JANFAZA, P. et al.** Surgical anatomy of the head and neck.. Harvard University Press, 15 June 2011
- **STEEN, EH et al.** *Adv. Wound Care (New Rochelle)*, 2020, vol. 9 (4), 184-98
- **O'GARRA, A. et al.** *Immunol. Rev.*, 2008, vol. 223, 114-31
- **VHORA, I. et al.** Applications of Polymers in Drug Delivery. Elsevier, Inc., 2021, vol. 8
- **VHORA, I. et al.** Applications of Polymers in Drug Delivery. Elsevier, Inc., 2021, vol. 8, 221-61, 228
- **MUGELE, RA** ; **EVANS, HD.** Droplet size distribution in sprays. *Indust. And Engineering Chemistry*, 1951, vol. 43 (6), 1317-24

- **LI, J. et al.** The Molecule Pages database. *Nature*, 2002, vol. 420, 716-17
- Neuroglial Cells. Neuroscience. 2001
- Biomarkers for Traumatic Brain Injury. **BRENNAN, J. et al.** Neuropsychological testing. Academic Press, 2020, 397-409
- **FERGUSON, S. et al.** *J. Neurotrauma*, 2016, vol. 34 (8), 1676-91
- **MARSCHNER, L. et al.** *Behav. Brain Res.*, 2019, vol. 365, 222-30
- **BROUSSARD, JI et al.** *Brain In.*, 2018, vol. 32 (1), 113-22
- **NYLEN, K. et al.** *J. Neurol. Sci.*, 2006, vol. 240 (1), 85-91
- FUNDAMENTAL IMMUNOLOGY. Lippincott-Raven Publishers, 1999, 1051-1053
- **NOGRADI, M.** Stereoselective Synthesis. Academic Press, 1983, vol. 3-5
- **JAQUES, J.** ; **COLLET, A.** ; **WILEN, S.** ; **ENANTIO-MER** ; **RACEMATES** ; **RESOLUTIONS**. John Wiley and Sons and Asymmetric Synthesis. Academic Press, 1981, vol. 2
- **KANEKO, N. et al.** *Inflammation and Regeneration*, 2019, vol. 39
- **DINARELLO, C. et al.** *Nature Immunol.*, 2010, vol. 11, 973
- **LACHMAN, LB et al.** *J. Immunol*, vol. 91977 (119), 2019-23
- **HIRANO, T.** *Intl Rev. Immunol*, 1998, vol. 16, 249
- **HUNTER, CA** ; **JONES**. *SA. Nat. Immunol*, 2015, vol. 16, 448
- **VAN SNICK**. *J. Annu. Rev. Immunol.*, 1990, vol. 8, 253
- **HEINRICH, PC et al.** *Biochem. J.*, 2003, vol. 374, 1
- **KAMIMURA, D. et al.** *Rev. Physiol. Biochem. Pharmacol.*, 2003, vol. 149, 1
- **HASEGAWA, H. et al.** *Front. Immunol.*, 2016, vol. 7, 479
- **MURAKAMI, M. et al.** *Immunity*, 2019, vol. 50, 812
- **KUILMAN, T. et al.** *Cell*, 2008, vol. 133, 1019
- **GRIVENNIKOV, S. et al.** *Cancer Cell*, 2009, vol. 15, 103
- **YU;, H. et al.** *Nat. Rev. Cancer*, 2009, vol. 9, 798
- **JONES, SA** ; **JENKINS, BJ.** *Nat. Rev. Immunol.*, 2018, vol. 18, 773
- **HIRANO, T. et al.** *Oncogene*, 2000, vol. 19, 2548
- **JENKINS, BJ et al.** *Blood*, 2007, vol. 109, 2380
- **WILLIS-KARP, M. et al.** *Mucosal Immunol.*, 2010, vol. 3, 104-10
- **RAJASINGH et al.** *FASEB J.*, 2006, vol. 20, 2112-14
- **KRISHNAMURTHY, P. et al.** *Circ. Res.*, 2009, vol. 104, e9-e18
- **KRISHNAMURTHY, P. et al.** *FASEB J.*, 2010, vol. 24, 2484-94
- **MIELKE, MM et al.** *Neurology*, 2019, vol. 93 (3), e252-260
- **HOFFMAN, PN et al.** *Proc. Natl Acad. Sci. USA*, 1987, vol. 84, 3472-6
- **NORGREN, N. et al.** *Brain Res.*, 2003, vol. 987, 25-31
- **MATTSSON, N. et al.** *JAMA Neurol.*, 2017, vol. 74, 557-66
- **KERN, S. et al.** *JAMA Neurol.*, 2019, vol. 76 (2), 187-933
- **SCHERLING, CS et al.** *Ann. Neurol.*, 2014, vol. 75, 116-26
- **TEUNISSEN, CE et al.** *Lancet Neurol.*, 2005, vol. 4, 32-41
- describe pharmaceutically acceptable salts in detail. **P. H. STAHL et al.** Handbook of Pharmaceutical Salts: Properties, Selection, and Use. Wiley VCH, 2002
- **MONDELLO, S. et al.** *BMC Neurology*, 2012, vol. 12
- Remington's Pharmaceutical Sciences. 2000
- Polymers in nasal drug delivery: an overview. **JAVIA, A. et al.** Applications of Polymers in Drug Delivery. Elsevier, 2021, 306-323
- **MARTTIN, E. et al.** Nasal mucociliary clearance as a factor in nasal drug delivery. *Adv. Drug Deliv. Rev.*, 1998, vol. 29, 13-38
- **ILLUM, L. NASAL**. drug delivery - possibilities, problems and solutions. *J. Controlled Re.*, 2003, vol. 87, 187-98
- **KIMBELL, JS et al.** Correlation of regional formaldehyde flux predictions with the distribution of formaldehyde-induced squamous metaplasia in F344 rat nasal passages. *Mutat. Res. Fund. Mol. Mech. Mutagen*, 1997, vol. 380, 143-54
- **ILLUM, L.** Is nose-to-brain transport of drugs in man a reality?. *J. Pharm. Pharmacol.*, 2004, vol. 56, 3-17
- **HANSON, LR** ; **FREY, WH**. Intranasal delivery bypasses the blood-brain barrier to target therapeutic agents to the central nervous system and treat neurodegenerative disease. *BMC Neurosci. 9: S5; Illum, L. Nasal drug delivery - possibilities, problems and solutions. J. Controlled Re.*, 2003, vol. 87, 187-98
- **LOCKHEAD, JJ** ; **THORNE, RG**. Intranasal delivery of biologics to the central nervous system. *Adv. Drug Deliv Revs.*, 2012, vol. 64, 614-28
- **REESE, TS** ; **KARNOVSKY**. *MJ. J. Cell Biol.*, 1967, vol. 34, 207-17
- **CRONE, C.** ; **OLESEN, SP.** *Brain Res.*, 1982, vol. 241, 49-55

- **BUTT, AM et al.** *J. Physiol*, 1990, vol. 429, 47-62
- **BANKS, WA.** *MBMC Neurol.*, 2009, vol. 9, S3
- **DICK, AP et al.** *Proc. Natl Acad. Sci. USA*, 1984, vol. 81, 7233-37
- **JEFFRIES, WA et al.** *Nature*, 1984, vol. 312, 162-3
- **BANKS**. *WA. Regul. Pept.*, 2008, vol. 149, 11-14
- **ILLUM, L.** Is nose-to-brain transport of drugs in man a reality. *J. Pharm. Pharmacol.*, 2004, vol. 56, 3-17
- **SINGH, AK et al.** Nasal cavity, a promising , a promising transmucosal platform for drug delivery and research approaches from nasal to brain targeting. *J. Drug Deliv. Thera.*, 2012, 2
- **ARORA, P. et al.** Permeability issues in nasal drug delivery. *DrugDiscov. Today*, 2002, vol. 7, 967-75
- **UGWOKE, ML et al.** The biopharmaceutical aspects of nasal mucoadhesive drug delivery. *J. Pharm. Pharmacol.*, 2001, vol. 53, 3-22
- **ROMEO, V. et al.** Effects of physicochemical properties and other factors on systemic nasal drug delivery. *Adv. Drug Deliv. Rev.*, 1998, vol. 29, 89
- **PIRES, A et al.** Intranasal drug delivery: how, why and what for. *J. Pharm. Pharm. Sci.*, 2009, vol. 12, 288-311
- **QURAISHI, M. et al.** The rheology of nasal mucus: a review. *Clin. Otolaryngol. Allied Sci.*, 1998, vol. 23, 403-13
- **ILLUM, L.** Nanoparticulate systems for nasal delivery of drugs: a real improvement over simple systems. *J. Pharm. Sci.*, 2007, vol. 96, 473-83
- **STANLEY, A. et al.** Characterization of ovine nasal-associated lymphoid tissue and identification of M cells in the overlying follicle-associated epithelium. *J. Comp. Pathol.*, 2001, vol. 125, 262-70
- **LOCKHEAD, JJ et al.** Intranasal delivery of biologics to the central nervous system. *Adv. Drug Deliv. Rev.*, 2012, vol. 64 (7), 614-28
- **SAKANE, T. et al.** The transport of a drug to the cerebrospinal fluid directly from the nasal cavity: the relation to the lipophilicity of the drug. *Chem. Pharm. Bull.*, 1991, vol. 39, 2456-58
- **TURKER, S. et al.** Nasal route and drug delivery systems. *Pharm World Sci.*, 2004, vol. 26, 137-42
- **CHARLTON, S. et al.** Evaluation of bioadhesive polymers as delivery systems for nose to brain delivery: in vitro characterization studies. *J. Controlled Rel.*, 2007, vol. 118, 225-34
- **SCHAEFER, ML et al.** Trigeminal collaterals in the nasal epithelium and olfactory bulb: a potential route for direct modulation of olfactory information by trigeminal stimuli. *J. comp. Neurol.*, 2002, vol. 444, 221-26
- **WIOLAND, M-A et al.** CFTR, MDR1 and MRP1 immunolocalization in normal human nasal respiratory mucosa. *J. Histochem. Cytochem.*, 2000, vol. 48, 1215-22
- **GRAFF, CL ; POLLACK, GM.** P-glycoprotein attenuates brain uptake of substrates after nasal instillation. *Pharm. Res.*, 2003, vol. 20, 1225-30
- **YAMAMOTO, A. et al.** Absorption of water-soluble compounds with different molecular weights and [Asu1.7]-eel calcitonin from various mucosal administration sites. *J. Controlled Rel.*, 2001, vol. 76, 363-74
- **CORBO, DC et al.** Characterization of the barrier properties of mucosal membranes. *J. Pharm. Sci.*, 1990, vol. 79, 202-6
- **HINCHCLIFFE, M. ; ILLUM, L.** Intranasal insulin delivery and therapy. *Adv. Drug Deliv. Rev.*, 1999, vol. 35, 199-234
- **SANDERS, P. et al.** Inhalers in healthy subjects and asthmatic patients. *STP Pharma Sci.*, 1997, vol. 7, 3000-76
- **ROMEO, V. et al.** Effects of physicochemical properties and other factors on systemic nasal drug delivery. *Adv. Drug Deliv. Rev.*, 1998, vol. 29, 39
- **ILLUM, L. et al.** Bioadhesive starch microspheres and absorption enhancing agents act synergistically to enhance the nasal absorption of polypeptides. *Int. J. Pharm.*, 2001, vol. 222, 109-19
- **MARTTIN, E. et al.** Efficacy, safety and mechanism of cyclodextrins as absorption enhancers in nasal delivery of peptide and protein drugs. *J. Drug Target*, 1998, vol. 6, 17-36
- **MERKUS, F. et al.** Cyclodextrins in nasal drug delivery. *Adv. Drug Deliv. Rev.*, 1999, vol. 36, 41-57
- **SHARMA, N. et al.** Mucoadhesive thermoreversible nasal delivery system. *J. Pharm. Res.*, 2010, vol. 3, 991-97
- **WANKA, G. et al.** The aggregation behavior of poly-(oxyethylene)-poly-(oxypropylene)-poly-(oxyethylene) block copolymers in aqueous solution. *Colloid Polym. Sci.*, vol. 268, 101-17
- **SHAIKH, RP et al.** A review of multi-responsive membranous systems for rate-modulated drug delivery. *AAPS PharmaSciTech.*, 2010, vol. 11, 441-59
- **PARK, SY ; BAE, YH.** Novel pH-sensitive polymers containing sulfonamide groups. *Macromol. Rapid Commun.*, 1999, vol. 20, 269-73
- **SALAMAT-MILLER, N. et al.** The use of mucoadhesive polymers in buccal drug delivery. *Adv. Drug Deliv. Rev.*, 2005, vol. 57, 1666-91
- **UGWOKE, ML et al.** Nasal mucoadhesive drug delivery: background, applications, trends and future perspectives. *Adv. Drug Deliv. Rev.*, 2005, vol. 57, 1640-65

- **JIMENEZ-CASTELLANOS, MR et al.** Mucoadhesive drug delivery systems. *Drug. Dev. Ind. Pharm.*, 1993, vol. 19, 143-94
- **YU, G. et al.** Fluid flow and particle diffusion in the human upper respiratory system. *Aerosol Science and Technol.*, 1998, vol. 28, 146-58

- *Aerosol Science and Technol.*, 1998, vol. 28, 146-58
- **LUONG, TN et al.** Assessment of motor balance and coordination in mice using the balance beam. *J. Vis. Exp.*, 2011, vol. 49, 2376